# EUROPEAN PATENT APPLICATION

(11) **EP 4 717 312 A1**
(43) Date of publication of application: **01.04.2026**
(21) Application number: 24306586.9
(22) Date of filing: 27.09.2024
(51) Int. Cl.: A61P 11/06, A61P 25/28, A61P 27/00, A61P 37/00, C07D 487/04, A61K 31/519

(54) **PYRAZOLO[1,5-A]PYRIMIDINE DERIVATIVES USEFUL AS A MEDICAMENT**

(71) Applicant: Perha Pharmaceuticals, 29680 Roscoff (FR)
(72) Inventor: ELIE, Jonathan, 29680 Roscoff (FR); MEIJER, Laurent, 29680 Roscoff (FR)
(74) Representative: Ipsilon

(57) **Abstract**

The present invention relates to a compound of formula (I) or any of its pharmaceutically acceptable salt

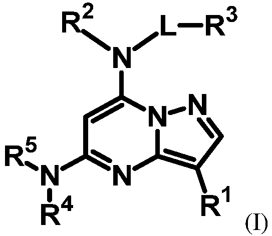

The present invention further relates to a synthesis process for manufacturing compound of formula (I) or any of its pharmaceutically acceptable salts, comprising at least a step of reacting a compound of formula (II) or (VIII) 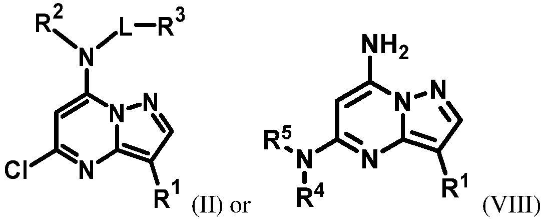 with respectively
an amine of formula (VII) 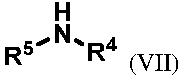 or with a compound of formula (IX)

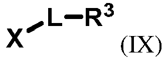

## Description

### FIELD OF THE INVENTION

The present invention relates to pyrazolo[1,5-a]pyrimidine derivatives useful in treating pathologies mediated by adenosine A3 receptor (A₃AR). It further relates to their manufacturing process and to pharmaceutical compositions containing them.

### BACKGROUND

Adenosine interacts with four types of G-protein-coupled receptors (GPCRs), the adenosine receptors (AR): A₁AR, A_{2A}AR, A_{2B}AR, A₃AR as described in IJzerman, A. P. et al., International Union of Basic and Clinical Pharmacology, CXII: Adenosine Receptors: A Further Update, Pharmacol Rev 2022, 74 (2), 340-372. A_{2A}AR has been most extensively studied while A₃AR is the least studied AR.

A large number of sub-type specific AR agonists and antagonists have been developed in the last few decades and evaluated in clinical trials for numerous indications as described in IJzerman, A. P. et al., International Union of Basic and Clinical Pharmacology, CXII: Adenosine Receptors: A Further Update, Pharmacol Rev 2022, 74, 340-372; and described in Jacobson, K. A. et al. Medicinal Chemistry of P2 and Adenosine Receptors: Common Scaffolds Adapted for Multiple Targets, Biochem Pharmacol 2021, 187, 114311. The approved AR drugs are the A₁AR agonist adenosine (paroxysmal supraventricular tachycardia), the A_{2A}AR agonist Regadenoson (used for myocardial perfusion imaging), the A₁AR antagonist Bamifylline (asthma) and the A_{2A}AR antagonist Istradefylline (Parkinson's disease).

A₃AR (P0DMS8) (gene located on human chromosome 1 (1p13.2); 318 amino acids in human) was discovered in the early nineties as described in Borea, P. A. et al., The A3 Adenosine Receptor: History and Perspectives, Pharmacol Rev 2015, 67, 74-102. A₃AR is coupled to Gi proteins, leading to inhibition of adenylate cyclase, and to Go proteins, leading to activation of phospholipase C and Ca⁺⁺ release. A₃AR is also coupled to PI3K/Akt, MAPKs and NF-κB signaling pathways. The biodistribution of A₃AR is rather species-dependent. In man, A₃AR is expressed in lung, liver, kidney, pancreas, brain, testis and cells of the immune system. A₃AR knock-out mice have been generated to investigate the functions of A₃AR. A₃AR^{-/-} mice are viable, fertile and apparently morphologically indistinguishable from wild-type mice, suggesting that inhibition of this receptor is unlikely to lead to gross toxicity.

Although A₁AR, A_{2A}AR and A_{2B}AR are well conserved throughout evolution, there are rather important differences in the A₃AR sequences of rodents and man (72% and 73% sequence identity between human A₃AR and rat or mouse A₃AR, respectively). Many human A₃AR antagonists are very poorly active or even inactive on rat and mouse orthologs. This complicates translational investigations on the action of A₃AR agonists and antagonists in rodent models of human diseases. A₃AR-humanized (A₃AR^{h/h}) and human/mouse chimeric A₃AR (A₃AR^{c/c}) mice have been constructed which are sensitive to the agonists developed against human A₃AR.

In cerebral ischemia A₃AR may play both a protective role, by inhibiting excitatory synaptic transmission in synergy with A_{1A}R, and a deleterious effect, by favoring excitotoxicity. A₃AR agonists may regulate pain signaling, in particular in the case of anticancer drugs-induced neuropathic pain. A₃AR is overexpressed in several tumors and has anti-proliferative and pro-proliferative effects. A₃AR agonists and antagonists display antitumor activity. A₃AR appears to play a key role in the modulation of inflammation by adenosine. A₃AR agonists have therefore been evaluated in clinical trials to treat various inflammatory diseases such as osteoarthritis of the knee, asthma, lung fibrosis. A₃AR agonists have been investigated for induction of hypothermia as well as for potential cardioprotective activity, yet the deletion of A₃AR appears to confer some resistance to myocardial ischemic injury.

The important role of A₃AR in the pathogenesis of vascular dementia (VaD) was also confimed, as described in Xu Y, Tang L, Zhou C, et al. Inhibition of ADORA3 promotes microglial phagocytosis and alleviates chronic ischemic white matter injury, CNS Neurosci Ther. 2024; 30:e14742. A₃AR antagonist alleviates chronic ischemic white matter injury (WMI) by modulating myelin clearance of microglia, which is a therapeutic target for the treatment of VaD.

Several potent and selective A₃AR agonists have been described, Piclidenoson (IB-MECA, N⁶-iodobenzyl-substituted methylcarboxamidoadenosine, CF101, Can-Fite BioPharma) and Namodenoson (2-C1-IB-MECA, CF102) being the most advanced drug candidates. Piclidenoson is in clinical trials against in rheumatoid arthritis and psoriasis, while Namodenoson is in clinical trials against hepatocellular carcinoma and non-alcoholic steatohepatitis (NASH).

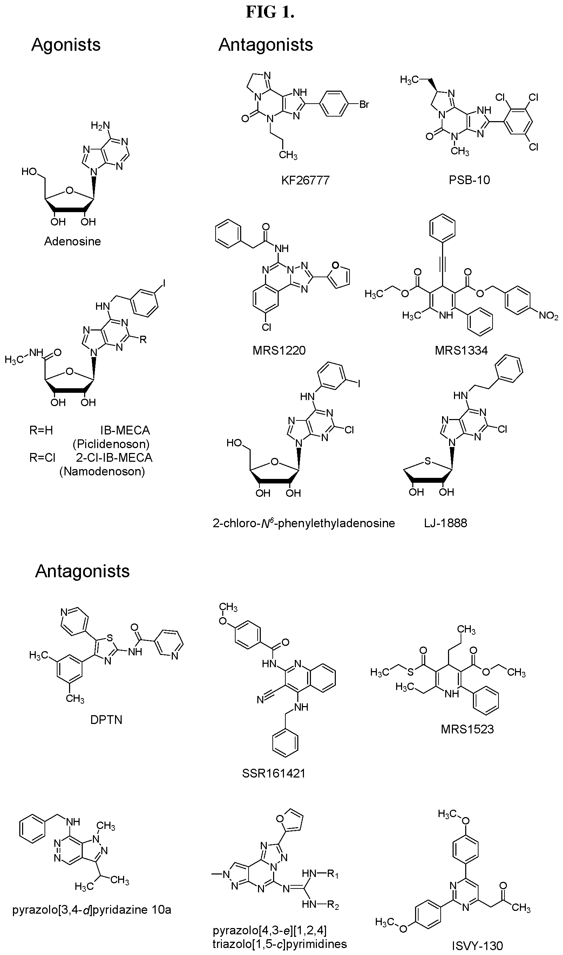

Several A₃AR antagonists have also been described, such as KF2677, PSB-10, DPTN (N-[4-(3,5-dimethylphenyl)-5-(4-pyridyl)-1,3-thiazol-2-yl]nicotinamide), MRS1523, SSR161421, LJ-1888, pyrazolo-triazolo-pyrimidines, ISVY-130 analogs, 2-chloro-N6-phenylethyladenosine as described in Borea, P. A. *et al.* previously mentioned, 7-Amino-pyrazolo[3,4-d]pyridazine 10a (dual A1/A3AR antagonist), as described in Gao, Z.-G. et al. Species Dependence of A3 Adenosine Receptor Pharmacology and Function, Purinergic Signal 2022, 1-28, as illustrated in **figure 1****.**

In prior art, only the compounds PBF-677 and PBF-1650, (undisclosed structures) have entered clinical trials, for ulcerative colitis (phase 2) and atopic dermatitis (phase 1), respectively (https://www.palobiofarma.com/pipeline-2/). A₃AR antagonists are evaluated for the treatment of glaucoma as they lower intraocular pressure (IOP) in animal models of glaucoma. A₃AR antagonists have been evaluated as treatment of asthma, ulcerative colitis, liver fibrosis, kidney diseases in particular kidney fibrosis, nephropathy, nephrotoxicity, atherosclerosis and hypercholestemia. Stimulation of A₃AR by adenosine, under hypoxia, contributes to the migration, invasiveness and chemoresistance of glioblastoma stem-like cells and probably in other cancer types under hypoxic conditions. A₃AR antagonists inhibit the proliferation of glioblastoma cell lines.

Hearing loss (HL) originates from exposure to ototoxic agents (such as platinum-based anticancer drugs or aminoglycoside antibiotics - there are over 600 ototoxic agents described, among which over 200 marketed drugs), from noise, from acoustic trauma (high intensity noise) or from aging. These aggressions trigger irreversible death of ear hair cells located in the organ of Corti (cochlea). The function of these cells is to capture sound vibrations and transduce them as neuronal signals sent to the brain auditory structures. There is considerable interest in the development of otoprotective compounds.

Cisplatin is a highly effective anticancer drug widely used for the treatment of cancers. Cisplatin's side effects include nephrotoxicity and ototoxicity. Cisplatin-induced ototoxicity occurs in 23-50% of adults and up to 60% in children. Elevated hearing thresholds may occur in up to 100% of cisplatin-treated cancer patients. Otoprotection would allow the use of these highly effective drugs for life threatening diseases at reduced risk of HL, thereby addressing a significant unmet clinical need. Similarly addressing cisplatin-induced nephrotoxicity would be beneficial. The same applies for the potent but HL-inducing antibiotics, in particular ototoxicity is observed in up to 47% of patients treated with gentamycin, and other products such as neomycin, tobramycin, kanamycin. Noise-induced HL occurs when the ear is exposed to unsafe levels of sounds. The industries where workers often experience noise exposure include agriculture, mining, construction, manufacturing and utilities, transportation, and the military. Globally, some 1.1 billion teenagers and young adults are at risk of HL due to the unsafe use of personal audio devices and exposure to damaging levels of sound at noisy entertainment venues. In all these situations, external protective ear coverings are either insufficient or inappropriate. Moreover, A₃R is in particular present in the rat cochlea and is predominantly expressed in the organ of Corti (inner and outer hair cells, Deiter's cells, Claudius cells, pillar cells). Furthermore, adenosine plays a protective role in nephrotoxicity induced by various drug agents. In particular, there is data available in the litterature suggesting that A₂AR and A₃AR antagonism may have some protective effects in kidneys exposed to nephrotoxic agents as described in Dewaeles et al. Istradefylline protects from cisplatin-induced nephrotoxicity and peripheral neuropathy while preserving cisplatin antitumor effects, J. Clin. Invest. 2022, 132, e152924; Lee et al. A3 adenosine receptor knockout mice are protected against ischemia- and myoglobinuria-induced renal failure, Am. J. Physiol. Renal Physiol. 2003, 284, 267-273; and Min et al. Renopprotective effects of a highly selective A3 adenosine receptor antagonist in a mouse model of adriamycin-induced nephropathy, J. Korean Med. Sci. 2016, 31, 1403-1412.

There is a thus a need to find therapeutic agents to target specific inhibition of A₃R.

### SUMMARY OF THE INVENTION

It has now been found that the compounds as defined in formula (I) hereinafter are useful in the treatment and/or prevention of diseases mediated by A₃R.

### FIGURE

**Figure 1** depicts two agonists and a few reported antagonists of A3AR.

### DEFINITIONS

As used herein, the term "*patient*" refers to either an animal, such as a valuable animal for breeding, company or preservation purposes, or preferably a human or a human child, which is afflicted with, or has the potential to be afflicted with one or more diseases and conditions described herein.

In particular, as used in the present application, the term "*patient*" refers to a mammal such as a rodent, cat, dog, primate or human, preferably said subject is a human.

The identification of those patients who are in need of treatment of herein-described diseases and conditions is well within the ability and knowledge of one skilled in the art. A veterinarian or a physician skilled in the art can readily identify, by the use of clinical tests, physical examination, medical/family history or biological and diagnostic tests, those patients who are in need of such treatment.

In the context of the invention, the term "*treating*" or "*treatment*", as used herein, means preventing, reversing, alleviating, inhibiting the progress of, or preventing the diseases as described herein after in the paragraph "PATHOLOGIES".

Therefore, the term "*treating*" or "*treatment*" encompasses within the framework of the present invention the improvement of medical conditions of patients suffering from the diseases as described herein after in the paragraph "PATHOLOGIES", mediated by the A₃AR receptor.

As used herein, an "A₃AR *inhibitor*" or "A₃AR *antagonist*" is a compound able to inhibit A₃AR in the assays displayed in example 6 in biological activity part described hereafter. In one embodiment, the IC₅₀ value from the compounds of formula (I) according to the invention may be less than or equal to 1000 nM, in particular less than or equal to 100 nM, and more particularly less than or equal to 10 nM, and even more particularly less than or equal to 1 nM, said IC₅₀ values all attesting the inhibitory activity of the compounds.

As used herein, an "*effective amount*" refers to an amount of a compound of the present invention which is effective in preventing, reducing, eliminating, treating or controlling the symptoms of the herein-described diseases and conditions. An "*effective amount*" also refers to an amount of a compound of the present invention which is effective in inhibiting A₃AR.

The term "*controlling*" is intended to refer to all processes wherein there may be a slowing, interrupting, arresting, or stopping of the progression of the diseases and conditions described herein, but does not necessarily indicate a total elimination of all disease and condition symptoms, and is intended to include prophylactic treatment.

The term "*effective amount*" includes "*prophylaxis-effective amount*" as well as "treatment-effective amount".

The term "*preventing*", as used herein, means reducing the risk of onset or slowing the occurrence of a given phenomenon, namely in the present invention, a disease mediated by ADORA3.

As used herein, "*preventing*" also encompasses "*reducing the likelihood of occurrence*" or "*reducing the likelihood of reoccurrence*"*.*

The term "*prophylaxis-effective amount*" refers to a concentration of compound of this invention that is effective in inhibiting, preventing, decreasing the likelihood of anyone of the hereabove described diseases.

Likewise, the term "*treatment-effective amount*" refers to a concentration of compound that is effective in treating the hereabove described diseases.

As used herein, the term "*pharmaceutically acceptable*" refers to those compounds, materials, excipients, compositions or dosage forms which are, within the scope of sound medical judgment, suitable for contact with the tissues of human beings and animals without excessive toxicity, irritation, allergic response or other problem complications commensurate with a reasonable benefit/risk ratio.

### DETAILED DESCRIPTION OF THE INVENTION

The inventors have surprisingly found that the compounds of formula (I) as disclosed herein after, present inhibition activity specific to the Adenosine receptor A3 (A₃AR).

This assertion is based on data as illustrated in the following examples and more detailed herein after.

According to a first aspect, a subject-matter of the present invention relates to a compound of formula (I) or any of its pharmaceutically acceptable salt 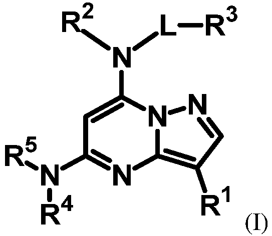 wherein
**R¹** represents a hydrogen atom, a linear or branched (C₁-C₆)alkyl group optionnaly deuterated, or a (C₃-C₆)cycloalkyl group;
**R²** represents a hydrogen atom or a (C₁-C₆)alkyl;
**R³** represents
   - a linear or branched (C₁-C₆)alkyl group, optionnaly substituted by
      - one phenyl group, optionally substituted by one to three substituents selected from a -OR⁶ group, a -NH₂ group, a halogen atom and a (C₁-C₆)alkyl group,
      - one (C₅-C₁₀)heteroaryl group, optionally substituted by one to three substituents selected from -OR⁶ group, a -NH₂ group, a halogen atom and a (C₁-C₆)alkyl group, or
      - one -OR⁶ group;
   - a phenyl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom;
   - a (C₅-C₆)heteroaryl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom; or
   - a (C₃-C₈)cycloalkyl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom;
**L** represents a bond or a -CO- group;
**R⁴** represents
   - a linear or branched (C₁-C₈)alkyl group, optionally substituted by
      - one phenyl group, optionally substituted by one to three substituents selected from a (C₁-C₄)alkyl group and a (C₁-C₄)alkoxy group,
      - one (C₃-C₈)cycloalkyl group, optionally substituted by one to three substituents selected from a halogen atom, a (C₁-C₄)alkyl group and a -OR⁶ group,
      - one (C₅-C₆)heterocycloalkyl,
      - one (C₅-C₆)heteroaryl group, optionally substituted by one to three (C₁-C₆)alkyl group, or
      - one or two -OR⁶ groups;
   - a (C₃-C₇)cycloalkyl group, optionally substituted by one to three substituents selected from a halogen atom, a (C₁-C₄)alkyl group and a -OR⁶ group;
   - a (C₅-C₆)heteroaryl group, optionally substituted by one to three substituents selected from a halogen atom, a (C₁-C₄)alkyl group and a -OR⁶ group; or
   - a (C₃-C₆)heterocycloalkyl group;
**R⁵** represents a hydrogen atom or a (C₁-C₄)alkyl group;
or **R⁴** and **R⁵** form together with the nitrogen atom bearing them a (C₅-C₆)heterocycloalkyl group; and
**R⁶** represents a hydrogen atom, a (C₁-C₄)alkyl group or a deuterated (C₁-C₄)alkyl group.

According to a particular embodiment, herein is further provided a compound of formula (I) as defined herein above, wherein **R¹** represents a linear or branched (C₁-C₆)alkyl group or a (C₃-C₆)cycloalkyl group, in particular a linear or branched (C₁-C₄)alkyl group or a (C₃-C₅)cycloalkyl group, and more particularly an isopropyl group or a cyclopropyl group.

According to a preferred embodiment, herein is provided a compound of formula (I) as defined herein above, wherein **R¹** represents a linear or branched (C₁-C₄)alkyl group or a (C₃-C₅)cycloalkyl group, and more particularly an isopropyl group or a cyclopropyl group.

According to a particular embodiment, herein is further provided a compound of formula (I) as defined herein above, wherein **R²** represents a hydrogen atom or a (C₁-C₄)alkyl, in particular a hydrogen atom or a methyl group.

According to a preferred embodiment, herein is provided a compound of formula (I) as defined herein above, wherein **R²** represents a hydrogen atom or a methyl group.

According to a particular embodiment, herein is further provided a compound of formula (I) as defined herein above, wherein **R³** represents
- a linear or branched (C₁-C₆)alkyl group, optionally substituted by
   - one phenyl group, optionally substituted by one to three -OR⁶ group(s), in particular one methoxy group, or
   - one (C₅-C₁₀)heteroaryl group, in particular one (C₅-C₆)heteroaryl group, more particularly selected from one pyridyl, one pyrazinyl, one pyrimidinyl and one pyridazinyl group, and even more particularly selected from one pyridyl and one pyridazinyl group, said (C₅-C₁₀)heteroaryl group being optionally substituted by one to three -OR⁶ group(s);
- a (C₅-C₆)heteroaryl group, in particular selected from a pyridyl, a pyrazinyl, a pyrimidinyl or a pyridazinyl group, said (C₅-C₆)heteroaryl group being optionally substituted by one to three substituents selected from a (C₁-C₄)alkyl group, a deuterated (C₁-C₄)alkyl group, a (C₁-C₄)alkoxy group, for example one methoxy group, a deuterated (C₁-C₄)alkoxy group, for example one deuterated methoxy group, and a halogen atom, for example one chlorine atom; or
- a (C₃-C₈)cycloalkyl group, in particular a (C₄-C₆)cycloalkyl group, and more particularly a cyclopentyl group; and
**R⁶** represents a hydrogen atom, a (C₁-C₄)alkyl group or a deuterated (C₁-C₄)alkyl group.

According to a preferred embodiment, herein is provided a compound of formula (I) as defined herein above, wherein **R³** represents
- a linear or branched (C₁-C₆)alkyl group, optionally substituted by
   - one phenyl group, optionally substituted by by one to three methoxy group(s), or
   - one (C₅-C₆)heteroaryl group, in particular selected from one pyridyl, one pyrazinyl, one pyrimidinyl and one pyridazinyl group, and even more particularly selected from one pyridyl and one pyridazinyl group;
- a (C₅-C₆)heteroaryl group, in particular selected from a pyridyl, a pyrazinyl, a pyrimidinyl or a pyridazinyl group, said (C₅-C₆)heteroaryl group being optionally substituted by one to three substituents selected from a (C₁-C₄)alkoxy group, for example one methoxy group, and a halogen atom, for example one chlorine atom; or
- a (C₄-C₆)cycloalkyl group, and in particular a cyclopentyl group.

According to a particular embodiment, herein is further provided a compound of formula (I) as defined herein above, wherein
**R⁴** represents
   - a linear or branched (C₁-C₈)alkyl group, in particular a linear or branched (C₃-C₈)alkyl group, said (C₁-C₈)alkyl group being optionally substituted by one to three -OR⁶ group(s), in particular one hydroxyl group;
   - a (C₃-C₇)cycloalkyl group, in particular a (C₄-C₆)cycloalkyl group, more particularly a cyclopentyl group;
   - a (C₅-C₆)heteroaryl group, in particular a pyrazolyl group, said (C₅-C₆)heteroaryl group being optionally substituted by one to three substituents selected from a halogen atom, a (C₁-C₄)alkyl group and a -OR⁶ group, in particular one methyl group; or
   - a (C₃-C₆)heterocycloalkyl group, in particular a tetrahydrofuranyl group; and
**R⁶** represents a hydrogen atom, a (C₁-C₄)alkyl group or a deuterated (C₁-C₄)alkyl group.

According to a preferred embodiment, herein is provided a compound of formula (I) as defined herein above, wherein
**R⁴** represents
- a linear or branched (C₃-C₈)alkyl group, optionally substituted by one hydroxyl group;
- a (C₄-C₆)cycloalkyl group, in particular a cyclopentyl group;
- a (C₅-C₆)heteroaryl group, in particular a pyrazolyl group, said (C₅-C₆)heteroaryl group being optionally substituted by one to three (C₁-C₄)alkyl group(s), in particular one methyl group; or
- a (C₃-C₆)heterocycloalkyl group, in particular a tetrahydrofuranyl group.

According to a particular embodiment, herein is further provided a compound of formula (I) as defined herein above, wherein **R⁵** represents a hydrogen atom.

According to a particular embodiment, herein is provided a compound of formula (I) as defined herein above, wherein
**R¹** represents a hydrogen atom, a linear or branched (C₁-C₆)alkyl group or a (C₃-C₆)cycloalkyl group;
**R²** represents a hydrogen atom or a (C₁-C₆)alkyl;
L represents a bond or a -CO- group;
**R³** represents
   - a linear or branched (C₁-C₆)alkyl group, optionnaly substituted by
      - one phenyl group, optionally substituted by one to three -OR⁶ group(s), or
      - one (C₅-C₁₀)heteroaryl group;
   - a (C₅-C₆)heteroaryl group, optionally substituted by one to three substituents selected from a (C₁-C₄)alkoxy group, in particular one methoxy group, and a halogen atom, in particular one chlorine atom; or
   - a (C₃-C₈)cycloalkyl group;
**L** represents a bond or a -CO- group;
**R⁴** represents
   - a linear or branched (C₁-C₈)alkyl group, optionally substituted by one to three -OR⁶ group(s);
   - a (C₃-C₇)cycloalkyl group;
   - a (C₅-C₆)heteroaryl group, optionally substituted by one to three (C₁-C₄)alkyl group(s); or
   - a (C₃-C₆)heterocycloalkyl group;
**R⁵** represents a hydrogen atom or a (C₁-C₄)alkyl group; and
**R⁶** represents a hydrogen atom or a (C₁-C₄)alkyl group.

According to a preferred embodiment, herein is provided a compound of formula (I) as defined herein above, wherein
**R¹** represents a linear or branched (C₁-C₄)alkyl group or a (C₃-C₅)cycloalkyl group, and more particularly an isopropyl group or a cyclopropyl group;
**R²** represents a hydrogen atom or a (C₁-C₄)alkyl, in particular a hydrogen atom or a methyl group;
**L** represents a bond or a -CO- group;
**R³** represents
   - a linear or branched (C₁-C₆)alkyl group, optionally substituted by
      - one phenyl group, optionally substituted by one to three -OR⁶ group(s), in particular one methoxy group, or
      - one (C₅-C₆)heteroaryl group, in particular selected from one pyridyl, one pyrazinyl, one pyrimidinyl and one pyridazinyl group, and even more particularly selected from one pyridyl and one pyridazinyl group;
   - a (C₅-C₆)heteroaryl group, in particular selected from a pyridyl, a pyrazinyl, a pyrimidinyl or a pyridazinyl group, said (C₅-C₆)heteroaryl group being optionally substituted by one to three substituents selected from a (C₁-C₄)alkoxy group, for example one methoxy group, and a halogen atom, for example one chlorine atom; or
   - a (C₄-C₆)cycloalkyl group, and in particular a cyclopentyl group;
**R⁴** represents
   - a linear or branched (C₃-C₈)alkyl group, optionally substituted by one hydroxyl group;
   - a (C₃-C₇)cycloalkyl group, in particular a (C₄-C₆)cycloalkyl group, more particularly a cyclopentyl group;
   - a (C₅-C₆)heteroaryl group, in particular a pyrazolyl group, said (C₅-C₆)heteroaryl group being optionally substituted by one to three (C₁-C₄)alkyl group, in particular one methyl group; or
   - a (C₃-C₆)heterocycloalkyl group, in particular a tetrahydrofuranyl group;
**R⁵** represents a hydrogen atom; and
**R⁶** represents a hydrogen atom or a (C₁-C₄)alkyl group.

According to a more preferred embodiment, herein is provided a compound of formula (I) as defined herein above, wherein
**R¹** represents an isopropyl group or a cyclopropyl group;
**R²** represents a hydrogen atom or a methyl group;
**L** represents a bond or a -CO- group;
**R³** represents
   - a linear or branched (C₁-C₆)alkyl group, optionally substituted by
      - one phenyl group, optionally substituted by one methoxy group,
      - one pyridyl, or
      - one pyridazinyl group;
   - a pyridyl, a pyrazinyl, a pyrimidinyl or a pyridazinyl group, optionally substituted by one to three substituents selected from one methoxy group and one chlorine atom; or
   - a cyclopentyl group;
**R⁴** represents
   - a linear or branched (C₃-C₈)alkyl group, optionally substituted by a hydroxyl group;
   - a cyclopentyl group;
   - a pyrazolyl group, optionally substituted by one to three methyl group(s), in particular one methyl group; or
   - a tetrahydrofuranyl group; and
**R⁵** represents a hydrogen atom.

According to a particular embodiment, herein is provided a compound of formula (I) as defined herein above, wherein
**R¹** represents a linear or branched (C₁-C₆)alkyl group or a (C₃-C₆)cycloalkyl group, in particular a linear or branched (C₁-C₄)alkyl group or a (C₃-C₅)cycloalkyl group, and more particularly an isopropyl group or a cyclopropyl group;
**R²** represents a hydrogen atom;
L represents a bond or a -CO- group;
**R³** represents
   - a linear or branched (C₁-C₆)alkyl group, optionally substituted by
      - one phenyl group, optionally substituted by at least optionally substituted by one to three -OR⁶ group(s), in particular one methoxy group, or
      - one (C₅-C₆)heteroaryl group, in particular selected from one pyridyl, one pyrazinyl, one pyrimidinyl and one pyridazinyl group, and even more particularly selected from one pyridyl and one pyridazinyl group;
   - a (C₅-C₆)heteroaryl group, in particular selected from a pyridyl, a pyrazinyl, a pyrimidinyl or a pyridazinyl group, said (C₅-C₆)heteroaryl group being optionally substituted by one to three substituents selected from a (C₁-C₄)alkoxy group, for example one methoxy group, and a halogen atom, for example one chlorine atom; or
   - a (C₃-C₈)cycloalkyl group, in particular a (C₄-C₆)cycloalkyl group, and more particularly a cyclopentyl group;
**R⁴** represents
   - a linear or branched (C₁-C₈)alkyl group, in particular a linear or branched (C₃-C₈)alkyl group, optionally substituted by one to three -OR⁶ group(s), in particular one hydroxyl group;
   - a (C₃-C₇)cycloalkyl group, in particular a cyclopentyl group;
      or
   - a (C₃-C₆)heterocycloalkyl group, in particular a tetrahydrofuranyl group;
**R⁵** represents a hydrogen atom; and
**R⁶** represents a hydrogen atom or a (C₁-C₄)alkyl group;
in particular, provided that, when **R⁴** is a branched C₄-alkyl group substituted by one hydroxyl group, **R³** is not a linear C₁-alkyl group substituted by one phenyl group.

According to a preferred embodiment, herein is provided a compound of formula (I) as defined herein above, wherein
**R¹** represents an isopropyl group or a cyclopropyl group;
**R²** represents a hydrogen atom;
**L** represents a bond or a -CO- group;
**R³** represents
   - a linear or branched (C₁-C₆)alkyl group, optionally substituted by
      - one phenyl group, optionally substituted by one methoxy group,
      - one pyridyl, or
      - one pyridazinyl group;
   - a pyridyl, a pyrazinyl, a pyrimidinyl or a pyridazinyl group, optionally substituted by one methoxy group or a halogen atom, for example a chlorine atom; or
   - a cyclopentyl group;
**R⁴** represents
   - a linear or branched (C₃-C₈)alkyl group, optionally substituted by one hydroxyl group;
   - a cyclopentyl group; or
   - a tetrahydrofuranyl group; and
**R⁵** represents a hydrogen atom,
in particular, provided that, when **R⁴** is a branched C₄-alkyl group substituted by one hydroxyl group, **R³** is not a linear C₁-alkyl group substituted by one phenyl group.

Herein is further provided a pharmaceutical composition comprising a compound of formula (I) as defined above or any of its pharmaceutically acceptable salts, or at least any of compounds **(1)** to **(58)** as defined hereinafter or any of their pharmaceutically acceptable salts.

As illustrated in example 6, the compounds of formula (I) have been tested as A₃AR antagonists. It has been observed that the compounds of formula (I) exert an inhibitory activity on A₃AR and thus may be useful in the treatment of pathologies mediated by A₃AR.

Additionaly, compounds of formula (I) as defined above or any of its pharmaceutically acceptable salts, or at least any of compounds **(1)** to **(58)** as defined hereinafter or any of their pharmaceutically acceptable salts, may display protective activity towards inner ear hair cells of the organ of Corti exposed to ototoxic agents, in particular platin-based anticancer drugs such as cisplatin, or antibiotics aminoglycosides such as gentamicin or neomycin.

In the context of the present invention, the term:
- "halogen" is understood to mean chlorine, fluorine, bromine, or iodine, and in particular denotes chlorine, fluorine or bromine,
- "(C₁-Cₓ)alkyl", as used herein, respectively refers to a C₁-Cₓ normal, secondary or tertiary monovalent saturated hydrocarbon radical, for example (C₁-C₆)alkyl. Examples are, but are not limited to, methyl, ethyl, propyl, *n*-propyl, isopropyl, butyl, isobutyl, *sec-*butyl, *tert*-butyl, pentyl, isopentyl, hexyl and isohexyl groups, and the like.
- "(C₁-Cₓ)alkoxy", as used herein, refers to a -O-(C₁-Cₓ)alkyl or -O-(C₃-Cₓ)cycloalkyl moiety, wherein alkyl and cycloalkyl are as defined above, for example (C₁-C₆)alkoxy. Examples are, but are not limited to, methoxy, ethoxy, 1-propoxy, 2-propoxy, cyclopropoxy, butoxy, *tert*-butoxy and pentoxy.
- "(C₃-C₈)cycloalkyl", as used herein, refers to a monocyclic saturated hydrocarbon, from 3 to 8 carbon atoms, saturated or partially unsaturated and unsubstituted or substituted. Examples of monocyclic rings are, but are not limited to, cyclopropyl, cyclobutyl, cyclopentyl, cyclohexyl, cycloheptyl and cyclooctyl.
- "(C₅-C₆)heterocycloalkyl group", as used herein, refers to a (C₅-C₆)cycloalkyl group wherein one or two of the carbon atoms are replaced with a heteroatom such as oxygen, nitrogen or sulphur, and more particularly with at least one nitrogen atom. Such heterocycloalkyl group may be saturated or partially saturated and unsubstituted or substituted. Examples are, but are not limited to piperazinyl, piperidinyl, pyrrolidinyl, aziridinyl, oxanyl, oxetanyl, tetrahydropyranyl, morpholinyl, tetrahydrofuranyl, oxazolidinyl, oxepanyl, diazepanyl, dioxanyl and tetrahydrothiopyranyl, and more particularly pyrrolidinyl, piperidinyl, morpholinyl, tetrahydropyranyl or one tetrahydrofuranyl group. In the framework of the present invention, the heterocycloalkyl group is advantageously a tetrahydrofuranyl group.
- a "(C₆-C₁₀)heteroaryl group", as used herein, refers to a monocyclic aromatic or a bicyclic group wherein one to three carbon atom is replaced by a heteroatom, such as nitrogen, oxygen or sulphur. By way of examples of monocyclic aromatic ring of heteroaryl groups, more particulary as a (C₅-C₆)heteroaryl group, mention may be made of, but not limited to: oxazolyl, isoxazolyl, pyridyl, pyrimidinyl, pyridazinyl, triazinyl, pyrazinyl, oxadiazolyl, furanyl, pyrazolyl, thiazolyl, isothiazolyl, thiadiazolyl, imidazolyl, triazolyl groups and the like. In the framework of the present invention, the heteroaryl group is advantageously a pyridyl, pyrimidinyl, pyridazinyl, pyrazinyl or a pyrazolyl group.
- an aromatic ring means, according to Hückel's rule, that a molecule has 4*n* + 2 π-electrons.
- "deuterated" refers to a group wherein at least one hydrogen atom is replaced by a deuterium atom, such as perdeuterated group wherein all hydrogen atoms are replaced by deuterium atoms. In other terms, a deuterated group may be partially or totally deuterated. By way of examples of deuterated group, more particularly as deuterated (C₁-C₄)alkyl group, mention may be made of, but not limited to: -CH₂D group, -CHD₂ group or -CD₃ group. By way of other examples of deuterated group, more particularly as deuterated (C₁-C₄)alkoxy group, mention may be made of, but not limited to: deuterated methoxy group such as - -OCH₂D group, -OCHD₂ group or -OCD₃ group.
- a "deuterium atom" (D or ²H) is a stable, non-radioactive isotope of hydrogen and has an atomic weight of 2.0144.
- "optionally substituted" means "unsubstituted or "substituted with".

In the context of the present invention, the terms "aromatic ring", and "heteroaryl" include all the positional isomers.

The nomenclature of the following compounds (1) to (58) was generated according to the principles of the International Union of Pure and Applied Chemistry, using ChemDraw^{®} Professional v22.0.0.22. To avoid any confusion, the "(±)" symbol added to designate a racemic mixture; "*cis*" and "*trans*" prefixes were also used to assign the relative stereochemistry of two adjacent chiral centers.

Specific compounds of formula (I) of the present invention are listed herein after:
**(1)** (2*R*)-2-((7-(benzylamino)-3-isopropylpyrazolo[1,5-*a*]pyrimidin-5-yl)amino)butan-1-ol;
**(2)** *N7*-benzyl-3-isopropyl-*N5*-[(3*R*)-tetrahydrofuran-3-yl]pyrazolo[1,5-*a*]pyrimidine-5,7-diamine;
**(3)** *N7*-benzyl-3-isopropyl-*N5*-(pentan-3-yl)pyrazolo[1,5-*a*]pyrimidine-5,7-diamine
**(4)** 3-isopropyl-N5-(pentan-3-yl)-*N7*-(pyridin-4-ylmethyl)pyrazolo[1,5-*a*]pyrimidine-5,7-diamine;
**(5)** 3-isopropyl-*N5*-(pentan-3-yl)-*N7*-(pyridin-3-ylmethyl)pyrazolo[1,5-*a*]pyrimidine-5,7-diamine;
**(6)** 3-isopropyl-*N5*-(pentan-3-yl)-*N7*-(pyridin-2-ylmethyl)pyrazolo[1,5-*a*]pyrimidine-5,7-diamine;
**(7)** 3-isopropyl-*N5*-(pentan-3-yl)*-N7*-(2-(pyridin-2-yl)ethyl)pyrazolo[1,5-*a*]pyrimidine-5,7-diamine;
**(8)** 3-isopropyl-*N5*-(pentan-3-yl)-*N7*-(2-(pyridin-3-yl)ethyl)pyrazolo[1,5-*a*]pyrimidine-5,7-diamine;
**(9)** 3-isopropyl-*N5*-(pentan-3-yl)-*N7*-(2-(pyridin-4-yl)ethyl)pyrazolo[1,5-*a*]pyrimidine-5,7-diamine;
**(10)** *N5*-(2-ethylbutyl)-3-isopropyl-*N7*-(2-(pyridin-2-yl)ethyl)pyrazolo[1,5-*a*]pyrimidine-5,7-diamine;
**(11)** *N5*-(2-ethylbutyl)-3-isopropyl-*N7*-(2-(pyridin-3-yl)ethyl)pyrazolo[1,5-*a*]pyrimidine-5,7-diamine;
**(12)** *N5,N7*-bis(2-ethylbutyl)-3-isopropylpyrazolo[1,5-*a*]pyrimidine-5,7-diamine
**(13)** *N5*-cyclopentyl-3-isopropyl-*N7*-(2-(2-pyridyl)ethyl)pyrazolo[1,5-*a*]pyrimidine-5,7-diamine;
**(14)** *N5*-cyclopentyl-3-isopropyl-*N7*-(2-(3-pyridyl)ethyl)pyrazolo[1,5-*a*]pyrimidine-5,7-diamine;
**(15)** *N5,N7*-dicyclopentyl-3-isopropylpyrazolo[1,5-*a*]pyrimidine-5,7-diamine
**(16)** 3-isopropyl-*N5*-(pentan-3-yl)-*N7*-(pyridazin-3-ylmethyl)pyrazolo[1,5-*a*]pyrimidine-5,7-diamine;
**(17)** 3-isopropyl-*N5*-(pentan-3-yl)-*N7*-(pyridazin-4-ylmethyl)pyrazolo[1,5-a]pyrimidine-5,7-diamine;
**(18)** 3-isopropyl-*N5*-(pentan-3-yl)-*N7*-(pyridin-2-yl)pyrazolo[1,5-*a*]pyrimidine-5,7-diamine;
**(19)** 3-isopropyl-*N5*-(pentan-3-yl)-*N7*-(pyridin-3-yl)pyrazolo[1,5-*a*]pyrimidine-5,7-diamine;
**(20)** 3-isopropyl-*N5*-(pentan-3-yl)-*N7*-(pyridazin-3-yl)pyrazolo[1,5-*a*]pyrimidine-5,7-diamine;
**(21)** *N5*-(2-ethylbutyl)-3-isopropyl-*N7*-(pyridazin-3-yl)pyrazolo[1,5-*a*]pyrimidine-5,7-diamine;
**(22)** *N5*-(3-ethylpentyl)-3-isopropyl-*N7*-(pyridazin-3-yl)pyrazolo[1,5-*a*]pyrimidine-5,7-diamine;
**(23)** (*S*)-2-((3-isopropyl-7-(pyridazin-3-ylamino)pyrazolo[1,5-*a*]pyrimidin-5-yl)amino)butan-1-ol;
**(24)** 3-isopropyl-*N5*-(pentan-3-yl)-*N7*-(pyrimidin-4-yl)pyrazolo[1,5-*a*]pyrimidine-5,7-diamine;
**(25)** 3-isopropyl-*N5*-(pentan-3-yl)-*N7*-(pyrazin-2-yl)pyrazolo[1,5-*a*]pyrimidine-5,7-diamine;
**(26)** 3-isopropyl-*N5*-(pentan-3-yl)-*N7*-(pyrimidin-5-yl)pyrazolo[1,5-*a*]pyrimidine-5,7-diamine;
**(27)** 3-isopropyl-*N7*-(6-methoxypyridin-2-yl)-*N5*-(pentan-3-yl)pyrazolo[1,5-*a*]pyrimidine-5,7-diamine;
**(28)** 3-isopropyl-*N7*-(5-methoxypyridin-2-yl)-*N5*-(pentan-3-yl)pyrazolo[1,5-*a*]pyrimidine-5,7-diamine;
**(29)** 3-isopropyl-*N7*-(6-methoxypyridin-3-yl)-*N5*-(pentan-3-yl)pyrazolo[1,5-*a*]pyrimidine-5,7-diamine;
**(30)** 3-isopropyl-*N7*-(2-methoxypyrimidin-4-yl)-*N5*-(pentan-3-yl)pyrazolo[1,5-*a*]pyrimidine-5,7-diamine;
**(31)** 3-isopropyl-*N7*-(6-methoxypyridazin-3-yl)-*N5*-(pentan-3-yl)pyrazolo[1,5-*a*]pyrimidine-5,7-diamine;
**(32)** 3-isopropyl-*N7*-(6-methoxypyrimidin-4-yl)-*N5*-(pentan-3-yl)pyrazolo[1,5-*a*]pyrimidine-5,7-diamine;
**(33)** *N7*-(6-chloropyridazin-3-yl)-3-isopropyl-*N5*-(pentan-3-yl)pyrazolo[1,5-*a*]pyrimidine-5,7-diamine;
**(34)** *N7*-(2-chloropyrimidin-4-yl)-3-isopropyl-*N5*-(pentan-3-yl)pyrazolo[1,5-*a*]pyrimidine-5,7-diamine;
**(35)** *N5*-(2-ethylbutyl)-3-isopropyl-*N7*-(pyridin-2-yl)pyrazolo[1,5-*a*]pyrimidine-5,7-diamine;
**(36)** *N5*-(2-ethylbutyl)-3-isopropyl-*N7*-(pyridin-3-yl)pyrazolo[1,5-*a*]pyrimidine-5,7-diamine;
**(37)** *N5*-(2-ethylbutyl)-3-isopropyl-*N7*-(2-methoxypyrimidin-4-yl)pyrazolo[1,5-*a*]pyrimidine-5,7-diamine;
**(38)** *N5*-(2-ethylbutyl)-3-isopropyl-*N7*-(6-methoxypyridazin-3-yl)pyrazolo[1,5-*a*]pyrimidine-5,7-diamine;
**(39)** 3-cyclopropyl-*N5*-(pentan-3-yl)-*N7*-(2-(pyridin-2-yl)ethyl)pyrazolo[1,5-*a*]pyrimidine-5,7-diamine;
**(40)** 3-cyclopropyl-*N5*-(pentan-3-yl)-*N7*-(pyridin-3-yl)pyrazolo[1,5-*a*]pyrimidine-5,7-diamine;
**(41)** 3-cyclopropyl-*N5*-(pentan-3-yl)-*N7-*(pyridin-2-yl)pyrazolo[1,5-*a*]pyrimidine-5,7-diamine;
**(42)** 3-cyclopropyl-*N5*-(pentan-3-yl)-*N7*-(pyrimidin-4-yl)pyrazolo[1,5-*a*]pyrimidine-5,7-diamine;
**(43)** 3-cyclopropyl-*N5*-(pentan-3-yl)-*N7*-(pyridazin-3-yl)pyrazolo[1,5-a]pyrimidine-5,7-diamine;
**(44)** 3-cyclopropyl-*N5*-(pentan-3-yl)-*N7*-(pyrazin-2-yl)pyrazolo[1,5-*a*]pyrimidine-5,7-diamine;
**(45)** 3-cyclopropyl-*N5*-(pentan-3-yl)-*N7*-(pyrimidin-5-yl)pyrazolo[1,5-a]pyrimidine-5,7-diamine;
**(46)** (*S*)-2-((3-cyclopropyl-7-(pyridazin-3-ylamino)pyrazolo[1,5-*a*]pyrimidin-5-yl)amino)butan-1-ol;
**(47)** 3-cyclopropyl-*N7*-(6-methoxypyridin-2-yl)-*N5*-(pentan-3-yl)pyrazolo[1,5-*a*]pyrimidine-5,7-diamine;
**(48)** 3-cyclopropyl-*N7*-(6-methoxypyridazin-3-yl)-*N5*-(pentan-3-yl)pyrazolo[1,5-*a*]pyrimidine-5,7-diamine;
**(49)** 3-cyclopropyl-*N7*-methyl-*N5*-(pentan-3-yl)-*N7*-(pyridazin-3-yl)pyrazolo[1,5-*a*]pyrimidine-5,7-diamine;
**(50)** 3-cyclopropyl-*N5*-(2-ethylbutyl)-*N7*-(pyridazin-3-yl)pyrazolo[1,5-*a*]pyrimidine-5,7-diamine;
**(51)** 3-cyclopropyl-*N5*-(2-ethylbutyl)-*N7*-(6-methoxypyridazin-3-yl)pyrazolo[1,5-*a*]pyrimidine-5,7-diamine;
**(52)** 3-cyclopropyl-*N5*-(2-ethylbutyl)-*N7*-(2-methoxypyrimidin-4-yl)pyrazolo[1,5-*a*]pyrimidine-5,7-diamine;
**(53)** 3-isopropyl-*N7*-(4-methoxybenzyl)-*N5*-(pentan-3-yl)pyrazolo[1,5-*a*]pyrimidine-5,7-diamine;
**(54)** *N*-(3-isopropyl-5-(pentan-3-ylamino)pyrazolo[1,5-*a*]pyrimidin-7-yl)-2-phenylacetamide
(55) *N*-(3-isopropyl-5-(pentan-3-ylamino)pyrazolo[1,5-*a*]pyrimidin-7-yl)-2-(pyridin-2-yl)acetamide;
(56) *N*-(3-isopropyl-5-(pentan-3-ylamino)pyrazolo[1,5-*a*]pyrimidin-7-yl)-2-(pyridin-3-yl)acetamide;
(57) *N*-(3-isopropyl-5-(pentan-3-ylamino)pyrazolo[1,5-*a*]pyrimidin-7-yl)-2-(pyridin-4-yl)acetamide;
(58) *N7*-benzyl-3-isopropyl-*N5*-(1-methyl-1*H*-pyrazol-5-yl)pyrazolo[1,5-*a*]pyrimidine-5,7-diamine; and their pharmaceutically acceptable salts.

Thus, herein is provided anyone of the compounds (1) to (58) as defined above or any of their pharmaceutically acceptable salts.

According to an even more preferred embodiment of the present invention, the compound of formula (I) is chosen from the group consisting of compounds (1) to (58) and their pharmaceutically acceptable salts.

According to an even more preferred embodiment of the present invention, the compound of formula (I) is chosen from the group consisting of compounds (2) to (48) and (50) to (57) and their pharmaceutically acceptable salts.

According to an even more particularly preferred embodiment of the present invention, the compound of formula (I) is chosen from the group consisting of compounds (5) to (10), (13), (16), (18) to (34), (36) to (48), (50) to (52) and (54) to (57) and their pharmaceutically acceptable salts.

According to another aspect, a subject-matter of the present invention relates to a compound of formula (I) as defined above or any of its pharmaceutically acceptable salts, or at least any of compounds (1) to (58) or any of their pharmaceutically acceptable salts, for use as a medicament.

"Pharmaceutically acceptable salt thereof' refers to salts which are formed from acid addition salts formed with inorganic acids (*e.g*. hydrochloric acid, hydrobromic acid,), as well as salts formed with organic acids such as acetic acid, tartaric acid, succinic acid.

Suitable physiologically acceptable acid addition salts of compounds of formula (I) include hydrobromide, tartrate, hydrochloride, mesylate, succinate and acetate.

The compounds of formula (I), and any of compounds (1) to (58) or any of their pharmaceutically acceptable salts may form solvates or hydrates and the invention includes all such solvates and hydrates.

The terms "hydrates" and "solvates" simply mean that the compounds (I) according to the invention can be in the form of a hydrate or solvate, *i.e.* combined or associated with one or more water or solvent molecules. This is only a chemical characteristic of such compounds, which can be applied for all organic compounds of this type.

The compounds of formula (I) can comprise one or more asymmetric carbon atoms. They can thus exist in the form of enantiomers or of diastereoisomers. These enantiomers, diastereoisomers and their mixtures, including the racemic mixtures, are encompassed within the scope of the present invention.

The compounds of the present invention can be prepared by conventional methods of organic synthesis practiced by those skilled in the art. The general reaction sequences outlined below represent a general method useful for preparing the compounds of the present invention and are not meant to be limiting in scope or utility.

### List of abbreviations:

| **Abbreviation/acronym** | **Name** |
|---|---|
| **A₁AR** | Adenosine receptor A₁ |
| **A_{2A}AR** | Adenosine receptor A_{2A} |
| **A_{2B}AR** | Adenosine receptor A_{2B} |
| **A₃AR** | Adenosine receptor A₃ |
| **ACN** | Acetonitrile |
| **AcOH** | Acetic acid |
| **Alk** | Alkyl |
| **ATP** | Adenosine triphosphate |
| **br s** | Broad singlet |
| **nBuOH** | Butan-1-ol |
| **C** | Molar concentration |
| **cHex** | Cyclohexane |
| **Cpd N°** | Compound number |
| **d** | Doublet |
| **DCM** | Methylene chloride |
| **dd** | Doublet of doublets |
| **DiPEA** | *N,N*-Diisopropylethylamine |
| **DMF** | Dimethylformamide |
| **DMSO** | Dimethylsulfoxide |
| **eq** | Equivalent |
| **ESI** | Electrospray ionization |
| **Et** | Ethyl |
| **EtOAc** | Ethyl Acetate |
| **GP** | General protocol |
| **Hal** | Halogen |
| **HPLC** | High pressure liquid chromatography |
| **IC₅₀** | Half maximal inhibitory concentration |
| **iPr** | isopropyl |
| **iPrOH** | Isopropanol |
| **KHMDS** | Potassium bis(trimethylsilyl)amide |
| **m** | Multiplet |
| **M** | Molarity |
| **Me** | Methyl |
| **2MePrOH** | 2-Methylpropan-1-ol |
| **2-MeTHF** | 2-Methyltetrahydrofuran |
| **MS** | Mass spectroscopy |
| **MW** | Molecular weight |
| **NMP** | *N*-Methyl-2-pyrrolidone |
| **NMR** | Nuclear magnetic resonance |
| **N/A** | Not applicable |
| **n.t.** | Not tested |
| **Phe** | phenyl |
| **nPrOH** | Propan-1-ol |
| **r.t.** | Room temperature |
| **s** | Singlet |
| **t** | Triplet |
| **TEA** | Triethylamine |
| **TFA** | Trifluoroacetic acid |
| **THE** | Tetrahydrofuran |
| **TLC** | Thin layer chromatography |
| **Ts** | Tosyl |
| **T °C** | Temperature in degrees Celsius |
| **UV** | Ultraviolet |
| **v/v** | Volume per volume |
| **w/v** | Weight per volume |
| **Δ** | chemical shift |
| **µw** | Microwave irradiation |

The compounds of general formula (I) can be prepared according to **Route 1** depicted in scheme 1 below.

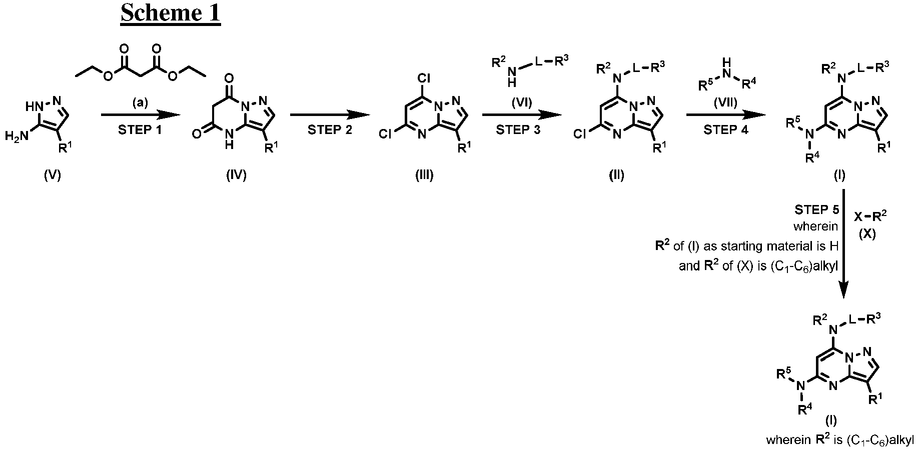

According to Route 1, the synthesis of compounds (I) according to the invention may be based on a functionalization of a compound of formula (II) wherein L, R¹, R² and R³ are as defined above by an amine of formula (VII) wherein R⁴ and R⁵ are as defined above. The compound of formula (II) may be placed in STEP 4 in an aprotic solvent such as Et₃N, DiPEA, THF, 2-MeTHF, dioxane, anisol, cineol, NMP, mixtures thereof, or without solvent. The amine of formula (VII) may be added, for example in a molar ratio ranging from 1 to 20 eq, in particular of 8 eq, with respect to the compound of formula (II). The reaction mixture may be heated at a temperature between 100 to 180°C. Upon completion of the reaction, the mixture may be brought back to room temperature.

A compound of formula (II) as defined above may be obtained in STEP 3 by reaction between a compound of formula (III) wherein R¹ is as defined above and an amine of formula (VI) wherein L, R² and R³ are as defined above, in an aprotic solvent such as THF, 2-MeTHF, dioxane, or mixtures thereof, with a base such as Et₃N, NaH or tBuOK, or without base. The amine of formula (VI) and base may be added, for example in a molar ratio ranging from 1 to 3 eq, in particular of 1.5 eq, with respect to the compound of formula (III). The resulting mixture may then be stirred, for example from 1 to 24 hours, in particular for 5 hours at a temperature between r.t. to 100 °C by refluxing or in a sealed tube. Upon completion of the reaction, the mixture may be brought back to room temperature.

A compound of formula (III) as defined above may be obtained in STEP 2 by reaction of a compound of formula (IV) wherein R¹ is as defined above with POCl₃ and *N,N-*dimethylaniline. POCl₃ reagent may be added, for example in a molar ratio ranging from 10 to 30 eq, in particular of 15 eq and *N,N*-dimethylaniline in a molar ratio ranging from 1 to 5 eq, in particular of 2.1 eq, with respect to the compound of formula (IV). The reaction mixture may be stirred at reflux temperature or in sealed tube without solvent for a duration ranging from 1 to 24 hours, for example for 5.5 hours. Upon completion of the reaction, the mixture may be brought back to room temperature.

A compound of formula (IV) as defined above may be obtained in STEP 1 by reaction of a compound of formula (V) wherein R¹ is as defined above with a diethyl malonate (a) and a base. The compound of formula (V) may be placed in STEP 1 in a protic solvent such as MeOH, EtOH iPrOH, or mixtures thereof. (a) may be added, for example in a molar ratio ranging from 1 to 2 eq, in particular of 1.02 eq, and base such as EtONa 21% in EtOH may be added, for example in a molar ratio ranging from 1 to 2 eq, in particular of 1.1 eq, with respect to the compound of formula (V). The reaction mixture may be heated at a temperature between 100 to 150 °C, in particular 130 °C, by refluxing or in a sealed tube or in a microwave reactor. Upon completion of the reaction, the mixture may be brought back to room temperature.

Also according to Route 1, the synthesis of compounds (I) according to the invention wherein R² is a (C₁-C₆)alkyl, R¹, R⁴ and R⁵ are as defined above, may be obtained by further reacting in STEP 5 other compounds (I) wherein R² is a hydrogen atom, R¹, R⁴ and R⁵ are as defined above with a compound of formula (X) wherein R² is a (C₁-C₆)alkyl and X represents a leaving group such as a halogen atom, in particular a fluorine atom, a bromine atom or a iodine atom, more particularly a iodine atom.

The compounds of general formula (I) can alternatively be prepared according to **Route 2,** depicted in scheme 2 below.

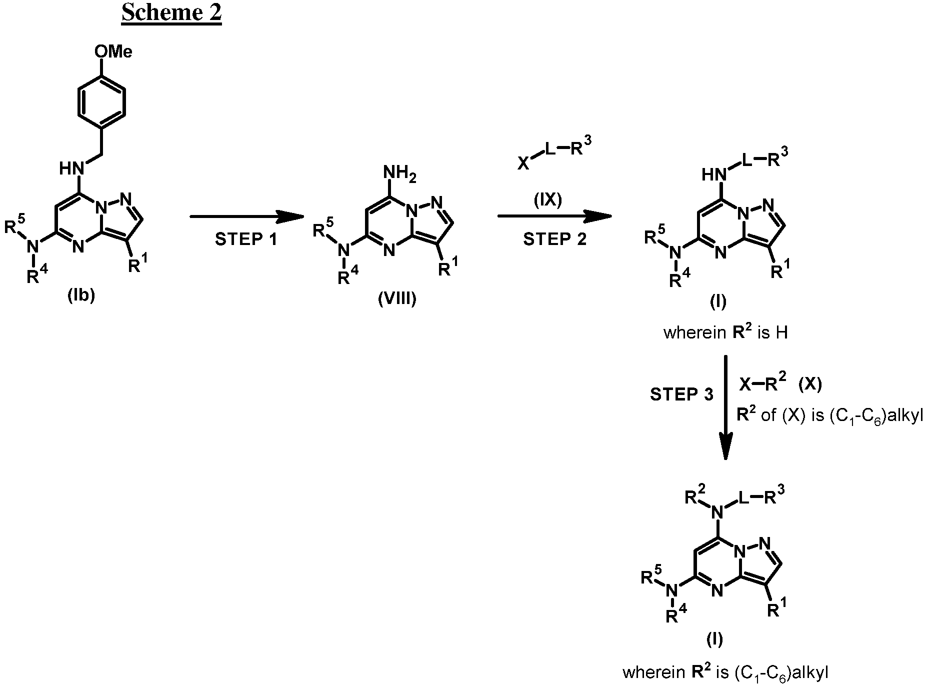

According to Route 2, the synthesis of compounds (I) according to the invention may be based on a functionalization of a compound of formula (VIII) wherein R² is a hydrogen atom, R¹, R⁴ and R⁵ are as defined above by a reagent of formula (IX) wherein L and R³ are as defined above and X represents a hydroxy group, a chlorine atom, a bromine atom, an (C₁-C₃)alkyl sulfonate group or a phenyl sulfonate group.

The compound of formula (VIII) may be placed, in STEP 2, in an aprotic solvent such as THF, 2-MeTHF, dioxane, cineol, DMF, NMP or mixtures thereof. The compound of formula (IX) may be added, for example in a molar ratio ranging from 1 to 3 eq, in particular of 1.1 eq, with respect to the compound of formula (VIII). The reaction mixture may be stirred at room temperature or 50 to 110 °C by refluxing or in a sealed tube for a duration ranging from 1 to 24 hours. Upon completion of the reaction, the mixture may be brought back to room temperature.

Also according to Route 2, the synthesis of compounds (I) according to the invention wherein R² is a (C₁-C₆)alkyl, R¹, R⁴ and R⁵ are as defined above, may be obtained by further reacting in STEP 3 other compounds (I) wherein R² is a hydrogen atom, R¹, R⁴ and R⁵ are as defined above with a compound of formula (X) wherein R² is a (C₁-C₆)alkyl and X represents a leaving group such as a halogen atom, in particular a fluorine atom, a bromine atom or a iodine atom, more particularly a iodine atom.

A compound of formula (VIII) as defined above may be obtained in STEP 1 by deprotection of para-methoxybenzyl group of a compound of formula (Ib) wherein R¹, R⁴ and R⁵ is as defined above, in an aprotic solvent such as THF, 2-MeTHF, dioxane, anisole or mixtures thereof. Such deprotection may be performed in an aprotic solvent with AlCl₃ or more particulary trifluoroacetic acid in a volume ratio 1:10 to 1:1, v:v, with respect to the compound of formula (VIII). The reaction mixture may be stirred at room temperature or 50 to 130 °C by refluxing or in a microwave reactor, for a duration ranging from 0.5 to 24 hours. Upon completion of the reaction, the mixture may be brought back to room temperature.

A compound of formula (Ib) is encompassed by compound of formula (I) and may be obtained according to route 1 of scheme 1 as described above.

Accordingly, herein is further provided a synthesis process for manufacturing a compound of formula (I) as defined above or any of its pharmaceutically acceptable salts, or at least any of compounds (1) to (58) as defined above or any of their pharmaceutically acceptable salts, comprising at least a step of reacting a compound of formula (II) 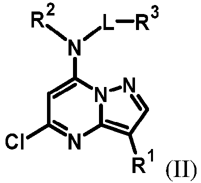
wherein L, R¹, R² and R³ are as defined above with an amine of formula (VII) 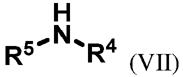
wherein R⁴ and R⁵ are as defined above, in particular in an aprotic solvent, protic solvent or without solvent, in particular in a molar ratio ranging from 1 to 20 eq with respect to the compound of formula (II).

In a particular embodiment, compound of formula (II) as defined above may be obtained by a step of reacting a compound of formula (III) 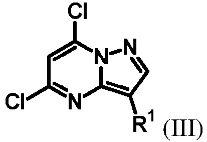
wherein R¹ is as defined above, with an amine of formula (VI) 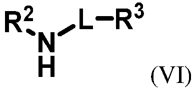
wherein L, R² and R³ are as defined above, in particular in an aprotic solvent, in particular in a molar ratio ranging from 1 to 3 eq and more particularly equal to 1.5 eq with respect to the compound of formula (III).

Herein is further provided another synthesis process for manufacturing a compound of formula (I) as defined above or any of its pharmaceutically acceptable salts, or at least any of compounds (1) to (58) as defined above or any of their pharmaceutically acceptable salts, comprising at least a step of reacting a compound of formula (VIII) 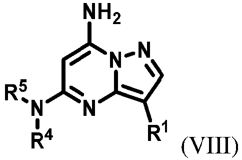
wherein R¹, R⁴ and R⁵ are as defined above, with a compound of formula (IX) 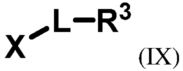
wherein L and R³ are as defined above, and X represents a hydroxy group, a chlorine atom, a bromine atom, an (C₁-C₃)alkyl sulfonate group or a phenyl sulfonate group, in particular in an aprotic solvent for example in a molar ratio ranging from 1 to 10 eq, with respect to the compound of formula (VIII).

In a particular embodiment, compound of formula (VIII) as defined above may be obtained by a step of deprotection of para-methoxybenzyl group of a compound of formula (Ib) 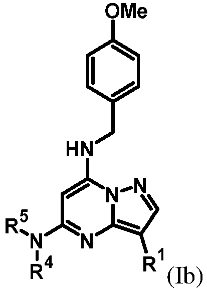 wherein R¹, R⁴ and R⁵ are as defined above, in particular in an aprotic solvent with trifluoroacetic acid and more particularly the trifluoroacetic acid being in a volume ratio of 1:10 to 1:1, with respect to the aprotic solvent.

Herein is further provided a compound of formula (II) or (VIII), in particular as intermediate compounds 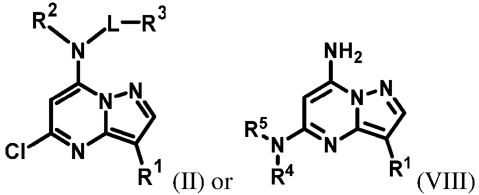 wherein L, R¹, R², R³, R⁴ and R⁵ are as defined above.

Herein is further provided a synthesis process for manufacturing a compound of formula (II) 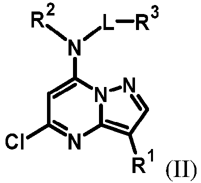
wherein L, R¹, R² and R³ are as defined above,
comprising at least a step of reacting a compound of formula (III) 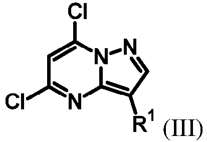
wherein R¹ is as defined above, with an amine of formula (VI) 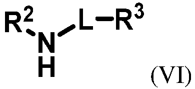
wherein L, R² and R³ are as defined above, in particular in an aprotic solvent, in particular in a molar ratio ranging from 1 to 3 eq and more particularly equal to 1.5 eq with respect to the compound of formula (III).

Herein is further provided a synthesis process for manufacturing a compound of formula (VIII) 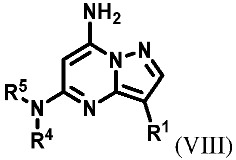
wherein R¹, R⁴ and R⁵ are as defined above,
comprising at least a step of deprotection of para-methoxybenzyl group of a compound of formula (Ib) 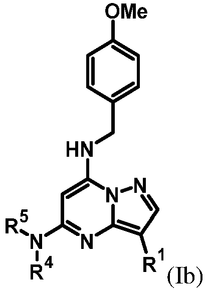
wherein R¹, R⁴ and R⁵ are as defined above, in particular in an aprotic solvent with trifluoroacetic acid and more particularly the trifluoroacetic acid being in a volume ratio of 1:10 to 1: 1, with respect to the aprotic solvent.

The chemical structures, the analytical and spectroscopic data of some compounds of formula (I) of the invention are illustrated respectively in the following **Table 1** and **Table 2.**

Reactions were performed using glassware or oven-dried glassware under inert atmosphere of argon or not. Unless otherwise noted, all reagent-grade chemicals and solvents were obtained from commercial suppliers and were used as received. Reactions were monitored by thin-layer chromatography with silica gel 60 F254 pre-coated aluminum plates (0.25 mm). Visualization was performed under UV light and 254 or 365 nm, or with appropriate TLC stains including, but not limited to: phosphomolybdic acid, KMnO₄, ninhydrin, CAM, vanillin, p-anisaldehyde.

Chromatographic purifications of compounds were achieved on an automated Interchim Puriflash XS420 equipped with 30 µm spherical silica-filled prepacked columns as stationary phase (normal phase) or with C18 silica prepacked columns as stationary phase (reversed phase). Nevertheless, a second purification could be achieved with preparative thin layer chromatography with standard silica. Purifications work with various solvent as pur or mixed such as ethyl acetate, cyclohexane, methanol, methanol with ammonia 7N, dichloromethane, triethylamine and tetrahydrofuran on a normal phase then ACN, MeOH, H₂O and NH₄OH on a reversed phase.
Mixtures possibilities: cHex/EtOAc, cHex/DCM, cHex/DCM/EtOAc, DCM/EtOAc, DCM/MeOH, DCM/MeOH NH₃ 7N, DCM/MeOH/Et₃N, DCM/MeOH/THF, EtOAc/MeOH, EtOAc/MeOH NH₃ 7N, EtOAc/THF, ACN/H₂O, ACN/NH₄OH, MeOH/H₂O, MeOH/NH₄OH.

Some compounds of the invention are described with their structure in the below **Table 1,** which is merely illustrative and does not limit the scope of the present invention.

**Table 1: Structure of compounds (1) to (58). Formulas and molecular weights were generated using Perkin Elmer's ChemDraw^{®} Professional v22.0.0.22.**

| 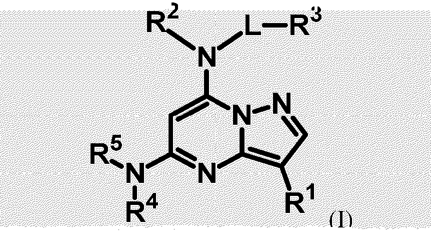 | | | | | | |
|---|---|---|---|---|---|---|
| **Cpd N°** | **NR²LR³** | **R¹** | **NR⁴R⁵** | **Formula** | **MW** | **Stereochemistry** |
| **(1)** | 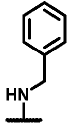 | 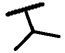 | 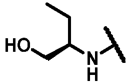 | C₂₀H₂₇N₅O | 353.47 | (R) |
| **(2)** | 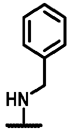 | 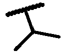 | 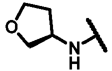 | C₂₀H₂₅N₅O | 351.45 | (R) |
| **(3)** | 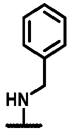 | 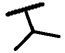 | 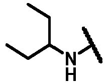 | C₂₁H₂₉N₅ | 351.5 | - |
| **(4)** | 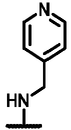 | 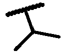 | 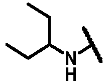 | C₂₀H₂₈N₆ | 352.49 | - |
| **(5)** | 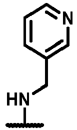 | 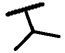 | 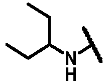 | C₂₀H₂₈N₆ | 352.49 | - |
| **(6)** | 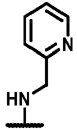 | 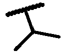 | 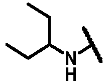 | C₂₀H₂₈N₆ | 352.49 | - |
| **(7)** | 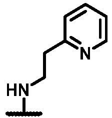 | 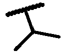 | 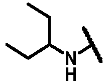 | C₂₁H₃₀N₆ | 366.51 | - |
| **(8)** | 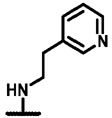 | 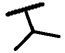 | 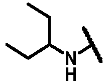 | C₂₁H₃₀N₆ | 366.51 | - |
| **(9)** | 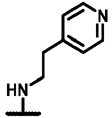 | 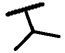 | 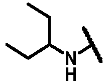 | C₂₁H₃₀N₆ | 366.51 | - |
| **(10)** | 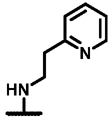 | 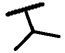 | 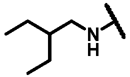 | C₂₂H₃₂N₆ | 380.54 | - |
| **(11)** | 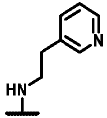 | 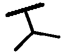 | 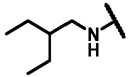 | C₂₂H₃₂N₆ | 380.54 | - |
| **(12)** | 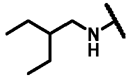 | 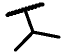 | 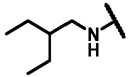 | C₂₁H₃₇N₅ | 359.56 | - |
| **(13)** | 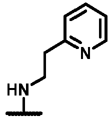 | 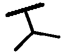 | 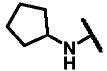 | C₂₁H₂₈N₆ | 364.50 | - |
| **(14)** | 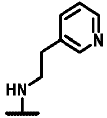 | 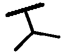 | 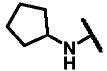 | C₂₁H₂₈N₆ | 364.50 | - |
| **(15)** | 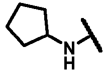 | 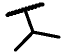 | 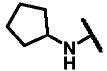 | C₁₉H₂₉N₅ | 327.48 | - |
| **(16)** | 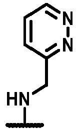 | 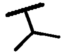 | 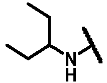 | C₁₉H₂₇N₇ | 353.47 | - |
| **(17)** | 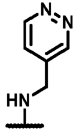 | 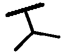 | 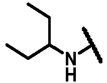 | C₁₉H₂₇N₇ | 353.47 | - |
| **(18)** | 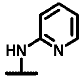 | 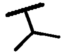 | 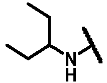 | C₁₉H₂₆N₆ | 338.46 | - |
| **(19)** | 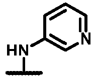 | 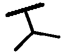 | 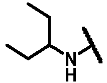 | C₁₉H₂₆N₆ | 338.46 | - |
| **(20)** | 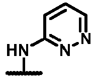 | 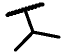 | 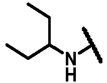 | C₁₈H₂₅N₇ | 339.45 | - |
| **(21)** | 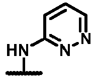 | 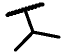 | 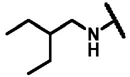 | C₁₉H₂₇N₇ | 353.47 | - |
| **(22)** | 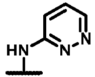 | 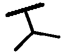 | 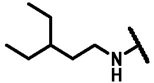 | C₂₀H₂₉N₇ | 367.50 | - |
| **(23)** | 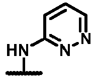 | 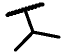 | 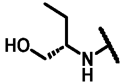 | C₁₇H₂₃N₇O | 341.42 | (S) |
| **(24)** | 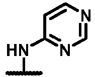 | 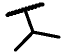 | 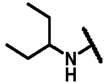 | C₁₈H₂₅N₇ | 339.45 | - |
| **(25)** | 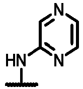 | 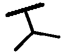 | 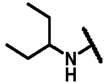 | C₁₈H₂₅N₇ | 339.45 | - |
| **(26)** | 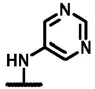 | 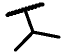 | 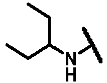 | C₁₈H₂₅N₇ | 339.45 | - |
| **(27)** | 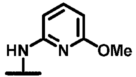 | 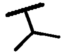 | 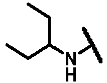 | C₂₀H₂₈N₆O | 368.49 | - |
| **(28)** | 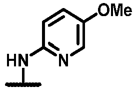 | 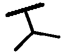 | 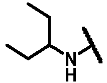 | C₂₀H₂₈N₆O | 368.49 | - |
| **(29)** | 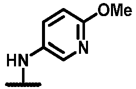 | 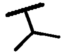 | 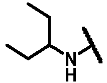 | C₂₀H₂₈N₆O | 368.49 | - |
| **(30)** | 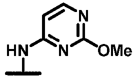 | 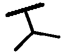 | 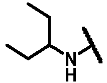 | C₁₉H₂₇N₇O | 369.47 | - |
| **(31)** | 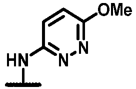 | 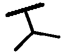 | 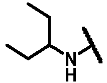 | C₁₉H₂₇N₇O | 369.47 | - |
| **(32)** | 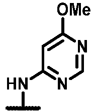 | 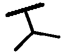 | 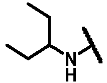 | C₁₉H₂₇N₇O | 369.47 | - |
| **(33)** | 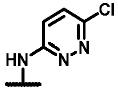 | 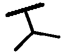 | 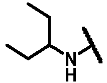 | C₁₈H₂₄ClN₇ | 373.89 | - |
| **(34)** | 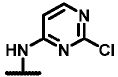 | 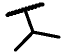 | 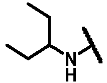 | C₁₈H₂₄ClN₇ | 373.89 | - |
| **(35)** | 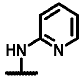 | 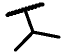 | 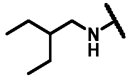 | C₂₀H₂₈N₆ | 352.49 | - |
| **(36)** | 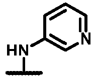 | 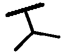 | 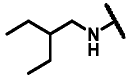 | C₂₀H₂₈N₆ | 352.49 | - |
| **(37)** | 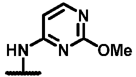 | 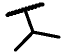 | 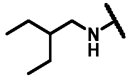 | C₂₀H₂₉N₇O | 383.50 | - |
| **(38)** | 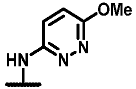 | 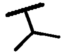 | 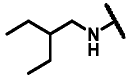 | C₂₀H₂₉N₇O | 383.50 | - |
| **(39)** | 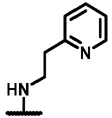 | 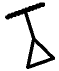 | 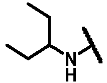 | C₂₁H₂₈N₆ | 364.50 | - |
| **(40)** | 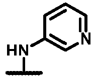 | 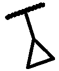 | 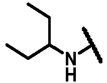 | C₁₉H₂₄N₆ | 336.44 | - |
| **(41)** | 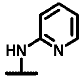 | 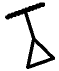 | 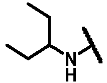 | C₁₉H₂₄N₆ | 336.44 | - |
| **(42)** | 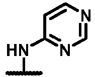 | 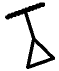 | 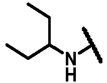 | C₁₈H₂₃N₇ | 337.43 | - |
| **(43)** | 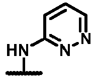 | 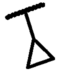 | 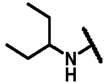 | C₁₈H₂₃N₇ | 337.43 | - |
| **(44)** | 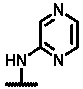 | 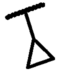 | 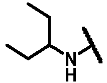 | C₁₈H₂₃N₇ | 337.43 | - |
| **(45)** | 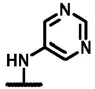 | 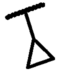 | 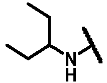 | C₁₈H₂₃N₇ | 337.43 | - |
| **(46)** | 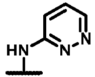 | 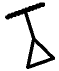 | 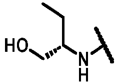 | C₁₇H₂₁N₇O | 339.40 | (S) |
| **(47)** | 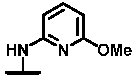 | 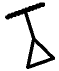 | 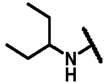 | C₂₀H₂₆N₆O | 366.47 | - |
| **(48)** | 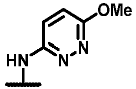 | 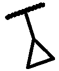 | 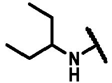 | C₁₉H₂₅N₇O | 367.46 | - |
| **(49)** | 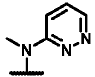 | 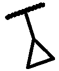 | 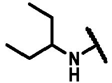 | C₁₉H₂₅N₇ | 351.46 | - |
| **(50)** | 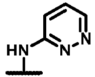 | 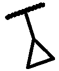 | 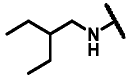 | C₁₉H₂₅N₇ | 351.46 | - |
| **(51)** | 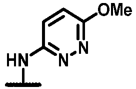 | 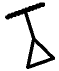 | 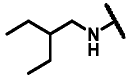 | C₂₀H₂₇N₇O | 381.48 | - |
| **(52)** | 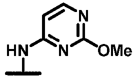 | 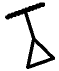 | 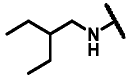 | C₂₀H₂₇N₇O | 381.48 | - |
| **(53)** | 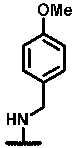 | 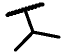 | 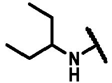 | C₂₂H₃₁N₅O | 381.52 | - |
| **(54)** | 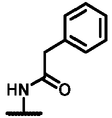 | 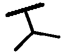 | 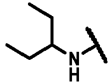 | C₂₂H₂₉N₅O | 379.51 | - |
| **(55)** | 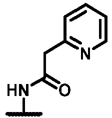 | 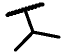 | 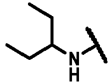 | C₂₁H₂₈N₆O | 380.50 | - |
| **(56)** | 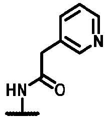 | 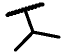 | 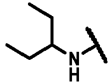 | C₂₁H₂₈N₆O | 380.50 | - |
| **(57)** | 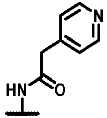 | 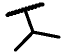 | 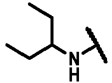 | C₂₁H₂₈N₆O | 380.50 | - |
| **(58)** | 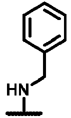 | 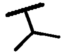 | 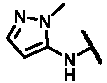 | C₂₀H₂₃N₇ | 361.45 | - |

The below **Table 2** describes the analytical and spectroscopic data of the compounds introduced in **Table 1.**

¹H NMR (400 MHz) and ¹³C NMR (101 MHz) spectra were recorded with a Bruker ULTRASHIELD 400 spectrometer. Processing and analyses of the spectra were performed with MestReNova. Data appear in the following order: chemical shifts in ppm which were referenced to the internal solvent signal, multiplicity, number of protons, and coupling constant *J* in Hertz.

Synthetic intermediates: reversed-phase UPLC/MS analyses were carried out with a UPLC Acquity (Waters) with an UV-DAD detector and a mass detector (SQD2). Compounds (0.2 to 0.6 mg) were solubilized in a mixture of DMSO/H₂O (111) and filtered on syringe filter 0.2 µm.
- Acidic conditions:
   Acquity BEH C18 column, 2.1 × 50 mm, 1.7 µm. Flow rate: 0.65 mL/min. Gradient: (H₂O + 0.1% HCOOH v/v)/(CAN + 0.1% HCOOH v/v) from 95/5 to 5/95 in 4.0 min.
   Final compounds: Reversed-phase HPLC/MS analyses were carried out with a HPLC Ultimate 3000 (ThermoScientific) with an UV-DAD detector. Mass detection processing with direct infusion in mass detector (SQD2) from UPLC Acquity (Waters). Compounds (0.2 to 0.6 mg) were solubilized in a mixture of DMSO/H₂O (111) and filtered on syringe filter 0.2 µm.
- Acidic conditions:
   Thermo Scientific Syncronis C18 column, 150 × 4.6 mm, 5 µm. Flow rate: 1 mL/min. Gradient: (H₂O + 0.1% HCOOH v/v)/(ACN + 0.1% HCOOH v/v) from 95/5 to 5/95 in 19.0 min.
- Alkaline conditions:
   Thermo Scientific Syncronis C18 column, 150 × 4.6 mm, 5 µm or XTERRA RP18, 150x4.6mm, 3.5µm. Flow rate: 1 mL/min. Gradient: (HCOONH₄ 10mM + NH₄OH 25% aq. to ajust pH = 10)/ACN from 95/5 to 5/95 in 19.0 min.

**Table 2: Spectroscopic and analytical characterization of compounds (1) to (58)**

| **Cpd N°** | **Structure** | **HPLC** | **Description** |
|---|---|---|---|
| (1) | 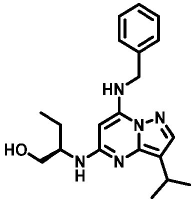 | > 98 % ^{a} | White solid. |
| | | | Rf (DCM/MeOH, 96/4) : 0.23. |
| | | | **¹H NMR (400 MHz, DMSO-*d₆*) δ :** 0.84 (t, *J =* 7.4 Hz, 3H, CH_{3-CH2}), 1.24 (d, *J =* 6.9 Hz, 6H, 2×CH_{3 iPr}), 1.32 - 1.66 (m, 2H, CH_{2-CH3}), 2.93 (hept, *J* = 6.9 Hz, 1H, H_{iPr}), 3.40 (dp, *J* = 19.2, 5.1 Hz, 2H, CH_{2-OH}), 3.81 (s, 1H, H_{C-NH}), 4.35 - 4.52 (m, 2H, CH_{2 Bn}), 4.71 (t, *J =* 5.3 Hz, 1H, OH), 5.18 (s, 1H, H_{Ar}), 6.35 (d, *J =* 7.8 Hz, 1H, NH), 7.19 - 7.40 (m, 5H, 5×H_{Ar}), 7.60 (s, 1H, H_{Ar}), 7.76 (t, *J =* 6.6 Hz, 1H, NH_{Bn}). |
| | | | **¹³C NMR (101 MHz, DMSO-*d₆*) δ :** 10.6 (CH_{3-CH2}), 23.1 (CH_{3 iPr}), 23.1 (CH_{3 iPr}), 23.4 (CH_{iPr}), 23.9 (CH_{2-CH3}), 44.6 (CH_{2 Bn}), 53.4 (CH_{Ar}), 63.1 (CH_{2-OH}), 72.7 (CH_{Ar}), 110.4 (C_{q}), 126.8 (2xCH_{Ar}), 126.9 (CH_{Ar}), 128.4 (2×CH_{Ar}), 138.6 (C_{q}), 139.6 (CH_{Ar}), 145.3 (C_{q}), 146.0 (C_{q}), 156.5 (C_{q}). |
| | | | **MS (ESI+) : m/z calcd for C₂₀H₂₇N₅O** : 354.23 [M+H]+, **found** : 354.31. |
| (2) | 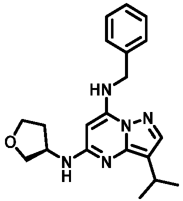 | > 99 % ^{a} | **¹H NMR (400 MHz, DMSO-*d₆*) δ :** 7.83 (t, *J =* 6.4 Hz, 1H), 7.62 (s, 1H), 7.44 - 7.30 (m, 4H), 7.30 - 7.18 (m, 1H), 6.82 (d, *J =* 5.8 Hz, 1H), 5.11 (s, 1H), 4.44 (d, *J* = 6.4 Hz, 2H), 4.39 - 4.26 (m, 1H), 3.86 (dd, *J =* 8.8, 5.9 Hz, 1H), 3.77 (q, *J =* 7.6 Hz, 1H), 3.67 (td, *J =* 8.1, 5.8 Hz, 1H), 3.45 (dd, *J =* 8.8, 4.2 Hz, 1H), 2.96 (hept, *J* = 6.9 Hz, 1H), 2.18 - 2.06 (m, 1H), 1.80 - 1.70 (m, 1H), 1.25 (dd, *J =* 6.9, 1.5 Hz, 6H). |
| | | | **¹³C NMR (101 MHz, DMSO-*d₆*) δ :** 155.92, 146.13, 145.36, 139.67, 138.52, 128.44 (2C), 126.96, 126.71 (2C), 110.78, 73.00, 72.63, 66.30, 51.11, 44.60, 32.13, 23.49, 23.08, 23.03. |
| | | | **MS (ESI+) : m/z calcd for C₂₁H₂₇N₅O** : 352.21 [M+H]+, **found** : 352.28. |
| (3) | 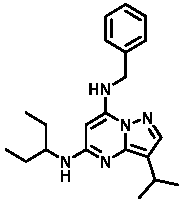 | > 94 % ^{a} | White solid. |
| | | | **¹H NMR (400 MHz, DMSO-*d₆*) δ :** 0.81 (t, *J =* 7.4 Hz, 6H, 2×CH_{3-CH2}), 1.24 (d, *J =* 6.9 Hz, 6H, 2×CH_{3 iPr}), 1.33 - 1.55 (m, 4H, 2×CH_{2-CH3}), 2.93 (hept, *J* = 6.9 Hz, 1H, H_{iPr}), 3.76 (s, 1H, H_{iPent}), 4.44 (d, *J* = 6.4 Hz, 2H, CH_{2 Bn}), 5.13 (s, 1H, H_{Ar}), 6.32 (d, *J* = 8.2 Hz, 1H, NH), 7.18 - 7.41 (m, 5H, 5×H_{Ar}), 7.58 (s, 1H, H_{Ar}), 7.70 (t, *J* = 6.4 Hz, 1H, NH_{Bn}). |
| | | | **¹³C NMR (101 MHz, DMSO-*d₆*) δ :** 10.4 (2xCH_{3-CH2}), 23.0 (2xCH_{3 iPr}), 23.5 (CH_{iPr}), 26.6 (2×CH_{2-CH3}), 44.6 (CH_{2 Bn}), 52.2 (CH_{iPent}), 72.5 (CH_{Ar}), 110.2 (C_{q}), 126.8 (2xCH_{Ar}), 126.9 (CH_{Ar}), 128.4 (2xCH_{Ar}), 138.6 (C_{q}), 139.5 (CH_{Ar}), 145.6 (C_{q}), 146.0 (C_{q}), 156.6 (C_{q}). |
| | | | MS **(ESI+) : m/z calcd for C₂₁H₂₉N₅O** : 352.25 [M+H]+, **found** : 352.35. |
| (4) | 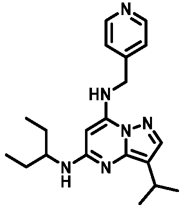 | > 98 % ^{a} | white solid. |
| | | | **¹H NMR (400 MHz, DMSO-*d₆*) δ :** 0.80 (t, *J =* 7.4 Hz, 6H, 2xCH_{3-CH2}), 1.25 (d, *J =* 6.9 Hz, 6H, 2×CH_{3 iPr}), 1.30 - 1.58 (m, 4H, 2×CH_{2-CH3}), 2.94 (hept, *J* = 6.9 Hz, 1H, H_{iPr}), 3.76 (s, 1H, H_{iPent}), 4.48 (d, *J* = 6.5 Hz, 2H, CH_{2 Bn}), 5.04 (s, 1H, H_{Ar}), 6.33 (d, *J* = 8.2 Hz, 1H, NH), 7.29 - 7.37 (m, 2H, 2×H_{Ar}), 7.60 (s, 1H, H_{Ar}), 7.81 (t, *J* = 6.5 Hz, 1H, NH_{Bn}), 8.47 - 8.57 (m, 2H, 2×H_{Ar}). **¹³C NMR (101 MHz, DMSO-*d₆*) δ :** 10.4 (2×CH_{3-CH2}), 23.0 (2xCH_{3 iPr}), 23.5 (CH_{iPr}), 26.5 (2×CH_{2-CH3}), 43.7 (CH_{2 Bn}), 52.2 (CH_{iPent}), 72.7 (CH_{Ar}), 110.3 (C_{q}), 121.8 (2xCH_{Ar}), 139.7 (CH_{Ar}), 145.6 (C_{q}), 145.9 (C_{q}), 147.8 (C_{q}), 149.7 (2xCH_{Ar}), 156.5 (C_{q}). |
| | | | **MS (ESI+) : m/z calcd for C₂₀H₂₈N₆ :** 353.49 [M+H]+, **found** : 353.32. |
| (5) | 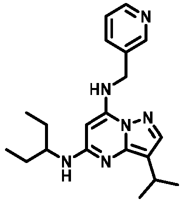 | > 98 % ^{a} | Light brown solid. |
| | | | **¹H NMR (400 MHz, DMSO-*d₆*) δ :** 0.81 (t, *J =* 7.4 Hz, 6H, 2xCH_{3-CH2}), 1.24 (d, *J =* 6.9 Hz, 6H, 2×CH_{3 iPr}), 1.45 (dtt, *J* = 37.4, 13.9, 6.5 Hz, 4H, 2×CH_{2-CH3}), 2.93 (hept, *J* = 7.0 Hz, 1H, H_{iPr}), 3.77 (s, 1H, H_{iPent}), 4.46 (d, *J* = 6.5 Hz, 2H, CH_{2 Bn}), 5.17 (s, 1H, H_{Ar}), 6.34 (d, *J* = 8.2 Hz, 1H, NH), 7.36 (dd, *J* = 7.9, 4.7 Hz, 1H, H_{Ar}), 7.58 (s, 1H, H_{Ar}), 7.71 - 7.86 (m, 2H, NH_{Bn} & H_{Ar}), 8.47 (dd, *J =* 4.8, 1.6 Hz, 1H, H_{Ar}), 8.61 (d, *J =* 2.2 Hz, 1H, H_{Ar}). |
| | | | **¹³C NMR (101 MHz, DMSO-*d₆*) δ :** 10.4 (2xCH_{3-CH2}), 23.0 (2xCH_{3 iPr}), 23.5 (CH_{iPr}), 26.6 (2×CH_{2-CH3}), 42.3 (CH_{2 Bn}), 52.2 (CH_{iPent}), 72.6 (CH_{Ar}), 110.3 (C_{q}), 123.6 (CH_{Ar}), 134.1 (C_{q}), 134.7 (CH_{Ar}), 139.6 (CH_{Ar}), 145.6 (C_{q}), 145.8 (C_{q}), 148.3 (CH_{Ar}), 148.6 (CH_{Ar}), 156.5 (C_{q}). |
| | | | **MS (ESI+) : m/z calcd for C₂₀H₂₈N₆** : 353.49 [M+H]+, **found** : 353.36. |
| (6) | 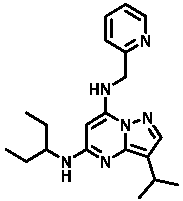 | > 98 % ^{a} | Light brown solid. |
| | | | **¹H NMR (400 MHz, DMSO-*d₆*) δ :** 0.81 (t, *J =* 7.4 Hz, 6H, 2xCH_{3-CH2}), 1.25 (d, *J =* 6.9 Hz, 6H, 2×CH_{3 iPr}), 1.32 - 1.56 (m, 4H, 2×CH_{2-CH3}), 2.94 (hept, *J* = 6.7 Hz, 1H, H_{iPr}), 3.77 (s, 1H, H_{iPent}), 4.53 (d, *J* = 6.1 Hz, 2H, CH_{2 Bn}), 5.14 (s, 1H, H_{Ar}), 6.39 (d, *J* = 8.1 Hz, 1H, NH), 7.29 (dd, *J* = 7.6, 4.9 Hz, 1H, H_{Ar}), 7.35 (d, *J =* 7.9 Hz, 1H, H_{Ar}), 7.60 (s, 1H, H_{Ar}), 7.71 (t, *J* = 6.2 Hz, 1H, NH_{Bn}), 7.77 (td, *J* = 7.7, 1.8 Hz, 1H, H_{Ar}), 8.50 - 8.62 (m, 1H, H_{Ar}). |
| | | | **¹³C NMR (101 MHz, DMSO-*d₆*) δ :** 10.4 (2×CH_{3-CH2}), 23.0 (2xCH_{3 iPr}), 23.5 (CH_{iPr}), 26.5 (2×CH_{2-CH3}), 46.4 (CH_{2 Bn}), 52.2 (CH_{iPent}), 72.7 (CH_{Ar}), 110.3 (C_{q}), 120.8 (CH_{Ar}), 122.3 (CH_{Ar}), 136.9 (CH_{Ar}), 139.6 (CH_{Ar}), 145.5 (C_{q}), 146.0 (C_{q}), 148.9 (CH_{Ar}), 156.6 (C_{q}), 157.5 (C_{q}). |
| | | | **MS (ESI+) : m/z calcd for C₂₀H₂₈N₆** : 353.49 [M+H]+, **found** : 353.32. |
| (7) | 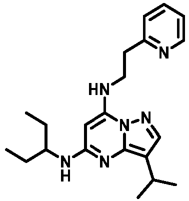 | > 97 % ^{a} | White solid. |
| | | | **¹H NMR (400 MHz, DMSO-*d₆*) δ :** 0.87 (t, *J =* 7.3 Hz, 6H, 2xCH_{3-CH2}), 1.24 (d, *J =* 6.9 Hz, 6H, 2×CH_{3 iPr}), 1.37 - 1.63 (m, 4H, 2×CH_{2-CH3}), 2.93 (dt, *J* = 13.4, 7.0 Hz, 3H, CH₂ & H_{iPr}), 3.45 (q, *J* = 6.9 Hz, 2H, CH₂), 3.84 (s, 1H, H_{iPent}), 5.33 (s, 1H, H_{Ar}), 6.34 (d, *J =* 8.2 Hz, 1H, NH_{-CH}), 7.08 (t, *J =* 6.1 Hz, 1H, NH_{-CH2}), 7.33 (dd, *J =* 7.8, 4.8 Hz, 1H, H_{Ar}), 7.54 (s, 1H, H_{Ar}), 7.71 (d, *J* = 7.8 Hz, 1H, H_{Ar}), 8.43 (dd, *J =* 4.7, 1.7 Hz, 1H, H_{Ar}), 8.52 (d, *J =* 2.3 Hz, 1H, H_{Ar}). |
| | | | **¹³C NMR (101 MHz, DMSO-*d₆*) δ :** 10.4 (2xCH_{3-CH2}), 23.0 (2xCH_{3 iPr}), 23.5 (CH_{iPr}), 26.7 (2×CH_{2-CH3}), 31.3 (CH₂), 42.4 (CH₂), 52.1 (CH_{iPent}), 72.0 (CH_{Ar}), 110.2 (C_{q}), 123.4 (CH_{Ar}), 134.6 (C_{q}), 136.2 (CH_{Ar}), 139.4 (CH_{Ar}), 145.6 (C_{q}), 145.8 (C_{q}), 147.5 (CH_{Ar}), 149.9 (CH_{Ar}), 156.7 (C_{q}). |
| | | | **MS (ESI+) : m/z calcd for C₂₀H₂₈N₆** : 367.26 [M+H]+, **found** : 367.43. |
| (8) | 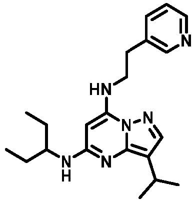 | > 97 % ^{a} | White oil. |
| | | | **¹H NMR (400 MHz, DMSO-*d₆*) δ :** 0.86 (t, *J* = 7.4 Hz, 6H, 2xCH_{3-CH2}), 1.24 (d, *J* = 6.8 Hz, 6H, 2×CH_{3 iPr}), 1.37 - 1.61 (m, 4H, 2×CH_{2-CH3}), 2.93 (hept, *J* = 6.9 Hz, 1H, H_{iPr}), 3.09 (t, *J =* 7.3 Hz, 2H, CH₂), 3.57 (q, *J* = 6.8 Hz, 2H, CH₂), 3.83 (s, 1H, H_{iPent}), 5.32 (s, 1H, H_{Ar}), 6.38 (d, *J* = 8.3 Hz, 1H, NH_{-CH}), 7.06 (t, *J* = 5.9 Hz, 1H, NH_{-CH2}), 7.17 - 7.28 (m, 1H, H_{Ar}), 7.34 (d, *J* = 7.8 Hz, 1H, H_{Ar}), 7.53 (s, 1H, H_{Ar}), 7.73 (td, *J* = 7.6, 1.9 Hz, 1H, H_{Ar}), 8.52 (d, *J* = 4.9 Hz, 1H, H_{Ar}). |
| | | | **¹³C NMR (101 MHz, DMSO-*d₆*) δ :** 10.4 (2×CH_{3-CH2}), 23.0 (2xCH_{3 iPr}), 23.5 (CH_{iPr}), 26.6 (2×CH_{2-CH3}), 36.2 (CH₂), 41.0 (CH₂), 52.1 (CH_{iPent}), 72.0 (CH_{Ar}), 110.2 (C_{q}), 121.6 (CH_{Ar}), 123.3 (CH_{Ar}), 136.6 (CH_{Ar}), 139.4 (CH_{Ar}), 145.6 (C_{q}), 145.9 (C_{q}), 149.1 (CH_{Ar}), 156.7 (C_{q}), 158.9 (C_{q}). |
| | | | **MS (ESI+) : m/z calcd for C₂₀H₂₈N₆** : 367.26 [M+H]+, **found** : 367.43. |
| (9) | 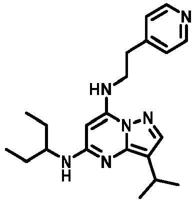 | > 96 % ^{a} | White solid. |
| | | | **¹H NMR (400 MHz, DMSO-*d₆*) δ :** 0.87 (t, *J =* 7.4 Hz, 6H, 2xCH_{3-CH2}), 1.24 (d, *J =* 6.9 Hz, 6H, 2×CH_{3 iPr}), 1.36 - 1.59 (m, 4H, 2×CH_{2-CH3}), 2.94 (dt, *J =* 10.2, 7.2 Hz, 3H, CH₂ & H_{iPr}), 3.41 - 3.54 (m, 2H, CH₂), 3.84 (s, 1H, H_{iPent}), 5.33 (s, 1H, H_{Ar}), 6.35 (d, *J* = 8.2 Hz, 1H, NH_{-CH}), 7.07 (t, *J* = 6.1 Hz, 1H, NH_{-CH2}), 7.27 - 7.37 (m, 2H, 2×H_{Ar}), 7.54 (s, 1H, H_{Ar}), 8.43 - 8.55 (m, 2H, 2×H_{Ar}). |
| | | | **¹³C NMR (101 MHz, DMSO-*d₆*) δ :** 10.4 (2×CH_{3-CH2}), 23.0 (2xCH_{3 iPr}), 23.5 (CH_{iPr}), 26.7 (2×CH_{2-CH3}), 33.3 (CH₂), 41.6 (CH₂), 52.1 (CH_{iPent}), 72.1 (CH_{Ar}), 110.2 (C_{q}), 124.2 (2xCH_{Ar}), 139.4 (CH_{Ar}), 145.6 (C_{q}), 145.8 (C_{q}), 148.0 (C_{q}), 149.5 (CH_{Ar}), 156.7 (C_{q}). |
| | | | MS **(ESI+)** : m/z calcd for **C₂₀H₂₈N₆** : 367.26 [M+H]+, **found** : 367.43. |
| (10) | 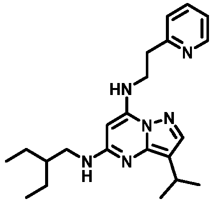 | > 99 % ^{a} | Cloudy oil. |
| | | | Rf (DCM/MeOH, 96/4) : 0.26. |
| | | | **¹H NMR (400 MHz, DMSO-*d₆*) δ :** 0.88 (t, *J =* 7.4 Hz, 6H, 2xCH_{3-CH2}), 1.24 (d, *J =* 6.9 Hz, 6H, 2×CH_{3 iPr}), 1_{.}28 - 1.36 (m, 4H, 2×CH_{2-CH3}), 1.48 (dq, *J* = 12.4, 6.3 Hz, 1H, HC-CH2-NH), 2.94 (hept, *J =* 6.9 Hz, 1H, H_{iPr}), 3.09 (t, *J =* 7.2 Hz, 2H, CH_{2-CH2-NH}), 3.24 (t, *J =* 5.9 Hz, 2H, _{CH-}CH_{2-NH}), 3.57 (q, *J =* 6.8 Hz, 2H, _{CH2-}CH_{2-NH}), 5.31 (s, 1H, H_{Ar}), 6.60 (t, *J =* 5.8 Hz, 1H, NH-_{CH2}), 7.09 (t, *J =* 6.0 Hz, 1H, NH_{-CH2}), 7.24 (ddd, *J =* 7.6, 4.8, 1.2 Hz, 1H, H_{Ar}), 7.33 (dt, *J =* 7.8, 1.1 Hz, 1H, H_{Ar}), 7.54 (s, 1H, H_{Ar}), 7.72 (td, *J =* 7.7, 1.9 Hz, 1H, H_{Ar}), 8.52 (ddd, *J* = 4.8, 1.9, 0.9 Hz, 1H, H_{Ar}). |
| | | | **¹³C NMR (101 MHz, DMSO-*d₆*) δ :** 11.0 (2xCH_{3-CH2}), 23.0 (2xCH_{3 iPr}), 23.4 (CH_{iPr}), 23.5 (2×CH_{2-CH3}), 36.2 (CH_{2-CH2-NH}), 40.6 (CH_{-CH2-NH}), 41.0 (_{CH2-}CH_{2-NH}), 42.5 (_{CH-}CH_{2-NH}), 71.9 (CH_{Ar}), 110.4 (C_{q}), 121.6 (CH_{Ar}), 123.3 (CH_{Ar}), 136.6 (CH_{Ar}), 139.4 (CH_{Ar}), 145.5 (C_{q}), 145.9 (C_{q}), 149.1 (CH_{Ar}), 156.8 (C_{q}), 158.8 (C_{q}). |
| | | | **MS (ESI+)** : **m/z calcd for C₂₂H₃₂N₆ :** 381.28 [M+H]+, **found** : 381.37. |
| (11) | 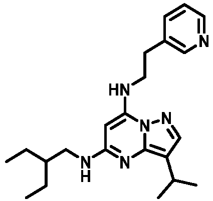 | > 98 *%* ^{a} | Brown solid. |
| | | | **¹H NMR (400 MHz, DMSO-*d₆*) δ :** 0.89 (t, *J* = 7.4 Hz, 6H, 2xCH_{3-CH2}), 1.24 (d, *J* = 6.9 Hz, 6H, 2xCH_{3 iPr}), 1.27 - 1.38 (m, 4H, 2xCH_{2-CH3}), 1.47 (p, *J* = 6.1 Hz, 1H, H_{C-CH2-NH}), 2.94 (h, *J* = 7.0 Hz, 3H, H_{iPr} & CH_{2-CH2}), 3.25 (t, *J* = 5.9 Hz, 2H, _{CH-}CH_{2-NH}), 3.41 - 3.50 (m, 2H, CH_{2-NH}), 5.32 (s, 1H, H_{Ar}), 6.56 (t, *J* = 5.8 Hz, 1H, NH_{-CH2}), 7.10 (t, *J* = 6.0 Hz, 1H, NH_{-CH2}), 7.33 (dd, *J* = 7.8, 4.8 Hz, 1H, H_{Ar}), 7.55 (s, 1H, H_{Ar}), 7.70 (dt, *J* = 7.8, 2.0 Hz, 1H, H_{Ar}), 8.43 (dd, *J* = 4.8, 1.6 Hz, 1H, H_{Ar}), 8.51 (d, *J* = 2.3 Hz, 1H, H_{Ar}). |
| | | | **¹³C NMR (101 MHz, DMSO-*d₆*) δ :** 11.0 (2xCH_{3-CH2}), 23.0 (2xCH_{3 iPr}), 23.4 (CH_{iPr}), 23.5 (2xCH_{2-CH3}), 31.2 (CH_{2-CH2}), 40.6 (CH_{-CH2-NH}), 42.4 (_{CH2-}CH_{2-NH}), 42.5 (_{CH-}CH_{2-NH}), 72.0 (CH_{Ar}), 110.4 (C_{q}), 123.4 (CH_{Ar}), 134.6 (C_{q}), 136.2 (CH_{Ar}), 139.4 (CH_{Ar}), 145.6 (C_{q}), 145.8 (C_{q}), 147.5 (CH_{Ar}), 149.9 (CH_{Ar}), 156.8 (C_{q}). |
| | | | **MS (ESI+) : m/z calcd for C₂₂H₃₂N₆** : 381.28 [M+H]+, **found :** 381.37. |
| (12) | 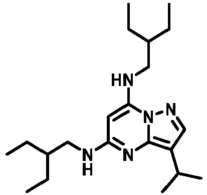 | > 97 *%* ^{a} | Light brown oil. |
| | | | **Rf** (DCM/MeOH, 96/4) : 0.77. |
| | | | **¹H NMR (400 MHz, DMSO-*d₆*) δ :** 0.88 (td, *J* = 7.4, 2.3 Hz, 12H, 4xCH_{3-CH2}), 1.24 (d, *J* = 6.9 Hz, 6H, 2xCH_{3 iPr}), 1.33 (tdd, *J* = 11.9, 9.0, 6.4 Hz, 8H, 4xCH_{2-CH3}), 1.47 (p, *J* = 6.2 Hz, 1H, H_{C-CH2-NH}), 1.64 (p, *J* = 6.4 Hz, 1H, H_{C-CH2-NH}), 2.95 (p, *J* = 6.9 Hz, 1H, H_{iPr}), 3.09 (t, *J* = 6.5 Hz, 2H, CH_{2-NH}), 3.23 (t, *J* = 6.0 Hz, 2H, CH_{2-NH}), 5.22 (s, 1H, H_{Ar}), 6.58 (t, *J* = 5.7 Hz, 1H, NH), 6.92 (t, *J* = 6.1 Hz, 1H, NH), 7.55 (s, 1H, H_{Ar}). |
| | | | **¹³C NMR (101 MHz, DMSO-*d₆*) δ :** 10.6 (2xCH_{3-CH2}), 11.0 (2xCH_{3-CH2}), 23.0 (2xCH_{3 iPr}), 23.1 (2xCH_{2-CH3}), 23.4 (CH_{iPr}), 23.5 (2xCH_{2-CH3}), 38.9 (CH_{-CH2-NH}), 40.6 (CH_{-CH2-NH}), 42.6 (_{CH-}CH_{2-NH}), 44.5 (_{CH}-CH_{2-NH}), 71.7 (CH_{Ar}), 110.4 (C_{q}), 139.4 (CH_{Ar}), 145.5 (C_{q}), 146.1 (C_{q}), 156.8 (C_{q}). |
| | | | **MS (ESI+) : m/z calcd for C₂₁H₃₇N₅** : 360.31 [M+H]+, **found :** 360.41. |
| (13) | 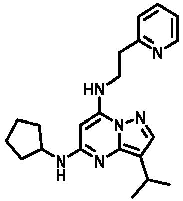 | > 98 *%* ^{a} | Light brown solid. |
| | | | **Rf** (DCM/MeOH, 96/4) : 0.26. |
| | | | **¹H NMR (400 MHz, DMSO-*d₆*) δ :** 1.24 (d, *J* = 6.9 Hz, 6H, 2xCH_{3 iPr}), 1.44 (dq, *J* = 13.7, 7.5, 6.9 Hz, 2H, 2xH_{(CH2) cPent}), 1.50 - 1.61 (m, 2H, 2xH_{(CH2) cPent}), 1.61 - 1.73 (m, 2H, 2xH_{(CH2) cPent}), 1.94 (dq, *J* = 12.2, 6.4 Hz, 2H, 2xH_{(CH2) cPent}), 2.95 (hept, *J* = 6.9 Hz, 1H, H_{iPr}), 3.09 (t, *J* = 7.2 Hz, 2H, CH_{2-CH2-NH}), 3.57 (q, *J* = 7.0 Hz, 2H, CH_{2-NH}), 4.16 (h, *J* = 6.4 Hz, 1H, H_{cPent}), 5.29 (s, 1H, H_{Ar}), 6.61 (d, *J* = 6.7 Hz, 1H, NH_{cPent}), 7.10 (t, *J* = 5.9 Hz, 1H, NH_{-CH2}), 7.24 (ddd, *J* = 7.5, 4.9, 1.2 Hz, 1H, H_{Ar}), 7.33 (dt, *J* = 7.8, 1.1 Hz, 1H, H_{Ar}), 7.55 (s, 1H, H_{Ar}), 7.72 (td, *J* = 7.6, 1.8 Hz, 1H, H_{Ar}), 8.47 - 8.61 (m, 1H, H_{Ar}). |
| | | | **¹³C NMR (101 MHz, DMSO-de) δ :** 23.1 (2xCH_{3 iPr}), 23.4 (CH_{iPr}), 23.5 (2xCH_{2 cPent}), 32.6 (2xCH₂ cPent), 36.2 (CH_{2-CH2-NH}), 41.0 (CH_{2-NH}), 51.9 (CH_{cPent}), 72.0 (CH_{Ar}), 110.5 (C_{q}), 121.6 (CH_{Ar}), 123.3 (CH_{Ar}), 136.6 (CH_{Ar}), 139.4 (CH_{Ar}), 145.6 (C_{q}), 145.9 (C_{q}), 149.1 (CH_{Ar}), 156.3 (C_{q}), 158.8 (C_{q}). |
| | | | **MS (ESI+) : m/z calcd for C₂₁H₂₈N₆ :** 365.24 [M+H]+, **found :** 365.35. |
| (14) | 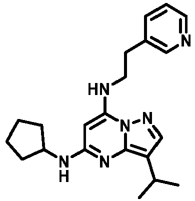 | > 98 *%* ^{a} | Brown solid. |
| | | | **Rf** (DCM/MeOH, 96/4) : 0.28. |
| | | | **¹H NMR (400 MHz, DMSO-*d₆*) δ :** 1.25 (d, *J* = 6.9 Hz, 6H, 2xCH_{3 iPr}), 1.44 (tdd, *J* = 13.6, 7.0, 3.6 Hz, 2H, 2xH_{(CH2) cPent}), 1.50 - 1.62 (m, 2H, 2xH_{(CH2) cPent}), 1.66 (td, *J* = 10.7, 9.8, 6.4 Hz, 2H, 2xH_{(CH2) cPent}), 1.90 - 2.02 (m, 2H, 2xH_{(CH2) cPent}), 2.95 (td, *J* = 7.0, 4.2 Hz, 3H, H_{iPr} & CH_{2-CH2-NH}), 3.37 - 3.50 (m, 2H, CH_{2-NH}), 4.17 (h, *J* = 6.6 Hz, 1H, H_{cPent}), 5.29 (s, 1H, H_{Ar}), 6.57 (d, *J* = 6.7 Hz, 1H, NH_{-CH}), 7.12 (t, *J* = 6.0 Hz, 1H, NH_{-CH2}), 7.33 (ddd, *J* = 7.8, 4.8, 0.9 Hz, 1H, H_{Ar}), 7.55 (s, 1H, H_{Ar}), 7.70 (dt, *J* = 7.8, 2.0 Hz, 1H, H_{Ar}), 8.43 (dd, *J* = 4.8, 1.7 Hz, 1H, H_{Ar}), 8.51 (d, *J* = 2.2 Hz, 1H, H_{Ar}). |
| | | | **¹³C NMR (101 MHz, DMSO-*d₆*) δ :** 23.1 (2xCH_{3 iPr}), 23.4 (CH_{iPr}), 23.5 (2xCH_{2 cPent}), 31.2 (CH_{2-CH2-NH}), 32.6 (2xCH_{2 cPent}), 42.4 (CH_{2-NH}), 51.9 (CH_{-NH}), 72.0 (CH_{Ar}), 110.5 (C_{q}), 123.5 (CH_{Ar}), 134.6 (C_{q}), 136.2 (CH_{Ar}), 139.5 (CH_{Ar}), 145.6 (C_{q}), 145.8 (C_{q}), 147.5 (CH_{Ar}), 149.9 (CH_{Ar}), 156.3 (C_{q}). |
| | | | **MS (ESI+) : m/z calcd for C₂₁H₂₈N₆** : 365.24 [M+H]+, **found :** 365.35. |
| (15) | 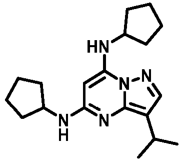 | > 99 *%* ^{a} | Light brown oil. |
| | | | **Rf** (DCM/MeOH, 96/4) : 0.67. |
| | | | **¹H NMR (400 MHz, DMSO-*d₆*) δ :** 1.24 (d, *J* = 6.9 Hz, 6H, 2xCH_{3 iPr}), 1.39 - 1.49 (m, 2H, CH_{2 cPent}), 1.55 (td, *J* = 7.5, 4.1 Hz, 4H, 2xCH_{2 cPent}), 1.68 (dp, *J* = 18.2, 5.9, 5.2 Hz, 6H, 3xCH_{2 cPent}), 1.95 (tt, *J* = 13.1, 6.8 Hz, 4H, 2xCH_{2 cPent}), 2.95 (hept, *J* = 7.0 Hz, 1H, H_{iPr}), 3.79 (h, *J* = 6.6 Hz, 1H, H_{cPent}), 4.16 (h, *J* = 6.5 Hz, 1H, H_{cPent}), 5.25 (s, 1H, H_{Ar}), 6.56 (d, *J* = 6.9 Hz, 1H, NH), 6.59 (d, *J* = 6.6 Hz, 1H; NH), 7.54 (s, 1H, H_{Ar}). |
| | | | **¹³C NMR (101 MHz, DMSO-*d₆*) δ :** 23.1 (2xCH_{3 iPr}), 23.4 (CH_{iPr}), 23.5 (2xCH_{2 cPent}), 23.6 (2xCH_{2 cPent}), 32.0 (2xCH_{2 cPent}), 32.6 (2xCH_{2 cPent}), 51.9 (CH_{cPent}), 52.9 (2xCH_{cPent}), 72.9 (CH_{Ar}), 110.5 (C_{q}), 139.3 (CH_{Ar}), 145.6 (C_{q}), 156.2 (C_{q}). |
| | | | **MS (ESI+) : m/z calcd for C₁₉H₂₉N₅ :** 328.25 [M+H]+, **found :** 328.33. |
| (16) | 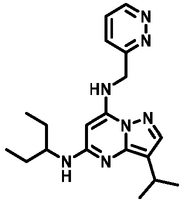 | > 98*%* ^{b} | White solid. |
| | | | **Rf** (DCM/MeOH, 94/6) : 0.21. |
| | | | **¹H NMR (400 MHz, DMSO-*d₆*) δ :** 0.80 (t, *J* = 7.4 Hz, 6H, 2xCH_{3-CH2}), 1.24 (d, *J* = 7.0 Hz, 6H, 2xCH_{3 iPr}), 1.31 - 1.59 (m, 4H, 2xCH_{2-CH3}), 2.93 (hept, *J* = 6.9 Hz, 1H, H_{iPr}), 3.75 (s, 1H, H_{iPent}), 4.73 (d, *J =* 6.4 Hz, 2H, NH_{Bn}), 5.16 (s, 1H, H_{Ar}), 6.42 (d, *J =* 8.1 Hz, 1H, NH_{iPent}), 7.60 (s, 1H, H_{Ar}), 7.64 (dd, *J* = 8.5, 1.9 Hz, 1H, H_{Ar}), 7.68 (dd, *J* = 8.5, 4.6 Hz, 1H, H_{Ar}), 7.87 (t, *J* = 6.4 Hz, 1H, NH_{Bn}), 9.16 (dd, *J* = 4.6, 1.9 Hz, 1H, H_{Ar}). |
| | | | **¹³C NMR (101 MHz, DMSO-*d₆*) δ :** 10.4 (2xCH_{3-CH2}), 23.0 (2xCH_{3 iPr}), 23.5 (CH_{iPr}), 26.5 (2xCH_{2-CH3}), 45.1 (CH_{2 Bn}), 52.2 (CH_{iPent}), 72.8 (CH_{Ar}), 110.3 (C_{q}), 125.1 (CH_{Ar}), 127.5 (CH_{Ar}), 139.7 (CH_{Ar}), 145.5 (C_{q}), 145.8 (C_{q}), 150.9 (CH_{Ar}), 156.5 (C_{q}), 160.2 (C_{q}). |
| | | | **MS (ESI+) : m/z calcd for C₁₉H₂₇N₇** : 354.24 [M+H]+, **found :** 354.34. |
| (17) | 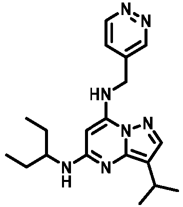 | > 95*%* ^{b} | Brown solid. |
| | | | **Rf** (DCM/MeOH, 94/6) : 0.17. |
| | | | **¹H NMR (400 MHz, DMSO-*d₆*) δ :** 0.80 (t, *J* = 7.3 Hz, 6H, 2xCH_{3-CH2}), 1.25 (d, *J* = 6.9 Hz, 6H, 2xCH_{3 iPr}), 1.33 - 1.55 (m, 4H, 2xCH_{2-CH3}), 2.94 (hept, *J* = 6.8 Hz, 1H, H_{iPr}), 3.76 (s, 1H, H_{iPent}), 4.53 (d, *J* = 6.4 Hz, 2H, CH_{2 Bn}), 5.06 (s, 1H, H_{Ar}), 6.32 (d, *J* = 8.2 Hz, 1H, NH_{iPent}), 7.57 (dd, *J* = 5.3, 2.4 Hz, 1H, H_{Ar}), 7.61 (s, 1H, H_{Ar}), 7.86 (t, *J* = 6.5 Hz, 1H, NH_{Bn}), 9.16 (dd, *J* = 5.3, 1.3 Hz, 1H, H_{Ar}), 9.24 (dd, *J* = 2.4, 1.3 Hz, 1H, H_{Ar}). |
| | | | **¹³C NMR (101 MHz, DMSO-*d₆*) δ :** 10.4 (2xCH_{3-CH2}), 23.0 (2xCH_{3 iPr}), 23.5 (CH_{iPr}), 26.5 (2xCH_{2-CH3}), 41.8 (CH_{2 Bn}), 52.2 (CH_{iPent}), 72.9 (CH_{Ar}), 110.4 (C_{q}), 124.5 (CH_{Ar}), 138.5 (C_{q}), 139.8 (CH_{Ar}), 145.6 (C_{q}), 145.7 (C_{q}), 150.9 (CH_{Ar}), 151.4 (CH_{Ar}), 156.5 (C_{q}). |
| | | | **MS (ESI+) : m/z calcd for C₁₉H₂₇N₇ :** 354.24 [M+H]+, **found :** 354.33. |
| (18) | 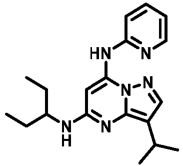 | > 98*%* ^{b} | White solid. |
| | | | **Rf (DCM,** 100%) : 0.11. |
| | | | **¹H NMR (400 MHz, DMSO-*d*₆) δ :** 0.88 (t, *J* = 7.4 Hz, 6H, 2xCH_{3-CH2}), 1.28 (d, *J* = 6.8 Hz, 6H, 2xCH_{3 iPr}), 1.41 - 1.64 (m, 4H, 2xCH_{2-CH3}), 2.99 (hept, *J* = 7.0 Hz, 1H, H_{iPr}), 3.90 (s, 1H, H_{iPent}), 6.84 (d, *J* = 8.1 Hz, 1H, NH_{iPent}), 7.02 (ddd, *J* = 7.2, 5.0, 0.9 Hz, 1H, H_{Ar}), 7.28 (s, 1H, H_{Ar}), 7.52 (d, *J* = 8.3 Hz, 1H, H_{Ar}), 7.66 (s, 1H, H_{Ar}), 7.74 (ddd, *J* = 9.0, 7.2, 2.0 Hz, 1H, H_{Ar}), 8.34 (dd, *J* = 5.2, 1.9 Hz, 1H, H_{Ar}), 9.58 (s, 1H, NH). |
| | | | **¹³C NMR (101 MHz, DMSO-*d₆*) δ :** 10.5 (2xCH_{3-CH2}), 23.0 (2xCH_{3 iPr}), 23.5 (CH_{iPr}), 26.6 (2xCH_{2-CH3}), 52.3 (CH_{iPent}), 81.1 (CH_{Ar}), 110.9 (C_{q}), 113.9 (CH_{Ar}), 117.4 (CH_{Ar}), 137.9 (CH_{Ar}), 139.2 (CH_{Ar}), 140.3 (C_{q}), 145.4 (C_{q}), 147.0 (CH_{Ar}), 153.5 (C_{q}), 156.5 (C_{q}). |
| | | | **MS (ESI+) : m/z calcd for C₁₉H₂₆N₆** : 339.23 [M+H]+, **found :** 339.41. |
| (19) | 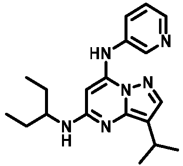 | > 98*%* ^{b} | White solid. |
| | | | **Rf** (DCM/MeOH, 98/2) : 0.22. |
| | | | **¹H NMR (400 MHz, DMSO-*d*₆) δ :** 0.85 (t, *J* = 7.4 Hz, 6H, 2xCH_{3-CH2}), 1.28 (d, *J* = 6.9 Hz, 6H, 2xCH_{3 iPr}), 1.36 - 1.62 (m, 4H, 2xCH_{2-CH3}), 2.98 (hept, *J* = 7.0 Hz, 1H, H_{iPr}), 3.85 (s, 1H, H_{iPent}), 5.72 (s, 1H, H_{Ar}), 6.55 (d, *J* = 8.1 Hz, 1H, NH_{iPent}), 7.45 (dd, *J* = 8.2, 4.7 Hz, 1H, H_{Ar}), 7.67 (s, 1H, H_{Ar}), 7.82 (dt, *J* = 8.4, 1.9 Hz, 1H, H_{Ar}), 8.38 (dd, *J* = 4.7, 1.5 Hz, 1H, H_{Ar}), 8.66 (d, *J* = 2.6 Hz, 1H, H_{Ar}), 9.26 (s, 1H, NH). |
| | | | **¹³C NMR (101 MHz, DMSO-*d₆*) δ :** 10.3 (2xCH_{3-CH2}), 23.0 (2xCH_{3 iPr}), 23.6 (CH_{iPr}), 26.5 (2XCH_{2-CH3}), 52.1 (CH_{iPent}), 75.0 (CH_{Ar}), 110.8 (C_{q}), 123.9 (CH_{Ar}), 130.4 (CH_{Ar}), 135.4 (C_{q}), 139.8 (CH_{Ar}), 143.7 (C_{q}), 144.9 (CH_{Ar}), 145.1 (CH_{Ar}), 145.9 (C_{q}), 156.4 (C_{q}). |
| | | | **MS (ESI+) : m/z calcd for C₁₉H₂₆N₆** : 339.23 [M+H]+, **found :** 339.41. |
| (20) | 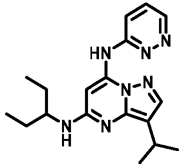 | > 98*%* ^{b} | White solid. |
| | | | **¹H NMR (400 MHz, DMSO-*d₆*) δ :** 0.88 (t, *J* = 7.4 Hz, 6H, 2xCH_{3-CH2}), 1.29 (d, *J* = 6.9 Hz, 6H, 2xCH_{3 iPr}), 1.41 - 1.65 (m, 4H, 2xCH_{2-CH3}), 3.00 (dp, *J* = 13.7, 6.9 Hz, 1H, H_{iPr}), 3.91 (s, 1H, H_{iPent}), 7.07 (d, *J* = 8.0 Hz, 1H, NH_{iPent}), 7.45 (s, 1H, H_{Ar}), 7.63 (dd, *J* = 9.0, 4.6 Hz, 1H, H_{Ar}), 7.69 (s, 1H, H_{Ar}), 7.87 (dd, *J* = 9.0, 1.4 Hz, 1H, H_{Ar}), 8.91 (dd, *J* = 4.5, 1.4 Hz, 1H, H_{Ar}), 9.92 (s, 1H, NH). |
| | | | **¹³C NMR (101 MHz, DMSO-*d₆*) δ :** 10.5 (2xCH_{3-CH2}), 22.9 (2xCH_{3 iPr}), 23.5 (CH_{iPr}), 26.5 (2xCH_{2-CH3}), 52.4 (CH_{iPent}), 82.8 (CH_{Ar}), 111.1 (C_{q}), 118.9 (C_{q}), 128.2 (CH_{Ar}), 139.4 (CH_{Ar}), 139.6 (C_{q}), 145.5 (C_{q}), 147.1 (CH_{Ar}), 156.4 (C_{q}), 156.5 (C_{q}). |
| | | | **MS (ESI+) : m/z calcd for C₁₈H₂₅N₇ :** 340.22 [M+H]+, **found :** 340.37. |
| (21) | 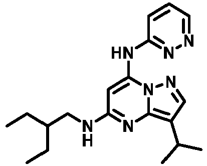 | > 99*%* ^{b} | White solid. |
| | | | **Rf** (EtOAc, Cyclohexane, 50/50) : 0.26. |
| | | | **¹H NMR (400 MHz, DMSO-*d₆*) δ :** 0.90 (t, *J* = 7.4 Hz, 6H, 2 X CH_{3 iPent}), 1.29 (d, *J* = 6.9 Hz, 6H, 2 X CH_{3 iPr}), 1.35 (dq, *J* = 13.9, 6.8 Hz, 4H, 2 X CH_{2 iPent}), 1.52 (h, *J* = 6.3 Hz, 1H, CH_{iPent}), 3.02 (hept, *J* = 6.9 Hz, 1H, CH_{iPr}), 3.29 (d, *J* = 6.0 Hz, 2H, CH₂-N), 7.28 (t, *J* = 5.7 Hz, 1H,; NH), 7.45 (s, 1H, CH_{Ar}), 7.63 (dd, *J* = 9.0, 4.6 Hz, 1H, CH_{Ar}), 7.70 (s, 1H, CH_{Ar}), 7.87 (dd, *J* = 9.0, 1.4 Hz, 1H, CH_{Ar}), 8.91 (dd, *J* = 4.6, 1.4 Hz, 1H, CH_{Ar}), 9.93 (s, 1H, NH). |
| | | | **¹³C NMR (101 MHz, DMSO-*d₆*) δ :** 11.0 (CH_{3 iPent}), 23.0 (CH_{3 iPr}), 23.5 (CH_{iPr}), 23.5 (CH_{2 iPent}), 40.4 (CH_{iPent}), 42.7 (CH_{2 iPr}), 82.7 (CH_{Ar}), 111.3 (C_{q}), 118.9 (CH_{Ar}), 128.2 (CH_{Ar}), 139.4 (CH_{Ar}), 139.6 (C_{q}), 145.5 (C_{q}), 147.1 (CH_{Ar}), 156.4 (C_{q}), 156.6 (C_{q}). |
| | | | **MS (ESI+) : m/z calcd for C₁₉H₂₆N₇O :** 354.24 [M+H]+, **found :** 354.43. |
| (22) | 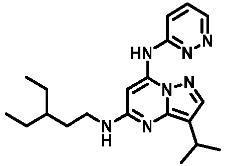 | > 98*%* ^{b} | White solid. |
| | | | **Rf** (DCM/MeOH, 98/2) : 0.11 |
| | | | **¹H NMR (400 MHz, DMSO-*d₆*) δ :** 0.85 (t, *J =* 7.0 Hz, 6H, 2 X CH_{3 iPent}), 1.29 (d, *J* = 6.9 Hz, 6H, 2 X CH_{3 iPr}), 1.30 - 1.35 (m, 5H, CH _{iPent} & 2 X CH_{2 iPent}), 1.53 (q, *J* = 6.7 Hz, 2H, CH₂-CH₂-NH), 3.02 (h, *J =* 6.8 Hz, 1H, CH _{iPr}), 3.33 - 3.37 (m, 2H, CH₂-NH), 7.27 (t, *J=* 5.5 Hz, 1H, NH), 7.41 (s, 1H, CH_{Ar}), 7.63 (dd, *J* = 8.9, 4.5 Hz, 1H, CH_{Ar}), 7.71 (s, 1H, CH_{Ar}), 7.87 (dd, *J* = 9.0, 1.4 Hz, 1H, CH_{Ar}), 8.91 (dd, *J* = 4.6, 1.4 Hz, 1H, CH_{Ar}), 9.94 (s, 1H, NH). |
| | | | **¹³C NMR (101 MHz, DMSO-*d₆*) δ :** 10.7 (2 X CH_{3 iPent}), 23.0 (2 X CH_{3 iPr}), 23.4 (CH_{iPr}), 25.0 (2 X CH_{2 iPent}), 31.9 (CH₂-CH_{2-NH}), 37.5 (CH _{iPent}), 38.2 (CH₂-NH), 82.7 (CH_{Ar}), 111.4 (C_{q}), 118.9 (CH_{Ar}), 128.2 (CH_{Ar}), 139.4 (C_{q}), 139.7 (CH_{Ar}), 145.5 (C_{q}), 147.1 (CH_{Ar}), 156.4 (C_{q}), 156.4 (C_{q}). |
| | | | **MS (ESI+) : m/z calcd for C₂₀H₂₉N₇** : 368.26 [M+H]+, **found :** 368.51. |
| (23) | 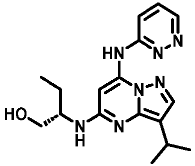 | > 98*%* ^{b} | Beige solid. |
| | | | **¹H NMR (400 MHz, DMSO-*d₆*) δ :** 0.91 (t, *J* = 7.4 Hz, 3H, CH_{3-CH2}), 1.29 (d, *J* = 6.9 Hz, 6H, 2xCH_{3 iPr}), 1.47 (dt, *J* = 13.4, 7.5 Hz, 1H, H_{(CH2)-CH3}), 1.69 (ddd, *J* = 13.1, 7.5, 5.3 Hz, 1H, H_{(CH2)-CH3}), 3.01 (hept, *J* = 7.0 Hz, 1H, H_{iPr}), 3.47 (ddt, *J* = 30.1, 10.9, 5.5 Hz, 2H, CH_{2-OH}), 3.89 - 4.03 (m, 1H, H_{C-NH}), 4.67 (t, *J* = 5.5 Hz, 1H, OH), 7.07 (d, *J* = 7.8 Hz, 1H, NH_{-CH}), 7.46 (s, 1H, H_{Ar}), 7.64 (dd, *J* = 9.0, 4.6 Hz, 1H, H_{Ar}), 7.71 (s, 1H, H_{Ar}), 7.87 (dd, *J* = 9.0, 1.4 Hz, 1H, H_{Ar}), 8.91 (dd, *J* = 4.5, 1.4 Hz, 1H, H_{Ar}), 9.94 (s, 1H, NH). |
| | | | **¹³C NMR (101 MHz, DMSO-*d₆*) δ :** 10.5 (CH_{3-CH2}), 23.0 (CH_{3 iPr}), 23.1 (CH_{3 iPr}), 23.5 (CH_{iPr}), 23.7 (CH_{2-CH3}), 53.5 (CH_{-NH}), 62.9 (CH_{2-OH}), 83.0 (CH_{Ar}), 111.2 (C_{q}), 118.9 (CH_{Ar}), 128.2 (CH_{Ar}), 139.4 (CH_{Ar}), 139.7 (C_{q}), 145.3 (C_{q}), 147.1 (CH_{Ar}), 156.4 (C_{q}), 156.5 (C_{q}). |
| | | | **MS (ESI+) : m/z calcd for C₁₇H₂₃N₇O :** 342.20 [M+H]+, **found :** 342.37. |
| (24) | 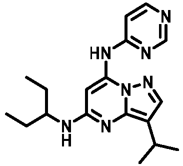 | > 98*%* ^{b} | White solid. |
| | | | **¹H NMR (400 MHz, DMSO-*d₆*) δ :** 0.88 (t, *J* = 7.4 Hz, 6H, 2xCH_{3-CH2}), 1.28 (d, *J* = 6.9 Hz, 6H, CH_{3 iPr}), 1.41 - 1.67 (m, 4H, 2xCH_{2-CH3}), 2.99 (hept, *J* = 7.1 Hz, 1H, H_{iPr}), 3.90 (s, 1H, H_{iPent}), 7.03 (d, *J =* 8.1 Hz, 1H, NH_{iPent}), 7.36 (s, 1H, H_{Ar}), 7.58 (dd, *J* = 5.9, 1.3 Hz, 1H, H_{Ar}), 7.69 (s, 1H, H_{Ar}), 8.52 (d, *J =* 5.9 Hz, 1H, H_{Ar}), 8.87 (d, *J* = 1.2 Hz, 1H, H_{Ar}), 10.18 (s, 1H, NH_{Pyrim}). |
| | | | **¹³C NMR (101 MHz, DMSO-*d₆*) δ :** 10.5 (2xCH_{3-CH2}), 22.9 (2xCH_{3 iPr}), 23.5 (CH_{iPr}), 26.5 (2xCH_{2-CH3}), 52.5 (CH_{iPent}), 84.3 (CH_{Ar}), 110.3 (CH_{Ar}), 111.2 (C_{q}), 139.2 (C_{q}), 139.5 (CH_{Ar}), 145.5 (C_{q}), 156.1 (C_{q}), 156.4 (CH_{Ar}), 157.6 (CH_{Ar}), 159.1 (C_{q}). |
| | | | **MS (ESI+) : m/z calcd for C₁₈H₂₅N₇ :** 340.22 [M+H]+, **found :** 340.36. |
| (25) | 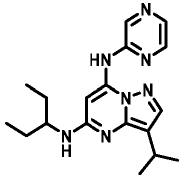 | > 99*%* ^{b} | White solid. |
| | | | **Rf** (DCM/EtOAc, 85/15) : 0,25 |
| | | | **¹H NMR (400 MHz, DMSO-*d₆*) δ :** 0.88 (t, *J* = 7.3 Hz, 6H, 2xCH_{3-CH2}), 1.29 (d, *J* = 6.8 Hz, 6H, 2xCH_{3 iPr}), 1.52 (ddp, *J =* 35.8, 14.5, 7.3, 6.8 Hz, 4H, 2xCH_{2-CH3}), 2.99 (hept, *J* = 6.8 Hz, 1H, H_{iPr}), 3.90 (s, 1H, H_{iPent}), 6.94 (d, *J =* 8.1 Hz, 1H, NH_{iPent}), 7.23 (s, 1H, H_{Ar}), 7.69 (s, 1H, H_{Ar}), 8.22 (d, *J =* 2.8 Hz, 1H, H_{Ar}), 8.30 - 8.43 (m, 1H, H_{Ar}), 8.89 (s, 1H, H_{Ar}), 10.12 (s, 1H, NH). |
| | | | **¹³C NMR (101 MHz, DMSO-*d₆*) δ :** 10.5 (2xCH_{3-CH2}), 22.9 (2xCH_{3 iPr}), 23.5 (CH_{iPr}), 26.5 (2xCH_{2-CH3}), 52.4 (CH_{iPent}), 81.9 (CH_{Ar}), 111.1 (C_{q}), 136.7 (CH_{Ar}), 137.4 (CH_{Ar}), 139.4 (CH_{Ar}), 139.8 (C_{q}), 140.8 (CH_{Ar}), 145.5 (C_{q}), 150.3 (C_{q}), 156.3 (C_{q}). |
| | | | **MS (ESI+) : m/z calcd for C₁₈H₂₅N₇ :** 340.22 [M+H]+, **found :** 340.38. |
| (26) | 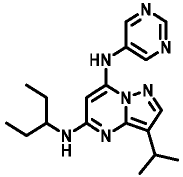 | > 98*%* ^{b} | Beige solid. |
| | | | Rf (DCM/EtOAc, 85/15) : 0,13 |
| | | | **¹H NMR (400 MHz, DMSO-*d₆*) δ :** 0.86 (t, *J* = 7.4 Hz, 6H, 2xCH_{3-CH2}), 1.28 (d, *J* = 6.9 Hz, 6H, 2xCH_{3 iPr}), 1.49 (ddq, *J =* 43.2, 14.0, 6.8 Hz, 4H, 2xCH_{2-CH3}), 2.99 (hept, *J* = 6.8 Hz, 1H, H_{iPr}), 3.86 (s, 1H, H_{iPent}), 5.83 (s, 1H, H_{Ar}), 6.58 (d, *J =* 8.0 Hz, 1H, NH_{iPent}), 7.69 (s, 1H, H_{Ar}), 8.90 (s, 2H, 2xH_{Ar}), 8.98 (s, 1H, H_{Ar}), 9.47 (s, 1H, NH). |
| | | | **¹³C NMR (101 MHz, DMSO-*d₆*) δ :** 10.3 (2xCH_{3-CH2}), 23.0 (2xCH_{3 iPr}), 23.5 (CH_{iPr}), 26.5 (2xCH_{2-CH3}), 52.1 (CH_{iPent}), 75.7 (CH_{Ar}), 111.0 (C_{q}), 134.5 (C_{q}), 140.0 (CH_{Ar}), 142.9 (C_{q}), 145.9 (C_{q}), 150.9 (2xCH_{Ar}), 153.5 (CH_{Ar}), 156.3 (C_{q}). |
| | | | **MS (ESI+) : m/z calcd for C₁₈H₂₅N₇** : 340.22 [M+H]+, **found :** 340.38. |
| (27) | 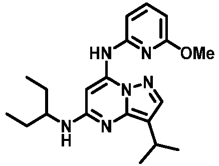 | > 98*%* ^{b} | White solid. |
| | | | **Rf** (DCM/MeOH, 95/5) : 0.53. |
| | | | **¹H NMR (400 MHz, DMSO-*d*₆) δ :** 0.88 (t, *J* = 7.3 Hz, 6H, 2xCH_{3-CH2}), 1.28 (d, *J* = 6.9 Hz, 6H, 2xCH_{3 iPt}), 1.53 (ddt, *J* = 41.0, 13.9, 7.0 Hz, 4H, 2xCH_{2-CH3}), 2.99 (p, *J* = 6.9 Hz, 1H, H_{iPr}), 3.88 (s, 1H, H_{iPent}), 3.95 (s, 3H, CH_{3-O}), 6.43 (d, *J* = 7.9 Hz, 1H, H_{Ar}), 6.72 (d, *J =* 8.2 Hz, 1H, NH_{iPent}), 6.98 (s, 1H, H_{Ar}), 7.04 (d, *J* = 7.8 Hz, 1H, H_{Ar}), 7.64 (d, *J* = 10.0 Hz, 2H, 2xH_{Ar}), 9.50 (s, 1H, NH). |
| | | | **¹³C NMR (101 MHz, DMSO-*d₆*) δ :** 10.6 (2xCH_{3-CH2}), 23.0 (2xCH_{3 iPr}), 23.5 (CH_{iPr}), 26.7 (2xCH_{2-CH3}), 52.6 (CH_{iPent}), 53.9 (CH_{3-O}), 80.7 (CH_{Ar}), 102.3 (CH_{Ar}), 105.3 (CH_{Ar}), 111.0 (C_{q}), 139.4 (CH_{Ar}), 140.4 (C_{q}), 140.7 (CH_{Ar}), 145.4 (C_{q}), 151.5 (C_{q}), 156.5 (C_{q}), 162.6 (C_{q}). |
| | | | **MS (ESI+) : m/z calcd for C₂₀H₂₈N₆O :** 369.49 [M+H]+, **found :** 369.44. |
| (28) | 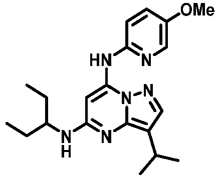 | > 96*%* ^{b} | White solid. |
| | | | **¹H NMR (400 MHz, DMSO-*d*₆) δ :** 0.87 (t, *J* = 7.4 Hz, 6H, 2xCH_{3-CH2}), 1.28 (d, *J* = 6.9 Hz, 6H, 2xCH_{3 iPr}), 1.40 - 1.65 (m, 4H, 2xCH_{2-CH3}), 2.98 (hept, *J* = 6.8 Hz, 1H, H_{iPr}), 3.82 (s, 3H, CH_{3-O}), 3.88 (s, 1H, H_{iPent}), 6.75 (d, *J =* 8.1 Hz, 1H, NH_{iPent}), 7.06 (s, 1H, H_{Ar}), 7.40 - 7.53 (m, 2H, 2xH_{Ar}), 7.64 (s, 1H, H_{Ar}), 8.04 (d, *J =* 2.5 Hz, 1H, H_{Ar}), 9.45 (s, 1H, NH). |
| | | | **¹³C NMR (101 MHz, DMSO-*d₆*) δ :** 10.5 (2xCH_{3-CH2}), 23.0 (2xCH_{3 iPr}), 23.6 (CH_{iPr}), 26.6 (2xCH_{2-CH3}), 52.3 (CH_{iPent}), 55.9 (CH_{3-O}), 79.4 (CH_{Ar}), 110.9 (C_{q}), 115.0 (CH_{Ar}), 124.7 (CH_{Ar}), 132.5 (CH_{Ar}), 139.2 (CH_{Ar}), 140.8 (C_{q}), 145.5 (C_{q}), 147.0 (C_{q}), 151.1 (C_{q}), 156.6 (C_{q}). |
| | | | **MS (ESI+) : m/z calcd for C₂₀H₂₈N₆O :** 369.49 [M+H]+, **found :** 369.45. |
| (29) | 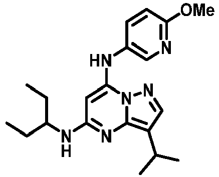 | > 98*%* ^{b} | White solid. |
| | | | **Rf** (DCM/MeOH, 100/0) : 0.03 |
| | | | **¹H NMR (400 MHz, DMSO-*d₆*) δ :** 0.84 (t, *J =* 7.4 Hz, 6H, 2 X CH_{3 iPent}), 1.28 (d, *J* = 6.9 Hz, 6H, 2 X CH_{3 iPr}), 1.41 (dq, *J =* 14.0, 7.3 Hz, 2H, CH_{2 iPent}), 1.53 (ddd, *J* = 13.1, 7.4, 5.4 Hz, 2H, CH_{2 iPent}), 2.98 (p, *J* = 6.9 Hz, 1H, CH _{iPr}), 3.84 (s, 1H, CH _{iPent}), 3.88 (s, 3H, O-CH₃), 5.39 (s, 1H, CH_{Ar}), 6.46 (d, *J=* 8.1 Hz, 1H, NH), 6.92 (d, *J* = 8.7 Hz, 1H, CH_{Ar}), 7.65 (s, 1H, CH_{Ar}), 7.74 (dd, *J* = 8.7, 2.7 Hz, 1H, CH_{Ar}), 8.20 (d, *J* = 2.7 Hz, 1H, CH_{Ar}), 8.96 (s, 1H, NH). |
| | | | **¹³C NMR (101 MHz, DMSO-*d₆*) δ :** 10.3 (2 X CH_{3 iPent}), 23.0 (2 X CH_{3 iPr}), 23.6 (CH _{iPr}), 26.5 (2 X CH_{2 iPent}), 52.0 (CH _{iPent}), 53.3 (O-CH₃), 74.1 (CH_{Ar}), 110.6 (C_{q}), 110.7 (CH_{Ar}), 129.0 (C_{q}), 136.8 (CH_{Ar}), 139.7 (CH_{Ar}), 143.3 (CH_{Ar}), 145.2 (C_{q}), 145.9 (C_{q}), 156.5 (C_{q}), 161.0 (C_{q}). |
| | | | **MS (ESI+) : m/z calcd for C₁₄H₁₅ClN₆O :** 369.24 [M+H]+, **found :** 369.50. |
| (30) | 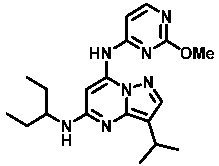 | > 98*%* ^{b} | White solid. |
| | | | **¹H NMR (400 MHz, DMSO-*d*₆) δ :** 0.88 (t, *J* = 7.4 Hz, 6H, 2xCH_{3-CH2}), 1.28 (d, *J* = 6.9 Hz, 6H, 2xCH_{3 iPr}), 1.43 - 1.66 (m, 4H, 2xCH_{2-CH3}), 2.99 (hept, *J* = 7.0 Hz, 1H, H_{iPr}), 3.91 (s, 1H, H_{iPent}), 3.95 (s, 3H, CH_{3-O}), 7.03 (d, *J* = 8.1 Hz, 1H, NH_{iPent}), 7.21 (d, *J* = 3.7 Hz, 1H, H_{Ar}), 7.21 (s, 1H, H_{Ar}), 7.68 (s, 1H, H_{Ar}), 8.31 (d, *J* = 5.7 Hz, 1H, H_{Ar}), 10.13 (s, 1H, NH). |
| | | | **¹³C NMR (101 MHz, DMSO-*d₆*) δ :** 10.5 (2xCH_{3-CH2}), 22.9 (2xCH_{3 iPr}), 23.5 (CH_{iPr}), 26.5 (2xCH_{2-CH3}), 52.6 (CH_{iPent}), 54.5 (CH_{3-O}), 84.5 (CH_{Ar}), 103.9 (CH_{Ar}), 111.1 (C_{q}), 139.1 (C_{q}), 139.6 (CH_{Ar}), 145.4 (C_{q}), 156.1 (C_{q}), 158.0 (CH_{Ar}), 161.1 (C_{q}), 164.5 (C_{q}). |
| | | | **MS (ESI+) : m/z calcd for C₁₉H₂₇N₇O :** 370.24 [M+H]+, **found :** 370.41. |
| (31) | 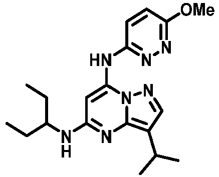 | > 98*%* ^{b} | White solid. |
| | | | **¹H NMR (400 MHz, DMSO-*d*₆) δ :** 0.88 (t, *J* = 7.4 Hz, 6H, 2xCH_{3-CH2}), 1.29 (d, *J* = 6.9 Hz, 6H, 2xCH_{3 iPr}), 1.41 - 1.64 (m, 4H, 2xCH_{2-CH3}), 2.99 (hept, *J* = 6.9 Hz, 1H, H_{iPr}), 3.92 (dd, *J* = 10.5, 6.5 Hz, 1H, H_{iPent}), 3.99 (s, 3H, CH_{3-O}), 7.02 (d, *J* = 8.1 Hz, 1H, NH_{iPent}), 7.25 (d, *J* = 9.5 Hz, 1H, H_{Ar}), 7.41 (s, 1H, H_{Ar}), 7.67 (s, 1H, H_{Ar}), 7.87 (d, *J* = 9.4 Hz, 1H, H_{Ar}), 9.82 (s, 1H, NH). |
| | | | **¹³C NMR (101 MHz, DMSO-*d₆*) δ :** 10.4 (2xCH_{3-CH2}), 23.0 (2xCH_{3 iPr}), 23.5 (CH_{iPr}), 26.5 (2xCH_{2-CH3}), 52.3 (CH_{iPent}), 54.1 (CH_{3-O}), 82.1 (CH_{Ar}), 111.0 (C_{q}), 119.6 (CH_{Ar}), 123.8 (CH_{Ar}), 139.2 (CH_{Ar}), 139.9 (C_{q}), 145.5 (C_{q}), 153.0 (C_{q}), 156.6 (C_{q}), 161.1 (C_{q}). |
| | | | **MS (ESI+) : m/z calcd for C₁₉H₂₇N₇O :** 370.24 [M+H]+, **found :** 370.46. |
| (32) | 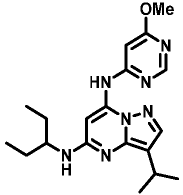 | > 97*%* ^{b} | White solid. |
| | | | **¹H NMR (400 MHz, DMSO-*d*₆) δ :** 0.88 (t, *J =* 7.4 Hz, 6H, 2xCH_{3-CH2}), 1.28 (d, *J* = 6.9 Hz, 6H, 2xCH_{3 iPr}), 1.52 (ddq, *J =* 35.4, 13.9, 7.2 Hz, 4H, 2xCH_{2-CH3}), 2.99 (dq, *J =* 13.6, 6.5 Hz, 1H, H_{iPr}), 3.90 (s, 4H, H_{iPent} & CH_{3-O}), 6.97 (d, *J* = 7.2 Hz, 2H, NH_{iPent} & H_{Ar}), 7.27 (s, 1H, H_{Ar}), 7.67 (s, 1H, H_{Ar}), 8.59 (s, 1H, H_{Ar}), 9.96 (s, 1H, NH). |
| | | | **¹³C NMR (101 MHz, DMSO-*d₆*) δ :** 10.5 (2xCH_{3-CH2}), 22.9 (2xCH_{3 iPr}), 23.5 (CH_{iPr}), 26.5 (2xCH_{2-CH3}), 52.4 (CH_{iPent}), 53.7 (CH_{3-O}), 83.5 (CH_{Ar}), 92.7 (CH_{Ar}), 111.1 (C_{q}), 139.5 (CH_{Ar}), 139.5 (C_{q}), 145.5 (C_{q}), 156.2 (C_{q}), 157.4 (CH_{Ar}), 160.6 (C_{q}), 169.7 (C_{q}). |
| | | | **MS (ESI+) : m/z calcd for C₁₉H₂₇N₇O :** 370.24 [M+H]+, **found :** 370.42. |
| (33) | 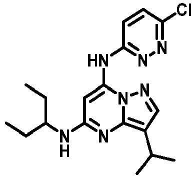 | > 99*%* ^{b} | White solid. |
| | | | **¹H NMR (400 MHz, DMSO-*d₆*) δ :** 0.88 (t, *J =* 7.4 Hz, 6H, 2xCH_{3-CH2}), 1.29 (d, *J* = 6.9 Hz, 6H, 2xCH_{3 iPr}), 1.40 - 1.67 (m, 4H, 2xCH_{2-CH3}), 3.00 (hept, *J =* 7.0 Hz, 1H, H_{iPr}), 3.90 (s, 1H, H_{iPent}), 7.08 (d, *J* = 8.1 Hz, 1H, NH_{iPent}), 7.37 (s, 1H, H_{Ar}), 7.70 (s, 1H, H_{Ar}), 7.82 (d, *J =* 9.3 Hz, 1H, H_{Ar}), 7.96 (d, *J =* 9.3 Hz, 1H, H_{Ar}), 10.17 (s, 1H, NH). |
| | | | **¹³C NMR (101 MHz, DMSO-*d₆*) δ :** 10.5 (2xCH_{3-CH2}), 22.9 (2xCH_{3 iPr}), 23.5 (CH_{iPr}), 26.5 (2xCH_{2-CH3}), 52.4 (CH_{iPent}), 83.4 (CH_{Ar}), 111.2 (C_{q}), 122.6 (CH_{Ar}), 129.9 (CH_{Ar}), 139.3 (C_{q}), 139.5 (CH_{Ar}), 145.5 (C_{q}), 150.1 (C_{q}), 156.0 (C_{q}), 156.4 (C_{q}). |
| | | | **MS (ESI+) : m/z calcd for C₁₈H₂₄ClN₇ :** 374.19 [M+H]+, **found :** 374.37. |
| (34) | 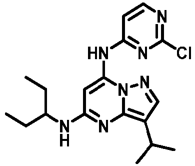 | > 99*%* ^{b} | White solid. |
| | | | **¹H NMR (400 MHz, DMSO-*d₆*) δ :** 0.89 (t, *J =* 7.3 Hz, 6H, 2xCH_{3-CH2}), 1.28 (d, *J* = 6.9 Hz, 6H, 2xCH_{3 iPr}), 1.43 - 1.70 (m, 4H, 2xCH_{2-CH3}), 2.99 (hept, *J =* 7.0 Hz, 1H, H_{iPr}), 3.90 (s, 1H, H_{iPent}), 7.13 (d, *J* = 9.2 Hz, 2H, NH_{iPent} & H_{Ar}), 7.56 (d, *J =* 5.8 Hz, 1H, H_{Ar}), 7.70 (s, 1H, H_{Ar}), 8.43 (d, *J =* 5.8 Hz, 1H, H_{Ar}), 10.55 (s, 1H, NH). |
| | | | **¹³C NMR (101 MHz, DMSO-*d₆*) δ :** 10.6 (2xCH_{3-CH2}), 22.9 (2xCH_{3 iPr}), 23.5 (CH_{iPr}), 26.6 (2xCH_{2-CH3}), 52.7 (CH_{iPent}), 85.1 (CH_{Ar}), 108.9 (CH_{Ar}), 111.3 (C_{q}), 138.7 (C_{q}), 139.8 (CH_{Ar}), 145.4 (C_{q}), 156.0 (C_{q}), 158.5 (CH_{Ar}), 158.8 (C_{q}), 160.9 (C_{q}). |
| | | | **MS (ESI+) : m/z calcd for C₁₈H₂₄ClN₇ :** 374.19 [M+H]+, **found :** 374.37. |
| (35) | 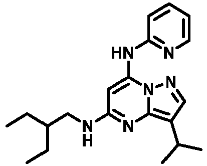 | > 93*%* ^{b} | White solid. |
| | | | **¹H NMR (400 MHz, DMSO-*d*₆) δ :** 0.89 (t, *J =* 7.4 Hz, 6H, 2xCH_{3-CH2}), 1.31 (dd, *J* = 17.1, 6.9 Hz, 10H, 2xCH_{2r-CH3} & 2xCH_{3 iPr}), 1.52 (p, *J =* 6.2 Hz, 1H, H_{C-CH2-NH}), 3.01 (hept, *J =* 6.9 Hz, 1H, H_{iPr}), 3.28 (t, *J* = 6.0 Hz, 2H, CH_{2-NH}), 7.02 (dd, *J =* 7.2, 5.0 Hz, 1H, H_{Ar}), 7.07 (t, *J =* 5.8 Hz, 1H, NH_{-CH2}), 7.28 (s, 1H, H_{Ar}), 7.52 (d, *J =* 8.3 Hz, 1H, H_{Ar}), 7.67 (s, 1H, H_{Ar}), 7.73 (ddd, *J =* 8.6, 7.2, 1.9 Hz, 1H, H_{Ar}), 8.34 (dd, *J* = 5.0, 1.8 Hz, 1H, H_{Ar}), 9.60 (s, 1H, NH). |
| | | | **¹³C NMR (101 MHz, DMSO-*d₆*) δ :** 11.0 (2xCH_{3-CH2}), 23.0 (2xCH_{3 iPr}), 23.5 (CH_{iPr}), 23.5 (2xCH_{2-CH3}), 40.4 (CH_{-CH2-NH}), 42.7 (CH_{2-NH}), 81.0 (CH_{Ar}), 111.2 (C_{q}), 113.9 (CH_{Ar}), 117.4 (CH_{Ar}), 137.9 (CH_{Ar}), 139.2 (CH_{Ar}), 140.3 (C_{q}), 145.4 (C_{q}), 147.1 (CH_{Ar}), 153.5 (C_{q}), 156.6 (C_{q}). |
| | | | **MS (ESI+) : m/z calcd for C₂₀H₂₈N₆O :** 353.49 [M+H]+, **found :** 353.44. |
| (36) | 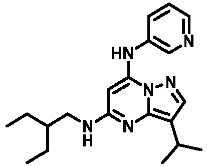 | > 99*%* ^{b} | White solid. |
| | | | **¹H NMR (400 MHz, DMSO-*d*₆) δ :** 0.87 (t, *J =* 7.4 Hz, 6H, 2xCH_{3-CH2}), 1.23 - 1.37 (m, 10H, 2xCH_{3 iPr} & 2xCH_{3-CH2}), 1.44 (hept, *J =* 6.3 Hz, 1H, H_{C-CH2-NH}), 3.00 (hept, *J =* 6.9 Hz, 1H, H_{iPr}), 3.25 (t, *J =* 5.8 Hz, 2H, CH_{2-NH}), 5.72 (s, 1H, H_{Ar}), 6.74 (t, *J =* 5.7 Hz, 1H, NH_{-CH2}), 7.45 (dd, *J =* 8.3, 4.8 Hz, 1H, H_{Ar}), 7.68 (s, 1H, H_{Ar}), 7.81 (ddd, *J =* 8.2, 2.7, 1.5 Hz, 1H, H_{Ar}), 8.38 (dd, *J* = 4.7, 1.5 Hz, 1H, H_{Ar}), 8.65 (d, *J* = 2.6 Hz, 1H, H_{Ar}), 9.28 (s, 1H, NH). |
| | | | **¹³C NMR (101 MHz, DMSO-*d₆*) δ :** 11.0 (2xCH_{3-CH2}), 23.0 (2xCH_{3 iPr}), 23.5 (CH_{iPr}), 23.5 (2xCH_{2-CH3}), 40.5 (CH_{-CH2-NH}), 42.3 (CH_{2-NH}), 75.0 (CH_{Ar}), 111.0 (C_{q}), 123.9 (CH_{Ar}), 130.4 (CH_{Ar}), 135.4 (C_{q}), 139.8 (CH_{Ar}), 143.7 (C_{q}), 144.9 (CH_{Ar}), 145.1 (CH_{Ar}), 145.9 (C_{q}), 156.6 (C_{q}). |
| | | | **MS (ESI+) : m/z calcd for C₂₀H₂₈N₆O :** 353.49 [M+H]+, **found :** 353.44. |
| (37) | 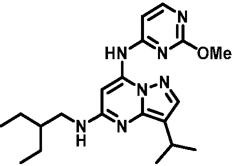 | > 99*%* ^{b} | White solid. |
| | | | **¹H NMR (400 MHz, DMSO-*d*₆) δ :** 0.90 (t, *J =* 7.4 Hz, 6H, 2xCH_{3-CH2}), 1.28 (d, *J* = 6.9 Hz, 6H, 2xCH_{3 iPr}), 1.34 (dt, *J =* 12.6, 7.0 Hz, 4H, 2xCH_{2-CH3}), 1.56 (hept, *J =* 6.2 Hz, 1H, H_{C-CH2-NH}), 3.00 (hept, *J =* 6.9 Hz, 1H, H_{iPr}), 3.28 (t, *J* = 6.0 Hz, 2H, CH₂₋co), 3.94 (s, 3H, CH_{3-O}), 7.21 (s, 1H, H_{Ar}), 7.21 (d, *J* = 5.7 Hz, 1H, H_{Ar}), 7.28 (t, *J* = 5.8 Hz, 1H, NH_{-CH2}), 7.69 (s, 1H, H_{Ar}), 8.31 (d, *J =* 5.7 Hz, 1H, H_{Ar}), 10.15 (s, 1H, NH). |
| | | | **¹³C NMR (101 MHz, DMSO-*d₆*) δ :** 10.9 (2xCH_{3-CH2}), 23.0 (2xCH_{3 iPr}), 23.5 (CH_{iPr}), 23.5 (2xCH_{2-CH3}), 40.2 (CH_{-CH2-NH}), 43.0 (CH_{2-NH}), 54.5 (CH_{3-O}), 84.4 (CH_{Ar}), 103.9 (CH_{Ar}), 111.3 (C_{q}), 139.1 (C_{q}), 139.6 (CH_{Ar}), 145.4 (C_{q}), 156.1 (C_{q}), 158.1 (CH_{Ar}), 161.0 (C_{q}), 164.5 (C_{q}). |
| | | | **MS (ESI+) : m/z calcd for C₂₀H₂₉N₇O :** 384.25 [M+H]+, **found :** 384.44. |
| (38) | 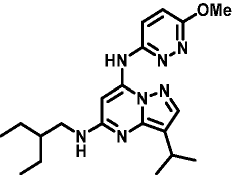 | > 99*%* ^{b} | White solid. |
| | | | **Rf** (DCM/EtOAc, 85/15) : 0.45. |
| | | | **¹H NMR (400 MHz, DMSO-*d*₆) δ :** 0.90 (t, *J =* 7.4 Hz, 6H, 2xCH_{3-CH2}), 1.29 (d, *J* = 6.9 Hz, 6H, 2xCH_{3 iPr}), 1.34 (dt, *J =* 14.3, 7.3 Hz, 4H, 2xCH_{2-CH3}), 1.51 (hept, *J =* 6.1 Hz, 1H, H_{C-CH2-NH}), 3.01 (hept, *J =* 6.9 Hz, 1H, H_{iPr}), 3.28 (t, *J =* 5.9 Hz, 2H, CH_{2-NH}), 3.99 (s, 3H, CH_{3-O}), 7.23 (t, *J =* 4.3 Hz, 1H, NH_{-CH2}), 7.24 (d, *J =* 4.3 Hz, 1H, H_{Ar}), 7.40 (s, 1H, H_{Ar}), 7.68 (s, 1H, H_{Ar}), 7.86 (d, *J =* 9.4 Hz, 1H, H_{Ar}), 9.82 (s, 1H, NH). |
| | | | **¹³C NMR (101 MHz, DMSO-*d₆*) δ :** 11.0 (2xCH_{3-CH2}), 23.0 (2xCH_{3 iPr}), 23.5 (CH_{iPr}), 23.6 (2xCH_{2-CH3}), 40.4 (CH_{-CH2-NH}), 42.6 (CH_{2-NH}), 54.2 (CH_{3-O}), 82.0 (CH_{Ar}), 111.2 (C_{q}), 119.6 (CH_{Ar}), 123.8 (CH_{Ar}), 139.3 (CH_{Ar}), 139.9 (C_{q}), 145.5 (C_{q}), 153.0 (C_{q}), 156.7 (C_{q}), 161.1 (C_{q}). |
| | | | **MS (ESI+) : m/z calcd for C₂₀H₂₉N₇O :** 384.25 [M+H]+, **found :** 384.43. |
| (39) | 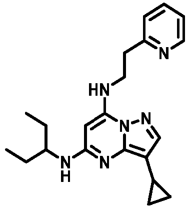 | > 96 *%* ^{b} | Brown oil. |
| | | | **Rf** (DCM/MeOH, 95/5) : 0.27. |
| | | | **¹H NMR (400 MHz, DMSO-*d₆*) δ :** 0.74 (tt, *J =* 8.3, 2.3 Hz, 4H, 2cCH_{2 cPr}), 0.87 (t, *J =* 7.4 Hz, 6H, 2xCH_{3-CH2}), 1.37 - 1.59 (m, 4H, 2xCH_{2-CH3}), 1.72 (tt, *J =* 8.2, 5.4 Hz, 1H, H_{cPr}), 3.09 (t, *J =* 7.2 Hz, 2H, CH_{2-CH2-NH}), 3.56 (q, *J =* 6.8 Hz, 2H, CH_{2-NH}), 3.79 (s, 1H, H_{iPent}), 5.30 (s, 1H, H_{Ar}), 6.40 (d, *J=* 8.2 Hz, 1H, NH_{iPent}), 7.06 (t, *J* = 6.0 Hz, 1H, NH_{-CH2}), 7.24 (dd, *J* = 7.5, 4.9 Hz, 1H, H_{Ar}), 7.33 (d, *J =* 7.8 Hz, 1H, H_{Ar}), 7.47 (s, 1H, H_{Ar}), 7.72 (td, *J =* 7.7, 1.9 Hz, 1H, H_{Ar}), 8.52 (dd, *J =* 5.0, 1.7 Hz, 1H, H_{Ar}). |
| | | | **¹³C NMR (101 MHz, DMSO-*d₆*) δ :** 5.4 (CH_{cPr}), 6.2 (2xCH_{2 cPr}), 10.4 (2xCH_{3-CH2}), 26.7 (2xCH_{2-CH3}), 36.2 (CH_{2-CH2-NH}), 41.0 (CH_{2-NH}), 52.1 (CH_{iPent}), 72.0 (CH_{Ar}), 105.2 (C_{q}), 121.6 (CH_{Ar}), 123.3 (CH_{Ar}), 136.6 (CH_{Ar}), 140.2 (CH_{Ar}), 145.9 (C_{q}), 146.0 (C_{q}), 149.1 (CH_{Ar}), 156.9 (C_{q}), 158.9 (C_{q}). |
| | | | **MS (ESI+) : m/z calcd for C₂₁H₂₈N₆ :** 365.25 [M+H]+, **found :** 365.43. |
| (40) | 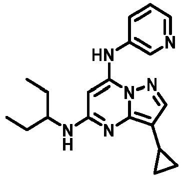 | > 98*%* ^{b} | White solid. |
| | | | **Rf** (DCM/MeOH, 95/5) : 0.27. |
| | | | **¹H NMR (400 MHz, DMSO-*d₆*) δ :** 0.73 - 0.83 (m, 4H, 2xCH_{2 cPr}), 0.85 (t, *J =* 7.7 Hz, 6H, 2xCH_{3-CH2}), 1.47 (ddq, *J =* 41.6, 14.0, 6.9 Hz, 4H, 2xCH_{2-CH3}), 1.78 (qd, *J* = 6.9, 4.5 Hz, 1H, H_{cPr}), 3.82 (s, 1H, H_{iPent}), 5.70 (s, 1H, H_{Ar}), 6.56 (d, *J* = 8.0 Hz, 1H, NH_{iPent}), 7.45 (dd, *J =* 8.3, 4.8 Hz, 1H, H_{Ar}), 7.62 (d, *J =* 1.8 Hz, 1H, H_{Ar}), 7.81 (d, *J =* 8.3 Hz, 1H, H_{Ar}), 8.37 (d, *J* = 4.7 Hz, 1H, H_{Ar}), 8.64 (d, *J =* 2.6 Hz, 1H, H_{Ar}), 9.24 (s, 1H, NH). |
| | | | **¹³C NMR (101 MHz, DMSO-de) δ :** 5.4 (CH_{cPr}), 6.3 (2xCH_{2 cPr}), 10.3 (2xCH_{3-CH2}), 26.5 (2xCH_{2-CH3}), 52.1 (CH_{iPent}), 75.1 (CH_{Ar}), 105.8 (C_{q}), 123.9 (CH_{Ar}), 130.4 (CH_{Ar}), 135.4 (C_{q}), 140.6 (CH_{Ar}), 143.7 (C_{q}), 144.9 (CH_{Ar}), 145.1 (CH_{Ar}), 146.3 (C_{q}), 156.6 (C_{q}). |
| | | | **MS (ESI+) : m/z calcd for C₁₉H₂₄N₆ :** 337.21 [M+H]+, **found :** 337.39. |
| (41) | 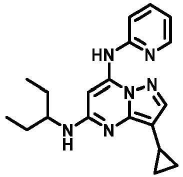 | > 99*%* ^{b} | White solid. |
| | | | **Rf** (DCM/EtOAc, 85/15) : 0.57. |
| | | | **¹H NMR (400 MHz, DMSO-*d₆*) δ :** 0.74 - 0.85 (m, 4H, 2xCH_{2 cPr}), 0.88 (t, *J* = 7.4 Hz, 6H, 2xCH_{3-CH2}), 1.53 (dtd, *J* = 41.1, 14.0, 7.3 Hz, 4H, 2xCH_{2-CH3}), 1.78 (td, *J* = 8.7, 8.2, 4.0 Hz, 1H, H_{cPr}), 3.86 (s, 1H, H_{iPent}), 6.85 (d, *J* = 8.1 Hz, 1H, NH_{iPent}), 6.97 - 7.09 (m, 1H, H_{Ar}), 7.26 (s, 1H, H_{Ar}), 7.50 (d, *J* = 8.3 Hz, 1H, H_{Ar}), 7.60 (s, 1H, H_{Ar}), 7.74 (t, *J =* 7.8 Hz, 1H, H_{Ar}), 8.34 (d, *J =* 5.0 Hz, 1H, H_{Ar}), 9.57 (s, 1H, NH). |
| | | | **¹³C NMR (101 MHz, DMSO-*d₆*) δ :** 5.4 (CH_{cPr}), 6.3 (2xCH_{2 cPr}), 10.5 (2xCH_{3-CH2}), 26.6 (2xCH_{2-CH3}), 52.3 (CH_{iPent}), 81.1 (CH_{Ar}), 106.0 (C_{q}), 113.9 (CH_{Ar}), 117.4 (CH_{Ar}), 137.9 (CH_{Ar}), 140.0 (CH_{Ar}), 140.3 (C_{q}), 145.8 (C_{q}), 147.0 (CH_{Ar}), 153.4 (C_{q}), 156.7 (C_{q}). |
| | | | **MS (ESI+) : m/z calcd for C₁₉H₂₄N₆ :** 337.21 [M+H]+, **found :** 337.36. |
| (42) | 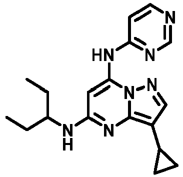 | 95*%* ^{b} | Yellow solid. |
| | | | **Rf** (EtOAc/Cyclo, 50/50) : 0.32. |
| | | | **¹H NMR (400 MHz, DMSO-*d₆*) δ :** 0.79 (dt, *J* = 8.4, 2.3 Hz, 2H, CH_{2 cPr}), 0.82 (dt, *J* = 4.9, 2.4 Hz, 2H, CH_{2 cPr}), 0.89 (t, *J =* 7.4 Hz, 6H, CH_{3 iPent}), 1.48 (dp, *J* = 14.5, 7.0 Hz, 2H, CH_{2 iPent}), 1.58 (ddd, *J =* 13.1, 7.5, 5.5 Hz, 2H, CH_{2 iPent}), 1.79 (tt, *J =* 8.2, 5.3 Hz, 1H, CH _{cPr}), 3.87 (s, 1H, CH _{iPent}), 7.03 (d, *J =* 8.1 Hz, 1H, NH), 7.34 (s, 1H, CH_{Ar}), 7.57 (dd, *J* = 5.9, 1.3 Hz, 1H, CH_{Ar}), 7.64 (s, 1H, CH_{Ar}), 8.52 (d, *J* = 5.8 Hz, 1H, CH_{Ar}), 8.86 (d, *J* = 1.2 Hz, 1H, CH_{Ar}), 10.16 (s, 1H, NH). |
| | | | **¹³C NMR (101 MHz, DMSO-*d₆*) δ :** 5.3 (CH_{cPr}), 6.3 (CH_{2 cPr}), 10.5 (CH_{3 iPent}), 26.5 (CH_{2 iPent}), 52.4 (CH _{iPent}), 84.3 (CH_{Ar}), 106.2 (C_{q}), 110.3 (CH_{Ar}), 139.2 (C_{q}), 140.3 (CH_{Ar}), 145.8 (C_{q}), 156.3 (CH_{Ar}), 156.4 (C_{q}), 157.5 (CH_{Ar}), 159.1 (C_{q}). |
| | | | **MS (ESI+) : m/z calcd for C₁₈H₂₃N₇** : 338.21 [M+H]+, **found :** 338.31. |
| (43) | 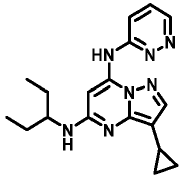 | > 95*%* ^{b} | Brown yellow solid. |
| | | | **Rf** (DCM/MeOH, 96/4) : 0.50. |
| | | | **¹H NMR (400 MHz, DMSO-*d₆*) δ :** 0.79 (dt, *J* = 8.4, 2.4 Hz, 2H, CH_{2 cPr}), 0.83 (dt, *J* = 5.5, 2.5 Hz, 2H, CH_{2 cPr}), 0.89 (t, *J =* 7.4 Hz, 6H, 2 X CH_{3 iPent}), 1.47 (dt, *J* = 13.9, 7.3 Hz, 2H, CH_{2 iPent}), 1.52 - 1.62 (m, 2H, CH_{2 iPent}), 1.80 (tt, *J =* 8.3, 5.3 Hz, 1H, CH _{cPr}), 3.88 (s, 1H, CH _{iPent}), 7.08 (d, *J =* 8.1 Hz, 1H, NH _{iPent}), 7.43 (s, 1H, CH_{Ar}), 7.63 (m, 2H, 2 X CH_{Ar}), 7.85 (dd, *J* = 9.0, 1.4 Hz, 1H, CH_{Ar}), 8.91 (dd, *J* = 4.6, 1.4 Hz, 1H, CH_{Ar}), 9.89 (s, 1H, NH). |
| | | | **¹³C NMR (101 MHz, DMSO-*d₆*) δ :** 5.4 (CH _{cPr}), 6.3 (CH_{2 cPr}), 10.5 (CH_{3 iPent}), 26.5 (CH_{2 iPent}), 52.3 (CH _{iPent}), 82.9 (CH_{Ar}), 106.1 (C_{q}), 118.9 (CH_{Ar}), 128.2 (CH_{Ar}), 139.6 (C_{q}), 140.1 (CH_{Ar}), 145.9 (C_{q}), 147.1 (CH_{Ar}), 156.4 (C_{q}), 156.7 (C_{q}). |
| | | | **MS (ESI+) : m/z calcd for C₁₈H₂₃N₇ :** 338.21 [M+H]+, **found :** 338.31. |
| (44) | 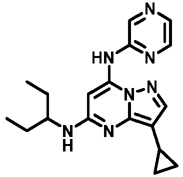 | > 95*%* ^{b} | Orange solid. |
| | | | **Rf** (DCM/MeOH, 96/4) : 0.66 |
| | | | **¹H NMR (400 MHz, DMSO-*d₆*) δ :** 0.79 (dt, *J* = 8.4, 2.3 Hz, 2H, CH_{2 cPr}), 0.82 (dt, *J* = 5.2, 2.4 Hz, 2H, CH_{2 cPr}), 0.88 (t, *J =* 7.4 Hz, 6H, CH_{3 iPent}), 1.47 (dt, *J =* 13.9, 7.2 Hz, 2H, CH_{2 iPent}), 1.51 - 1.62 (m, 2H, CH_{2 iPent}), 1.79 (ddd, *J* = 13.7, 8.3, 5.2 Hz, 1H, CH _{cPr}), 3.87 (s, 1H, CH _{iPent}), 6.94 (d, *J =* 8.1 Hz, 1H, NH), 7.21 (s, 1H, CH_{Ar}), 7.64 (s, 1H, CH_{Ar}), 8.21 (d, *J* = 2.8 Hz, 1H, CH_{Ar}), 8.33 (s, 1H, CH_{Ar}), 8.88 (d, *J =* 1.4 Hz, 1H, CH_{Ar}), 10.09 (s, 1H, NH). |
| | | | **¹³C NMR (101 MHz, DMSO-*d₆*) δ :** 5.3 (CH_{cPr}), 6.3 (CH_{2 cPr}), 10.5 (CH_{3 iPent}), 26.5 (CH_{2 iPent}), 52.3 (CH _{iPent}), 81.9 (CH_{Ar}), 106.1 (C_{q}), 136.8 (CH_{Ar}), 137.4 (CH_{Ar}), 139.8 (C_{q}), 140.2 (CH_{Ar}), 140.8 (CH_{Ar}), 145.8 (C_{q}), 150.2 (C_{q}), 156.5 (C_{q}). |
| | | | **MS (ESI+) : m/z calcd for C₁₈H₂₃N₇ :** 338.21 [M+H]+, **found :** 338.31. |
| (45) | 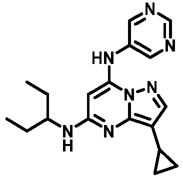 | > 97*%* ^{b} | White solid. |
| | | | **Rf** (DCM/MeOH, 95/5) : 0.13. |
| | | | **¹H NMR (400 MHz, DMSO-*d₆*) δ :** 0.78 (dt, *J =* 8.4, 2.3 Hz, 2H, 2xH_{(CH2) cPr}), 0.82 (dt, *J =* 5.0, 2.4 Hz, 2H, 2xH_{(CH2) cPr}), 0.86 (t, *J =* 7.4 Hz, 6H, 2xCH_{3-CH2}), 1.35 - 1.61 (m, 4H, 2xCH_{2-CH3}), 1.79 (tt, *J =* 8.3, 5.2 Hz, 1H, CH_{cPr}), 3.83 (s, 1H, H_{iPent}), 5.81 (s, 1H, NH_{iPent}), 6.59 (d, *J =* 8.1 Hz, 1H, H_{Ar}), 7.64 (s, 1H, H_{Ar}), 8.90 (s, 2H, 2xH_{Ar}), 8.97 (s, 1H, H_{Ar}), 9.45 (s, 1H, NH). |
| | | | **¹³C NMR (101 MHz, DMSO-*d₆*) δ :** 5.4 (CH_{cPr}), 6.3 (2xCH_{2 cPr}), 10.3 (2xCH_{3-CH2}), 26.4 (2xCH_{2-CH3}), 52.1 (CH_{iPent}), 75.7 (CH_{Ar}), 106.0 (C_{q}), 134.5 (C_{q}), 140.8 (CH_{Ar}), 142.9 (C_{q}), 146.3 (C_{q}), 150.9 (2xCH_{Ar}), 153.5 (CH_{Ar}), 156.5 (C_{q}). |
| | | | **MS (ESI+) : m/z calcd for C₁₈H₂₃N₇ :** 338.21 [M+H]+, **found :** 338.38. |
| (46) | 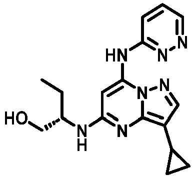 | > 98 *%* ^{a} | Grey solid. |
| | | | **¹H NMR (400 MHz, DMSO-*d₆*) δ :** 0.77 - 0.83 (m, 4H, 2xCH_{2 cPr}), 0.91 (t, *J =* 7.4 Hz, 3H, CH_{3-CH2}), 1.46 (dp, *J* = 15.0, 7.5 Hz, 1H, H_{(CH2)-CH3}), 1.69 (ddd, *J =* 13.1, 7.6, 5.3 Hz, 1H, H_{(CH2)-CH3}), 1.81 (p, *J =* 6.8 Hz, 1H, H_{cPr}), 3.46 (ddt, *J* = 32.6, 11.1, 5.6 Hz, 2H, CH_{2-CH3}), 3.87 - 4.06 (m, 1H, OH), 4.67 (t, *J =* 5.5 Hz, 1H, H_{C-NH}), 7.07 (d, *J* = 7.9 Hz, 1H, H_{Ar}), 7.44 (s, 1H, H_{Ar}), 7.63 (q, *J =* 4.6 Hz, 1H, H_{Ar}), 7.64 (s, 1H, H_{Ar}), 7.85 (dd, *J* = 9.0, 1.4 Hz, 1H, H_{Ar}), 8.91 (dd, *J* = 4.6, 1.4 Hz, 1H, H_{Ar}), 9.92 (s, 1H, NH). |
| | | | **¹³C NMR (101 MHz, DMSO-*d₆*) δ :** 5.3 (CH_{cPr}), 6.4 (CH_{2 cPr}), 6.5 (CH_{2 cPr}), 10.5 (CH_{3-CH2}), 23.8 (CH_{2-CH3}), 53.4 (CH_{-NH}), 62.9 (CH_{2-OH}), 83.0 (CH_{Ar}), 106.3 (C_{q}), 118.9 (CH_{Ar}), 128.2 (CH_{Ar}), 139.7 (C_{q}), 140.0 (CH_{Ar}), 145.9 (C_{q}), 147.1 (CH_{Ar}), 156.4 (C_{q}), 156.7 (C_{q}). |
| | | | **MS (ESI+) : m/z calcd for C₁₇H₂₁N₇O** : 340.19 [M+H]+, **found :** 340.35. |
| (47) | 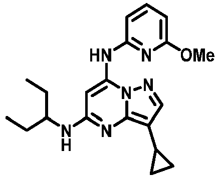 | > 98*%* ^{b} | White solid. |
| | | | **Rf** (DCM/MeOH, 96/4) : 0.83 |
| | | | **¹H NMR (400 MHz, DMSO-*d₆*) δ :** 0.80 (dq, *J =* 8.4, 2.8, 2.2 Hz, 4H, 2 X CH_{2 cPr}), 0.89 (t, *J =* 7.4 Hz, 6H, 2 X CH_{3 iPent}), 1.49 (dt, *J* = 14.0, 7.3 Hz, 2H, CH_{2 iPent}), 1.59 (ddd, *J* = 13.1, 7.5, 5.5 Hz, 2H, CH_{2 iPent}), 1.80 (tt, *J =* 8.1, 5.6 Hz, 1H, CH _{cPr}), 3.85 (s, 1H, CH _{iPent}), 3.95 (s, 3H, CH₃-O), 6.45 (d, *J =* 8.0 Hz, 1H, NH), 6.78 (s, 1H, CH_{Ar}), 6.99 (s, 1H, CH_{Ar}), 7.04 (d, *J =* 7.8 Hz, 1H, CH_{Ar}), 7.62 (s, 1H, CH_{Ar}), 7.66 (t, *J* = 7.9 Hz, 1H, CH_{Ar}), 9.52 (s, 1H, NH). |
| | | | **¹³C NMR (101 MHz, DMSO-*d₆*) δ :** 5.3 (CH _{cPr}), 6.4 (CH_{2 cPr}), 10.6 (CH_{3 iPent}), 26.7 (CH_{2 iPent}), 52.8 (CH _{iPent}), 53.9 (CH₃), 80.7 (CH_{Ar}), 102.5 (CH_{Ar}), 105.5 (CH_{Ar}), 106.0 (C_{q}), 140.3 (CH_{Ar}), 140.6 (C_{q}), 140.7 (CH_{Ar}), 145.6 (C_{q}), 151.4 (C_{q}), 156.5 (C_{q}), 162.6 (C_{q}). |
| | | | **MS (ESI+) : m/z calcd for C₂₀H₂₆N₆O :** 367.22 [M+H]+, **found :** 367.32. |
| (48) | 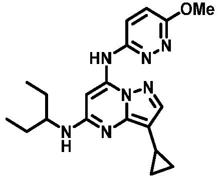 | > 94 *%* ^{a} | White solid. |
| | | | **Rf** (EtOAc, Cyclohexane, 50/50) : 0.67. |
| | | | **¹H NMR (400 MHz, DMSO-*d₆*) δ :** 0.75 - 0.81 (m, 2H, CH_{2 cPr}), 0.82 (dd, *J* = 5.5, 3.1 Hz, 2H, CH_{2 cPr}), 0.88 (t, *J* = 7.3 Hz, 6H, CH_{3 iPent}), 1.46 (dt, *J* = 13.9, 7.3 Hz, 2H, CH_{2 iPent}), 1.51 - 1.63 (m, 2H, CH_{2 iPent}), 1.79 (ddd, *J* = 13.8, 8.3, 5.2 Hz, 1H, CH_{cPr}), 3.87 (s, 1H, CH _{iPent}), 3.98 (s, 3H, O-CH₃), 7.03 (d, *J* = 8.1 Hz, 1H, NH), 7.24 (d, *J =* 9.4 Hz, 1H, CH_{Ar}), 7.38 (s, 1H, CH_{Ar}), 7.62 (s, 1H, CH_{Ar}), 7.85 (d, *J* = 9.5 Hz, 1H, CH_{Ar}), 9.79 (s, 1H, NH). |
| | | | **¹³C NMR (101 MHz, DMSO-*d₆*) δ :** 5.4 (CH_{cPr}), 6.3 (CH_{2 cPr}), 10.4 (CH_{3 iPent}), 26.5 (CH_{2 iPent}), 52.3 (CH _{iPent}), 54.1 (O-CH₃), 82.1 (CH_{Ar}), 106.0 (C_{q}), 119.6 (CH_{Ar}), 123.8 (CH_{Ar}), 139.9 (C_{q}), 140.0 (CH_{Ar}), 145.9 (C_{q}), 153.0 (C_{q}), 156.8 (C_{q}), 161.1 (C_{q}). |
| | | | **MS (ESI+) : m/z calcd for C₁₉H₂₆N₇O** : 368.22 [M+H]+, **found :** 368.31. |
| (49) | 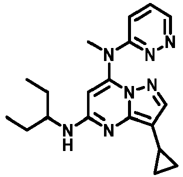 | > 98*%* ^{b} | Yellow solid. |
| | | | **Rf** (EtOAc/Cyclo, 70/30) : 0.13. |
| | | | **¹H NMR (400 MHz, DMSO-*d₆*) δ :** 0.73 - 0.80 (m, 4H, 2 X CH_{2 cPr}), 0.89 (t, *J* = 7.4 Hz, 6H, 2 X CH_{3 iPent}), 1.41 - 1.63 (m, 4H, 2 X CH_{2 iPent}), 1.75 (ddd, *J =* 13.7, 8.3, 5.6 Hz, 1H, CH _{cPr}), 3.79 (s, 3H, N-CH₃), 3.83 (s, 1H, CH _{iPent}), 5.63 (s, 1H, CH_{Ar}), 6.56 (d, *J=* 8.2 Hz, 1H, CH_{Ar}), 6.64 (dd, *J* = 9.5, 1.7 Hz, 1H, CH_{Ar}), 7.13 (dd, *J =* 9.4, 3.9 Hz, 1H, CH_{Ar}), 7.39 (s, 1H, CH_{Ar}), 7.84 (dd, *J =* 3.9, 1.6 Hz, 1H, NH). |
| | | | **¹³C NMR (101 MHz, DMSO-*d₆*) δ :** N.D. MS (ESI+) : **m/z calcd for C₁₄H₁₅ClN₆O :** 352.22 [M+H]+, **found :** 352.43. |
| (50) | 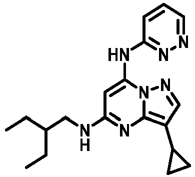 | > 99*%* ^{b} | white solid. |
| | | | **Rf** (EtOAc/Cyclo, 70/30) : 0.67 |
| | | | **¹H NMR (400 MHz, DMSO-*d₆*) δ :** 0.81 (ddd, *J =* 12.7, 6.7, 2.2 Hz, 4H, CH_{2 cPr}), 0.90 (td, *J =* 7.4, 1.7 Hz, 6H, CH_{3 iPent}), 1.26 - 1.42 (m, *J =* 7.0 Hz, 4H, 2 X CH_{2 iPent}), 1.51 (h, *J =* 6.4 Hz, 1H, CH _{iPent}), 1.81 (ddt, *J =* 13.0, 6.9, 3.4 Hz, 1H, CH _{cPr}), 3.28 (t, *J =* 6.0 Hz, 2H, CH₂-NH), 7.27 (t, *J =* 5.8 Hz, 1H, NH), 7.44 (d, *J* = 1.8 Hz, 1H, CH_{Ar}), 7.56 - 7.68 (m, 2H, 2 X CH_{Ar}), 7.85 (d, *J =* 9.0 Hz, 1H, CH_{Ar}), 8.91 (d, *J =* 4.5 Hz, 1H, CH_{Ar}), 9.90 (s, 1H, NH). |
| | | | **¹³C NMR (101 MHz, DMSO-*d₆*) δ :** 5.3 (CH _{cPr}), 6.3 (2 X CH_{2 cPr}), 11.0 (2 X CH_{3 iPent}), 23.5 (2 X CH_{2 iPent}), 40.3 (CH _{iPent}), 42.8 (CH₂-NH), 82.8 (CH_{Ar}), 106.3 (C_{q}), 118.9 (CH_{Ar}), 128.2 (CH_{Ar}), 139.6 (C_{q}), 140.1 (CH_{Ar}), 145.9 (C_{q}), 147.1 (CH_{Ar}), 156.4 (C_{q}), 156.7 (C_{q}). |
| | | | **MS (ESI+) : m/z calcd for C₁₄H₁₅ClN₆O :** 352.22 [M+H]+, **found :** 352.43. |
| (51) | 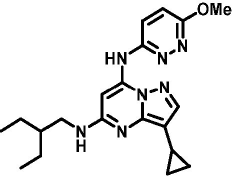 | > 99*%* ^{b} | Red solid. |
| | | | **Rf** (EtOAc/Cyclo, 50/50) : 0.21 |
| | | | **¹H NMR (400 MHz, DMSO-*d₆*) δ :** 0.75 - 0.85 (m, 4H, 2 X CH_{2 cPr}), 0.90 (t, *J =* 7.4 Hz, 6H, 2 X CH_{3 iPent}), 1.34 (dq, *J =* 13.9, 7.2 Hz, 4H, 2 X CH_{2 iPent}), 1.51 (p, *J* = 6.2 Hz, 1H, CH _{iPent}), 1.80 (tt, *J =* 8.3, 5.4 Hz, 1H, CH _{cPr}), 3.27 (d, *J =* 11.9 Hz, 2H, CH₂-NH), 3.99 (s, 3H, O-CH₃), 7.21 (d, *J =* 5.8 Hz, 1H, CH_{Ar}), 7.25 (d, *J =* 9.5 Hz, 1H, CH_{Ar}), 7.39 (s, 1H, CH_{Ar}), 7.62 (s, 1H, CH_{Ar}), 7.85 (d, *J =* 9.4 Hz, 1H, CH_{Ar}), 9.81 (s, 1H, NH). |
| | | | **¹³C NMR (101 MHz, DMSO-*d₆*) δ :** 5.3 (CH _{cPr}), 6.3 (2 X CH_{2 cPr}), 11.0 (2 X CH_{3 iPent}), 23.5 (2 X CH_{2 iPent}), 40.3 (CH _{iPent}), 42.7 (CH₂-NH), 54.2 (O-CH₃), 82.0 (CH_{Ar}), 106.3 (C_{q}), 119.6 (CH_{Ar}), 123.8 (CH_{Ar}), 139.9 (C_{q}), 140.0 (CH_{Ar}), 145.9 (C_{q}), 152.9 (C_{q}), 156.9 (C_{q}), 161.1 (C_{q}). |
| | | | **MS (ESI+) : m/z calcd for C₁₄H₁₅ClN₆O :** 382.24 [M+H]+, **found :** 382.43. |
| (52) | 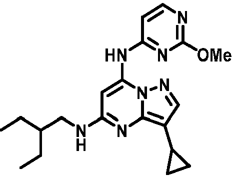 | > 96*%* ^{b} | Yellow solid. |
| | | | **Rf** (DCM/MeOH, 98/2) : 0.35 |
| | | | **¹H NMR (400 MHz, DMSO-*d₆*) δ :** 0.79 (tt, *J* = 8.4, 2.3 Hz, 4H, 2 X CH_{2 cPr}), 0.90 (t, *J* = 7.4 Hz, 6H, 2 X CH_{3 iPent}), 1.34 (dp, *J* = 13.9, 6.8 Hz, 4H, 2X CH_{2 iPent}), 1.55 (p, *J* = 6.3 Hz, 1H, CH _{iPent}), 1.79 (tt, *J* = 8.2, 5.4 Hz, 1H, CH _{cPr}), 3.26 (t, *J =* 5.9 Hz, 2H, CH₂-NH), 3.94 (s, 3H, O-CH₃), 7.19 (d, *J* = 5.7 Hz, 2H, 2 X CH_{Ar}), 7.26 (t, *J =* 5.7 Hz, 1H, NH), 7.64 (s, 1H, CH_{Ar}), 8.30 (d, *J* = 5.7 Hz, 1H, CH_{Ar}), 10.12 (s, 1H, NH). |
| | | | **¹³C NMR (101 MHz, DMSO-*d₆*) δ :** 4.9 (CH_{cPr}), 6.0 (2 X CH_{2 cPr}), 10.6 (2 X CH_{3 iPent}), 23.1 (2 X CH_{2 iPent}), 39.9 (CH _{iPent}), 42.8 (CH₂-NH), 54.2 (O-CH₃), 84.2 (CH_{Ar}), 103.6 (CH_{Ar}), 106.1 (C_{q}), 138.8 (C_{q}), 140.0 (CH_{Ar}), 145.5 (C_{q}), 155.9 (C_{q}), 157.8 (CH_{Ar}), 160.7 (C_{q}), 164.2 (C_{q}). |
| | | | **MS (ESI+) : m/z calcd for C₁₄H₁₅ClN₆O :** 382.24 [M+H]+, **found :** 382.48. |
| (53) | 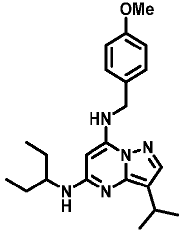 | > 99*%* ^{b} | White solid. |
| | | | **Rf** (DCM, 100*%*) : 0.14. |
| | | | **¹H NMR (400 MHz, DMSO-*d*₆) δ :** 0.82 (t, *J =* 7.4 Hz, 6H, 2xCH_{3-CH3}), 1.24 (d, *J* = 6.9 Hz, 6H, 2xCH_{3 iPr}), 1.33 - 1.57 (m, 4H, 2xCH_{2-CH3}), 2.93 (hept, *J =* 6.8 Hz, 1H, H_{iPr}), 3.72 (s, 3H, CH_{3-O}), 3.76 (s, 1H, H_{iPent}), 4.35 (d, *J* = 6.4 Hz, 2H, CH_{2 Bn}), 5.15 (s, 1H, H_{Ar}), 6.32 (d, *J =* 8.2 Hz, 1H, NH_{iPent}), 6.84 - 6.95 (m, 2H, 2xH_{Ar}), 7.23 - 7.34 (m, 2H, 2xH_{Ar}), 7.56 (s, 1H, H_{Ar}), 7.61 (t, *J =* 6.4 Hz, 1H, NH_{Bn}). |
| | | | **¹³C NMR (101 MHz, DMSO-*d₆*) δ :** 10.4 (2xCH_{3-CH2}), 23.0 (2xCH_{3 iPr}), 23.5 (CH_{iPr}), 26.6 (2xCH_{2-CH3}), 44.0 (CH_{2 Bn}), 52.2 (CH_{iPent}), 55.0 (CH_{3-O}), 72.6 (CH_{Ar}), 110.2 (C_{q}), 113.8 (2xCH_{Ar}), 128.1 (2xCH_{Ar}), 130.5 (C_{q}), 139.5 (CH_{Ar}), 145.5 (C_{q}), 145.9 (C_{q}), 156.6 (C_{q}), 158.3 (C_{q}). |
| | | | **MS (ESI+) : m/z calcd for C₂₂H₃₁N₅O** : 382.26 [M+H]+, **found :** 382.46. |
| (54) | 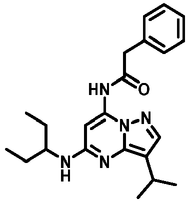 | > 99*%* ^{b} | Brown oil. |
| | | | **¹H NMR (400 MHz, DMSO-*d₆*) δ :** 0.85 (t, *J =* 7.4 Hz, 6H, 2xCH_{3-CH2}), 1.26 (d, *J* = 6.9 Hz, 6H, 2xCH_{3 iPr}), 1.38 - 1.61 (m, 4H, 2xCH_{2-CH3}), 2.96 (hept, *J* = 6.8 Hz, 1H, H_{iPr}), 3.86 (s, 1H, H_{iPent}), 3.97 (s, 2H, CH_{2-CO}), 6.98 (s, 1H, H_{Ar}), 7.02 (d, *J =* 8.1 Hz, 1H, NH_{iPent}), 7.22 - 7.29 (m, 1H, H_{Ar}), 7.30 - 7.40 (m, 4H, 4xH_{Ar}), 7.66 (s, 1H, H_{Ar}), 10.47 (s, 1H, NH_{-CO}). |
| | | | **¹³C NMR (101 MHz, DMSO-*d₆*) δ :** 10.4 (2xCH_{3-CH2}), 22.9 (2xCH_{3 iPr}), 23.4 (CH_{iPr}), 26.4 (2xCH_{2-CH3}), 42.9 (CH_{2-CO}), 52.4 (CH_{iPent}), 85.2 (CH_{Ar}), 111.2 (C_{q}), 126.8 (CH_{Ar}), 128.4 (2xCH_{Ar}), 129.3 (2xCH_{Ar}), 135.2 (C_{q}), 138.5 (C_{q}), 139.8 (CH_{Ar}), 145.3 (C_{q}), 156.0 (C_{q}), 170.8 (C_{q}). |
| | | | **MS (ESI+) : m/z calcd for C₂₂H₂₉N₅O** : 380.25 [M+H]+, **found :** 380.45. |
| (55) | 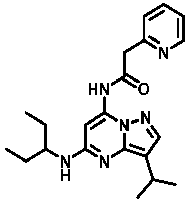 | 93 *%* ^{b} | Yellow solid. |
| | | | **¹H NMR (400 MHz, DMSO-*d₆*) δ :** 0.85 (t, *J =* 7.4 Hz, 6H, 2xCH_{3-CH2}), 1.26 (d, *J* = 6.9 Hz, 6H, 2xCH_{3 iPr}), 1.37 - 1.68 (m, 4H, 2xCH_{2-CH3}), 2.96 (h, *J =* 6.9 Hz, 1H, H_{iPr}), 3.86 (s, 1H, H_{iPent}), 4.14 (s, 2H, CH₂₋co), 6.99 (s, 1H, H_{Ar}), 7.03 (d, *J =* 8.1 Hz, 1H, NH_{iPent}), 7.32 (ddd, *J* = 7.6, 4.9, 1.2 Hz, 1H, H_{Ar}), 7.43 (dd, *J =* 7.9, 1.1 Hz, 1H, H_{Ar}), 7.67 (s, 1H, H_{Ar}), 7.80 (td, *J =* 7.7, 1.9 Hz, 1H, H_{Ar}), 8.56 (ddd, *J =* 4.8, 1.9, 0.9 Hz, 1H, H_{Ar}), 11.05 (s, 1H, NH_ co). |
| | | | **¹³C NMR (101 MHz, DMSO-*d₆*) δ :** 10.4 (2xCH_{3-CH2}), 22.9 (2xCH_{3 iPr}), 23.5 (CH_{3 iPr}), 26.4 (2xCH_{2-CH3}), 45.3 (CH_{2-CO}), 52.4 (CH_{iPr}), 84.9 (CH_{Ar}), 111.3 (C_{q}), 122.3 (CH_{Ar}), 124.2 (CH_{Ar}), 137.2 (CH_{Ar}), 138.4 (C_{q}), 140.0 (CH_{Ar}), 145.3 (C_{q}), 149.0 (CH_{Ar}), 155.1 (C_{q}), 156.0 (C_{q}), 169.3 (C_{q}). |
| | | | **MS (ESI+) : m/z calcd for C₂₁H₂₈N₆O** : 381.24 [M+H]+, **found :** 381.40. |
| (56) | 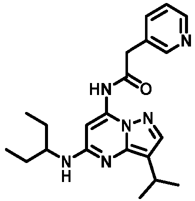 | > 95*%* ^{b} | White solid. |
| | | | **¹H NMR (400 MHz, DMSO-*d₆*) δ :** 0.85 (t, *J =* 7.4 Hz, 6H, 2xCH_{3-CH2}), 1.26 (d, *J* = 6.9 Hz, 6H, 2xCH_{3 iPr}), 1.36 - 1.63 (m, 4H, 2xCH_{2-CH3}), 2.97 (hept, *J* = 6.9 Hz, 1H, H_{iPr}), 3.86 (s, 1H, H_{iPent}), 4.04 (s, 2H, CH_{2-CO}), 6.98 (s, 1H, H_{Ar}), 7.01 (d, *J =* 8.1 Hz, 1H, NH_{iPent}), 7.36 (ddd, *J* = 7.8, 4.8, 0.9 Hz, 1H, H_{Ar}), 7.68 (s, 1H, H_{Ar}), 7.76 (dt, *J =* 7.8, 2.0 Hz, 1H, H_{Ar}), 8.47 (dd, *J* = 4.8, 1.7 Hz, 1H, H_{Ar}), 8.55 (d, *J =* 2.2 Hz, 1H, H_{Ar}), 10.67 (s, 1H, NH_{-CO}). |
| | | | **¹³C NMR (101 MHz, DMSO-*d₆*) δ :** 10.4 (2xCH_{3-CH2}), 22.9 (2xCH_{3 iPr}), 23.4 (CH_{iPr}), 26.4 (2xCH_{2-CH3}), 39.9 (CH_{2-CO}), 52.4 (CH_{iPent}), 85.5 (CH_{Ar}), 111.2 (C_{q}), 123.5 (CH_{Ar}), 131.0 (C_{q}), 136.9 (CH_{Ar}), 138.5 (C_{q}), 139.8 (CH_{Ar}), 145.4 (C_{q}), 148.0 (CH_{Ar}), 150.3 (CH_{Ar}), 155.9 (C_{q}), 170.3 (C_{q}). |
| | | | **MS (ESI+) : m/z calcd for C₂₁H₂₈N₆O :** 381.24 [M+H]+, **found :** 381.41. |
| (57) | 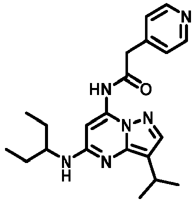 | > 97*%* ^{b} | Light yellow solid. |
| | | | **¹H NMR (400 MHz, DMSO-*d₆*) δ :** 0.85 (t, *J =* 7.4 Hz, 6H, 2xCH_{3-CH2}), 1.26 (d, *J* = 6.9 Hz, 6H, 2xCH_{3 iPr}), 1.38 - 1.62 (m, 4H, 2xCH_{2-CH3}), 2.97 (hept, *J =* 6.9 Hz, 1H, H_{iPr}), 3.86 (s, 1H, H_{iPent}), 4.05 (s, 2H, CH_{2-CO}), 6.98 (s, 1H, H_{Ar}), 7.02 (d, *J =* 8.1 Hz, 1H, NH_{iPent}), 7.31 - 7.38 (m, 2H, 2xH_{Ar}), 7.68 (s, 1H, H_{Ar}), 8.43 - 8.58 (m, 2H, 2xH_{Ar}), 10.70 (s, 1H, NH_{-CO}). |
| | | | **¹³C NMR (101 MHz, DMSO-*d₆*) δ :** 10.4 (2xCH_{3-CH2}), 22.9 (2xCH_{3 iPr}), 23.5 (CH_{iPr}), 26.4 (2xCH_{2-CH3}), 42.0 (CH_{2-CO}), 52.4 (CH_{iPent}), 85.6 (CH_{Ar}), 111.3 (C_{q}), 124.7 (2xCH_{Ar}), 138.5 (C_{q}), 139.9 (CH_{Ar}), 144.1 (C_{q}), 145.4 (C_{q}), 149.6 (2xCH_{Ar}), 156.0 (C_{q}), 169.7 (C_{q}). |
| | | | **MS (ESI+) : m/z calcd for C₂₁H₂₈N₆O :** 381.24 [M+H]+, **found :** 381.41. |
| (58) | 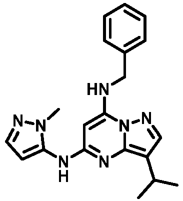 | > 99 *%* ^{a} | White solid. |
| | | | **¹H NMR (400 MHz, DMSO-*d₆*) δ :** 1.26 (d, *J =* 6.9 Hz, 6H, 2xCH_{3 iPr}), 2.99 (hept, *J =* 7.4 Hz, 1H, H_{iPr}), 3.62 (s, 3H, CH_{3-N}), 4.49 (d, *J=* 6.4 Hz, 2H, CH_{2 Bn}), 5.41 (s, 1H, H_{Ar}), 6.24 (d, *J=* 1.9 Hz, 1H, H_{Ar}), 7.21 - 7.42 (m, 6H, 6xH_{Ar}), 7.76 (s, 1H, H_{Ar}), 8.19 (t, *J=* 6.3 Hz, 1H, NH_{Bn}), 8.79 (s, 1H, NH). |
| | | | **¹³C NMR (101 MHz, DMSO-*d₆*) δ :** 23.2 (2xCH_{3 iPr}), 23.4 (CH_{iPr}), 35.4 (CH_{3-N}), 44.6 (CH_{2 Bn}), 72.9 (CH_{Ar}), 97.0 (CH_{Ar}), 112.0 (C_{q}), 126.7 (2xCH_{Ar}), 127.1 (CH_{Ar}), 128.5 (2xCH_{Ar}), 137.2 (CH_{Ar}), 138.2 (C_{q}), 138.8 (C_{q}), 140.4 (CH_{Ar}), 144.6 (C_{q}), 146.9 (C_{q}), 153.2 (C_{q}). |
| | | | **MS (ESI+) : m/z calcd for C₂₀H₂₃N₇ :** 362.21 [M+H]+, **found :** 362.33 |

| | | | |
|---|---|---|---|
| ^{a}Acidic conditions, ^{b}Alkaline conditions | | | |

### PATHOLOGIES

Examples of diseases mediated A₃AR include, but are not limited to, ulcerative colitis, atopic dermatitis, glaucoma, asthma, liver fibrosis, kidney diseases in particular kidney fibrosis, nephropathy, acute kidney injury (AKI), chronic kidney disease (CKD) more particularly induced by ischemia and/or reperfusion injury, toxic nephropathy or myoglobinuria, diabetic kidney disease, kidney diseases induced by nephrotoxicity caused by therapeutic drugs, atherosclerosis, hypercholestemia, partial or a complete hearing loss induced by noise, acoustic trauma or ototoxic agents or conditions, dementia, in particular vascular dementia, and cancers developing under hypoxic conditions, in particular glioblastoma.

The compounds of formula (I) or any of its pharmaceutically acceptable salts, or at least any of compounds (1) to (58) as defined above or any of their pharmaceutically acceptable salts, may in particular be useful in the prevention and/or the treatment of ulcerative colitis, atopic dermatitis, glaucoma, asthma, liver fibrosis, kidney diseases, atherosclerosis, hypercholestemia, hearing loss, dementia, in particular vascular dementia, and cancers developing under hypoxic conditions, in particular glioblastoma.

The compounds of formula (I) or any of its pharmaceutically acceptable salts, or at least any of compounds (1) to (58) as defined above or any of their pharmaceutically acceptable salts, may in particular be useful in the treatment and/or in the prevention of hearing loss, in particular induced by ototoxic agents, as well as in the treatment and/or in the prevention of kidney diseases, in particular induced by nephrotoxicity.

The compounds of formula (I) or any of its pharmaceutically acceptable salts, or at least any of compounds (1) to (58) as defined above or any of their pharmaceutically acceptable salts, may in particular be useful in the treatment and/or in the prevention of partial or a complete hearing loss, in particular induced by noise, acoustic trauma or ototoxic agents or conditions, more particularly induced by ototoxic agents or conditions.

The compounds of formula (I) or any of its pharmaceutically acceptable salts, or at least any of compounds (1) to (58) as defined above or any of their pharmaceutically acceptable salts, may in particular be useful in the treatment and/or in the prevention of partial or a complete hearing loss induced by ototoxic agents or conditions as described hereinafter.

As ototoxic agents, i.e. causing hear loss, the following may be cited:
- solvents, such as polyethylene glycol, propylene glycol or benzalkonium chloride;
- antibiotics, in particular topical and/or systemic antibiotics, more particularly aminoglycosides, macrolides or phenicol antibiotics, such as gentamycin, neomycin, tobramycin, kanamycin, nystatin, polymyxin B, amphotericin B, bacitracin or chloramphenicol;
- anticancer drugs, in particular platinum-based anticancer drugs, such as cisplatin or carboplatin,
- antiseptics, in particular acetic acid, alcohols such as ethanol, chlorhexidine, cresylate, gentian violet or povidone iodine;
- nonsteroidal anti-inflammatory drugs (NSAIDs), in particular salicylates, cyclodextrins, indomethacin, ibuprofen, phenylbutazone or paracetamol;
- topical combinations, in particular polymyxin/neomycin/hydrocortisone or ticarcilline/clavulanate;
- drugs for COVID-19, in particular lopinavir or ritonavir;
- antimalarial drugs, in particular quinine or chloroquine;
- cardiovascular drugs, in particular loop diuretics; or
- erectile dysfunction drugs, in particular phosphodiesterase type 5 (PDE-5) inhibitors.

The compounds of formula (I) or any of its pharmaceutically acceptable salts, or at least any of compounds (1) to (58) as defined above or any of their pharmaceutically acceptable salts, , or at least any of compounds (1) to (58) as defined above or any of their pharmaceutically acceptable salts, may in particular be in the prevention and/or the treatment of hearing loss being a partial or a complete hearing loss, in particular being induced by noise, acoustic trauma or ototoxic agents or conditions, more particularly platin-based anticancer drugs such as cisplatin or carboplatin, antibiotics, even more particularly macrolides or aminoglycosides such as gentamycin or neomycin, drugs for COVID-19 such as lopinavir or ritonavir, antimalarial drugs such as quinine or chloroquine, cardiovascular drugs such as loop diuretics, non-steroidal anti-inflammatory drugs such as salicylate or cyclodextrins, erectile dysfunction drugs such as phosphodiesterase type 5 inhibitors.

The compounds of formula (I) or any of its pharmaceutically acceptable salts, or at least any of compounds (1) to (58) as defined above or any of their pharmaceutically acceptable salts, may in particular be useful in the treatment and/or in the prevention of kidney diseases, in particular selected from nephropathy, acute kidney injury (AKI), chronic kidney disease (CKD) more particularly induced by ischemia and/or reperfusion injury, toxic nephropathy or myoglobinuria, and diabetic kidney disease, or kidney diseases induced by nephrotoxicity caused by therapeutic drugs.

The compounds of formula (I) or any of its pharmaceutically acceptable salts, or at least any of compounds (1) to (58) as defined above or any of their pharmaceutically acceptable salts, may in particular be useful in the treatment and/or in the prevention of kidney diseases induced by nephrotoxicity caused by therapeutic drugs as described hereinafter.

As therapeutic drugs causing nephrotoxicity, the following may be cited: acyclovir, ambisome, amikacin, aminoglycosides, antibiotics, amphotericin B, captopril, carboplatin, cefotaxime, ceftazidime, cefuroxime, cephalosporins, cidofovir, ciprofloxacin, cisplatin, colistimethate, cyclosporine, dapsone, enalaprilat, enalapril, Foscarnet, gadopentetate dimeglumine, gadoxetate, ganciclovir gentamicin, ibuprofen, ifosfamide, iodixanol, iohexol, iopamidol, ioversol, ketorolac, lisinopril, lithium, mesalamine, methotrexate, nafcillin disodium, penicillins, piperacillin/tazobactam, piperacillin, rifampin, sirolimus, sulfasalazine, tacrolimus, ticarcillin/clavulanic acid, tobramycin, topiramate, valacyclovir, valganciclovir, vancomycin or zonisamide.

In one embodiment, the compounds of formula (I) or any of its pharmaceutically acceptable salts, or at least any of compounds (1) to (58) as defined above or any of their pharmaceutically acceptable salts, may in particular be useful in the prevention and/or the treatment of kidney disease being selected from kidney fibrosis, nephropathy, acute kidney injury, chronic kidney disease in particular induced by ischemia and/or reperfusion injury, toxic nephropathy or myoglobinuria, and diabetic kidney disease, or the kidney disease is induced by nephrotoxicity caused by therapeutic drugs, more particularly cytotoxic agents, such as platin-based anticancer drugs for example cisplatin or carboplatin.

The following examples illustrate in detail the preparation of some compounds according to the invention. The structures of the products obtained have been confirmed by NMR analyses and mass spectroscopy. The following examples further illustrate some biological activities of some compounds according to the invention.

### Example 1: Synthesis of 5,7-dichloropyrazolo[1,5-a]pyrimidine derivatives, as illustrated in step 1 of scheme 1

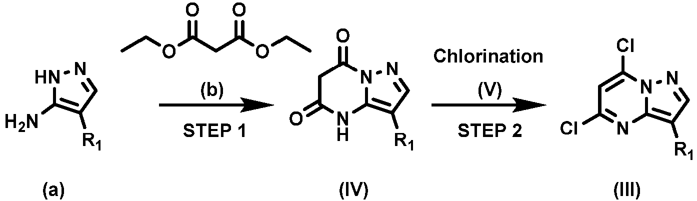

The intermediate product (a) may be obtained commercially or according to methods known to the man skilled in the art.

### Example 1.1: Synthesis of 3-isopropylpyrazolo[1,5-a]pyrimidine-5,7(4H,6H)-dione (1.1)

A microwave vial 10 - 20 mL with a stir bar was charged with 4-isopropyl-1*H-*pyrazol-5-amine (1.000 g, 7.99 mmol, 1.0 eq.), EtOH (12.0 mL), diethyl malonate (1.28 mL, 8.43 mmol, 1.06 eq.) and sodium ethoxide 21 *%* in EtOH (3.28 mL, 8.79 mmol, 1.1 eq.). The vial was closed and put in a microwave reactor: 130 °C, 2 h. After cooling, the solvent was removed and the residue was solubilized in a minimum amout of H₂O. The pH of the solution was adjusted to 4-5 with HCl 1 M to give a precipitate. This precipitate was filtered off, washed with cold water then the solid was taken up in MeOH and dried under vacuum to give intermediate (**1.1**) (1.025 g, 66 *%*) as a beige solid. The product was used without other purification in the next step.
**¹H NMR (400 MHz, DMSO-*d₆*) δ :** 1.11 - 1.19 (m, 6H, 2xCH_{3 iPr}), 2.99 (d, *J* = 27.4 Hz, 1H, H_{iPr}), 3.75 (s, 2H, CH₂), 7.78 (s, 1H, H_{Ar}), 11.37 (s, 1H, NH), 11.97 (s, 1H, NH).

### Example 1.2: Synthesis of 3-cyclopropylpyrazolo[1,5-a]pyrimidine-5,7(4H,6H)-dione (1.2)

To a solution of 4-cyclopropyl-1*H*-pyrazol-3-amine (0.2 g, 1.62 mmol, 1.0 eq.) in EtOH (2 mL) was added successively sodium ethoxide 21*%* in EtOH (0.67 mL, 1.79 mmol, 1.1 eq.) and diethyl malonate (0.26 mL, 1.71 mmol, 1.06 eq.). The mixture was heated at 120 °C in a microwave reactor during 2 h, concentrated and diluted with H₂O. The pH of the solution was adjusted to 3 with HCl (2 M) to give a precipitate. This precipitate was filtered off, washed with cold water (3×1.0 mL) to give intermediate **(1.2)** (0.211 g, 68 *%*) as a brown solid.
**¹H NMR (400 MHz, DMSO-*d₆*) δ :** 0.42 - 0.61 (m, 2H, 2xH_{C(CH2 cPr)}), 0.74 - 0.89 (m, 2H, 2xH_{C(CH2 cPr)}), 1.68 - 1.90 (m, 1H, H_{cPr}), 3.75 (s, 2H, CH_{2-CO}), 7.50 (s, 1H, H_{Ar}), 11.54 (s, 1H, NH), 12.17 (s, 1H, NH).
**¹³C NMR (101 MHz, DMSO-*d₆*) δ :** 3.3 (CH _{cPr}), 7.1 (2 X CH_{2 cPr}), 40.9 (CH_{2 Ar}), 106.9 (C_{q}), 139.5 (C_{q}), 143.9 (CH_{Ar}), 160.6 (C_{q}), 165.4 (C_{q}).
**MS (ESI+) : m/z calcd for C₉H₉N₃O :** 192.1 [M+H]+, **found :** 192.0

### Example 1.3: Synthesis of 5,7-dichloro-3-isopropylpyrazolo[1,5-a]pyrimidine (1.3)

A solution of intermediate (**1.1**) (4.000 g, 20.70 mmol, 1.0 eq.) in POCl₃ (38.98 mL, 414.06 mmol, 20.0 eq.) and *N*,*N*-dimethylaniline (5.57 mL, 43.48 mmol, 2.1 eq.) was refluxed during 5.5 h. After completion, the mixture was concentrated then the residue was diluted in DCM (80.0 mL) and washed with cold water (80.0 mL). Aqueous layer was extracted with DCM (2 X 40.0 mL) then organic layers were combined, dried over MgSO₄ and concentrated. The residue was purified with flash chromatography with cyclohexane/DCM (80/20 to 10/90) as eluent to give intermediate (**1.3**) (4.393 g, 92 *%*) as a yellow crystalline solid.
**Rf** (DCM/Cyclohexane, 60/40) : 0.46.
**¹H NMR (400 MHz, DMSO-*d₆*) δ :** 1.31 (d, *J =* 6.9 Hz, 6H, 2 X CH_{3 iPr}), 3.18 (hept, *J =* 7.0 Hz, 1H, CH _{iPr}), 7.57 (s, 1H, CH_{Ar}), 8.30 (s, 1H, CH_{Ar}).
**¹³C NMR (101 MHz, DMSO-*d₆*) δ :** 23.0 (CH_{3 iPr}), 23.3 (CH _{iPr}), 108.3 (CH_{Ar}), 118.2 (C_{q}), 139.4 (C_{q}), 143.8 (CH_{Ar}), 144.0 (C_{q}), 147.0 (C_{q}).
**MS (ESI+) : m/z calcd for C₉H₇Cl₂N₃ :** 230.0 [M+H]+, **found :** 230.0.

### Example 1.4: Synthesis of 5,7-dichloro-3-cyclopropylpyrazolo[1,5-a]pyrimidine (1.4)

A solution of intermediate (**1.2**) (3.000 g, 15.69 mmol, 1.0 eq.) in POCl₃ (22.16 mL, 235.36 mmool, 15.0 eq.) and *N*,*N*-dimethylaniline (4.22 mL, 32.95 mmol, 2.1 eq.) was refluxed during 5.5 h. After cooling, POCl₃ was removed under vacuum and the residue was taken up in DCM (80.0 mL) then organic layer was washed with cold water (80.0 mL). Layers were separated and aqueous layer was extracted twice with DCM (2x40.0 mL) then organic layers were combined, dried over MgSO₄, filtered and concentrated. The residue was purified by flash chromatography with coyclohexane/DCM (80/20 to 10/90) as eluent to give intermediate (**1.4**) (3.125 g, 87 *%*) as a yellow solid.
**Rf** (Cylohexane/EtOAc, 90/10) : 0.36.
**¹H NMR (400 MHz, DMSO-*d₆*) δ :** 0.78 - 0.84 (m, 2H, CH_{2 cPr}), 0.92 - 0.98 (m, 2H, CH_{2 cPr}), 1.98 (tt, *J =* 8.4, 5.1 Hz, 1H, CH _{cPr}), 7.54 (s, 1H, CH_{Ar}), 8.16 (s, 1H, CH_{Ar}).
**¹³C NMR (101 MHz, DMSO-*d₆*) δ :** 4.7 (CH _{cPr}), 7.3 (2 X CH_{2 cPr}), 108.3 (CH_{Ar}), 114.0 (C_{q}), 139.4 (C_{q}), 143.7 (CH_{Ar}), 144.8 (C_{q}), 147.1 (C_{q}).
**MS (ESI+) : m/z calcd for C₉H₇Cl₂N₃ :** 228.0 [M+H]+, **found :** 228.1.

### Example 2: Synthesis of 6-chloro-pyrazolo[1,5-a]pyrimidin-7-amine derivatives, as illustrated in step 3 of scheme 1

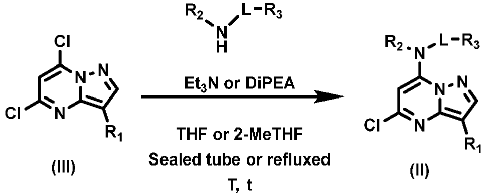

### Example 2.1: Synthesis of N-benzyl-5-chloro-3-isopropylpyrazolo[1,5-a]pyrimidin-7-amine (2.1)

To a solution of intermediate (**1.3**) (0.150 g, 0.65 mmol, 1.0 eq.) in 2-MeTHF (13.0 mL), benzylamine (0.08 mL, 0.72 mmol, 1.1 eq.) and Et₃N (0.10 mL, 0.72 mmol, 1.1 eq.) were added then the mixture was refluxed 1 h. After cooling, HCl 0.1M (10.0 mL) was added then vigorously stirred 10 min. Layers were separated and aqueous layer was extracted twice with 2-MeTHF (2x5.0 mL) then organic layers were combined, dried over MgSO₄, filtered and concentrated. The residue was purified by flash chromatography with DCM/Cyclohexane (90/10) as eluent to give (**2.1**) (0.187 g, 97 *%*) as a white solid.
**Rf** (DCM, 100*%*) : 0.68.
**¹H NMR (400 MHz, DMSO-*d₆*) δ :** 1.27 (d, *J =* 6.9 Hz, 6H, 2xCH_{3 iPr}), 3.08 (hept, *J* = 6.8 Hz, 1H, H_{iPr}), 4.62 (d, *J =* 6.6 Hz, 2H, CH_{2 Bn}), 6.07 (s, 1H, H_{Ar}), 7.22 - 7.29 (m, 1H, H_{Ar}), 7.30 - 7.45 (m, 4H, 4xH_{Ar}), 8.05 (s, 1H, H_{Ar}), 8.92 (t, *J=* 6.6 Hz, 1H, NH_{Bn}).
**¹³C NMR (101 MHz, DMSO-*d₆*) δ :** 23.1 (CH_{iPr}), 23.2 (2xCH_{3 iPr}), 44.4 (CH_{2 Bn}), 83.9 (CH_{Ar}), 115.2 (C_{q}), 127.0 (2xCH_{Ar}), 127.2 (CH_{Ar}), 128.5 (2xCH_{Ar}), 137.8 (C_{q}), 141.6 (C_{q}), 143.8 (C_{q}), 147.3 (C_{q}), 149.0 (C_{q}).
**MS (ESI+) : m/z calcd for C₁₆H₁₇ClN₄ :** 301.12 [M+H]+, **found :** 301.15.

### Example 2.2: Synthesis of 5-chloro-3-isopropyl-N-(pyridin-4-ylmethyl)pyrazolo[1,5-a]pyrimidin-7-amine (2.2)

To a solution of intermediate (**1.3**) (0.300 g, 1.30 mmol, 1.0 eq.) in 2MeTHF (4.3 mL), 2-pyridylmethanamine (0.183 g, 1.70 mmol, 1.3 eq.) and Et₃N (0.29 mL, 2.09 mmol, 1.6 eq.) were added then the mixture was refluxed 1.5 h. After cooling, THF (10.0 mL) was added then the solid was triturated, filtered and washed with THF. The filtrate was concentrated and the residue was purified by flash chromatography with DCM/MeOH (99/1 to 95/5) as eluent to give (**2.2**) (0.377 g, 96 *%*) as a beige crystalline solid.
**Rf** (DCM/MeOH, 94/6) : 0.50.
**1H NMR (400 MHz, DMSO-*d₆*) δ :** 1.28 (d, *J =* 6.9 Hz, 6H, 2xCH_{3 iPr}), 3.10 (p, *J =* 6.9 Hz, 1H, H_{iPr}), 4.67 (d, *J* = 3.5 Hz, 2H, CH_{2 Bn}), 6.08 (s, 1H, H_{Ar}), 7.32 - 7.38 (m, 2H, 2xH_{Ar}), 8.07 (s, 1H, H_{Ar}), 8.48 - 8.54 (m, 2H, 2xH_{Ar}), 8.96 (s, 1H, NH_{Bn}).
**13C NMR (101 MHz, DMSO-*d₆*) δ :** 23.1 (CH_{iPr}), 23.2 (2xCH_{3 iPr}), 43.4 (CH_{2 Bn}), 84.0 (CH_{Ar}), 115.3 (C_{q}), 121.9 (2xCH_{Ar}), 141.7 (CH_{Ar}), 143.8 (C_{q}), 146.8 (C_{q}), 147.4 (C_{q}), 149.1 (C_{q}), 149.7 (2xCH_{Ar}).
**MS (ESI+) : m/z calcd for C₁₅H₁₆ClN₅ :** 302.1 [M+H]+, **found :** 302.1.

### Example 2.3: Synthesis of 5-chloro-3-isopropyl-N-(pyridin-3-ylmethyl)pyrazolo[1,5-a]pyrimidin-7-amine (2.3)

To a solution of intermediate (**1.3**) (0.300 g, 1.30 mmol, 1.0 eq.) in 2MeTHF (4.3 mL), 2-pyridylmethanamine (0.183 g, 1.70 mmol, 1.3 eq.) and Et₃N (0.29 mL, 2.09 mmol, 1.6 eq.) were added then the mixture was refluxed 1.5 h. After cooling, THF (10.0 mL) was added then the solid was triturated, filtered and washed with THF. The filtrate was concentrated and the residue was purified by flash chromatography with DCM/MeOH (99/1 to 96/4) as eluent to give intermediate (**2.3**) (0.383 g, 97 *%*) as a white crystalline solid.
**Rf** (DCM/MeOH, 94/6) : 0.54.
**¹H NMR (400 MHz, DMSO-*d₆*) δ :** 1.27 (d, *J* = 6.9 Hz, 6H, 2xCH_{3 iPr}), 3.08 (hept, *J* = 6.9 Hz, 1H, H_{iPr}), 4.67 (d, *J =* 6.6 Hz, 2H, CH_{2 Bn}), 6.20 (s, 1H, H_{Ar}), 7.36 (dd, *J =* 7.9, 4.8 Hz, 1H, H_{Ar}), 7.80 (dt, *J =* 7.8, 2.0 Hz, 1H, H_{Ar}), 8.05 (s, 1H, H_{Ar}), 8.47 (dd, *J =* 4.7, 1.6 Hz, 1H, H_{Ar}), 8.65 (d, *J =* 2.3 Hz, 1H, H_{Ar}), 8.95 (t, *J* = 6.7 Hz, 1H, NH_{Bn}).
**¹³C NMR (101 MHz, DMSO-*d₆*) δ :** 23.1 (CH_{iPr}), 23.2 (2xCH_{3 iPr}), 42.1 (CH_{2 Bn}), 83.9 (CH_{Ar}), 115.2 (C_{q}), 123.6 (CH_{Ar}), 133.4 (C_{q}), 134.9 (CH_{Ar}), 141.6 (CH_{Ar}), 143.8 (C_{q}), 147.2 (C_{q}), 148.5 (CH_{Ar}), 148.7 (CH_{Ar}), 149.1 (C_{q}).
**MS (ESI+) : m/z calcd for C₁₅H₁₆ClN₅ :** 302.1 [M+H]+, **found :** 302.2.

### Example 2.4: Synthesis of 5-chloro-3-isopropyl-N-(pyridin-2-ylmethyl)pyrazolo[1,5-a]pyrimidin-7-amine (2.4)

To a solution of intermediate (**1.3**) (0.300 g, 1.30 mmol, 1.0 eq.) in 2-MeTHF (4.3 mL), 2-pyridylmethanamine (0.183 g, 1.70 mmol, 1.3 eq.) and Et₃N (0.29 mL, 2.09 mmol, 1.6 eq.) were added then the mixture was refluxed 1.5 h. After cooling, THF (10.0 mL) was added then the solid was triturated, filtered and washed with THF. The filtrate was concentrated and the residue was purified by flash chromatography with DCM/MeOH (99/1 to 96/4) as eluent to give intermediate (**2.4**) (0.375 g, 95 *%*) as a white crystalline solid.
**Rf** (DCM/MeOH, 94/6) : 0.80.
**¹H NMR (400 MHz, DMSO-*d₆*) δ :** 1.28 (d, *J =* 6.9 Hz, 6H, 2xCH_{3 iPr}), 3.10 (hept, *J* = 6.9 Hz, 1H, H_{iPr}), 4.72 (d, *J=* 6.3 Hz, 2H, CH_{2 Bn}), 6.09 (s, 1H, H_{Ar}), 7.31 (ddd, *J =* 7.6, 4.8, 1.1 Hz, 1H, H_{Ar}), 7.38 (d, *J=* 7.8 Hz, 1H, H_{Ar}), 7.79 (td, *J =* 7.7, 1.8 Hz, 1H, H_{Ar}), 8.05 (s, 1H, H_{Ar}), 8.55 (dt, *J =* 4.9, 1.3 Hz, 1H, H_{Ar}), 8.77 (t, *J=* 6.3 Hz, 1H, NH_{Bn}).
**¹³C NMR (101 MHz, DMSO-*d₆*) δ :** 23.1 (CH_{iPr}), 23.2 (2xCH_{3 iPr}), 46.3 (CH_{2 Bn}), 84.2 (CH_{Ar}), 115.2 (C_{q}), 121.4 (CH_{Ar}), 122.6 (CH_{Ar}), 137.1 (CH_{Ar}), 141.7 (CH_{Ar}), 143.7 (C_{q}), 147.5 (C_{q}), 149.0 (CH_{Ar}), 156.5 (C_{q}).
**MS (ESI+) : m/z calcd for C₁₅H₁₆ClN₅ :** 302.1 [M+H]+, **found :** 302.2.

### Example 2.5: Synthesis of 5-chloro-3-isopropyl-N-(2-(pyridin-2-yl)ethyl)pyrazolo[1,5-a]pyrimidin-7-amine (2.5)

A solution of intermediate (**1.3**) (0.330 g, 1.43 mmol, 1.0 eq.), corresponding amine (0.221 g, 1.72 mmol, 1.2 eq.), Et₃N (0.24 mL, 1.72 mmol, 1.2 eq.) in 2-MeTHF (4.3 mL) was refluxed 1 h. After cooling, THF (10.0 mL) was added and the solid was triturated, filtered off, washed with THF. The filtrate was concentrated and the residue was purified by flash chromatography with DCM/MeOH (98/2) as eluent to give intermediate (**2.5**) (0.430 g, 95 *%*) as a white crystalline solid.
**Rf** (DCM/MeOH, 96/4) : 0.54.
**¹H NMR (400 MHz, DMSO-*d₆*) δ :** 1.26 (d, *J* = 7.0 Hz, 6H, CH_{3 iPr}), 3.02 - 3.14 (m, 3H, H_{iPr} & CH_{2-CH2-NH}), 3.76 (q, *J=* 6.7 Hz, 2H, CH_{2-NH}), 6.11 (s, 1H, CH_{Ar}), 7.23 (ddd, *J* = 7.5, 4.9, 1.2 Hz, 1H, CH_{Ar}), 7.33 (dt, *J =* 7.7, 1.1 Hz, 1H, CH_{Ar}), 7.70 (td, *J =* 7.7, 1.9 Hz, 1H, CH_{Ar}), 7.99 (s, 1H, CH_{Ar}), 8.32 (t, *J=* 6.0 Hz, 1H, NH), 8.52 (ddd, *J =* 4.9, 1.9, 0.9 Hz, 1H, CH_{Ar}).
**¹³C NMR (101 MHz, DMSO-*d₆*) δ :** 23.1 (CH_{iPr}), 23.2 (2xCH_{3 iPr}), 36.3 (CH_{2-CH2-NH}), 41.1 (CH_{2-NH}), 83.5 (CH_{Ar}), 115.0 (C_{q}), 121.7 (CH_{Ar}), 123.5 (CH_{Ar}), 136.6 (CH_{Ar}), 141.4 (CH_{Ar}), 143.7 (C_{q}), 147.2 (C_{q}), 149.1 (CH_{Ar}), 149.2 (C_{q}), 158.5 (C_{q}).
**MS (ESI+) : m/z calcd for C₁₅H₁₆ClN₅ :** 316.1 [M+H]+, **found :** 316.2.

### Example 2.6: Synthesis of 5-chloro-3-isopropyl-N-(2-(pyridin-3-yl)ethyl)pyrazolo[1,5-a]pyrimidin-7-amine (2.6)

A solution of intermediate (**1.3**) (0.330 g, 1.43 mmol, 1.0 eq.), corresponding amine (0.221 g, 1.72 mmol, 1.2 eq.), Et₃N (0.24 mL, 1.72 mmol, 1.2 eq.) in 2-MeTHF (4.3 mL) was refluxed 1 h. After cooling, THF (10.0 mL) was added and the solid was triturated, filtered off, washed with THF. The filtrate was concentrated and the residue was purified by flash chromatography with DCM/MeOH (97/3) as eluent to give intermediate (**2.6**) (0.430 g, 95 *%*) as a light brown solid.
**Rf** (DCM/MeOH, 96/4) : 0.32.
**¹H NMR (400 MHz, DMSO-*d₆*) δ :** 1.26 (d, *J =* 6.9 Hz, 6H, 2xCH_{3 iPr}), 2.96 (t, *J =* 7.1 Hz, 2H, CH_{2-CH2, NH}), 3.07 (hept, *J =* 6.9 Hz, 1H, H_{iPr}), 3.66 (q, *J=* 6.7 Hz, 2H, CH_{2-NH}), 6.20 (s, 1H, H_{Ar}), 7.30 (dd, *J =* 7.8, 4.7 Hz, 1H, H_{Ar}), 7.71 (dt, *J =* 7.8, 2.0 Hz, 1H, H_{Ar}), 7.99 (s, 1H, H_{Ar}), 8.29 (d, *J =* 5.9 Hz, 1H, NH), 8.41 (dd, *J* = 4.8, 1.7 Hz, 1H, H_{Ar}), 8.48 (d, *J =* 2.3 Hz, 1H, H_{Ar}).
**¹³C NMR (101 MHz, DMSO-*d₆*) δ :** 23.1 (CH_{iPr}), 23.2 (2xCH_{3 iPr}), 31.5 (CH_{2-CH2-NH}), 42.2 (CH_{2-NH}), 83.6 (CH_{Ar}), 115.0 (C_{q}), 123.3 (CH_{Ar}), 134.3 (C_{q}), 136.4 (CH_{Ar}), 141.4 (CH_{Ar}), 143.7 (C_{q}), 147.2 (C_{q}), 147.5 (CH_{Ar}), 149.3 (C_{q}), 150.0 (CH_{Ar}).
**MS (ESI+) : m/z calcd for C₁₅H₁₆ClN₅ :** 316.1 [M+H]+, **found :** 316.2.

### Example 2.7: Synthesis of 5-chloro-3-isopropyl-N-(2-(pyridin-4-yl)ethyl)pyrazolo[1,5-a]pyrimidin-7-amine (2.7)

A solution of intermediate (**1.3**) (0.330 g, 1.43 mmol, 1.0 eq.), corresponding amine (0.221 g, 1.72 mmol, 1.2 eq.), Et₃N (0.24 mL, 1.72 mmol, 1.2 eq.) in 2-MeTHF (4.3 mL) was refluxed 1 h. After cooling, THF (10.0 mL) was added and the solid was triturated, filtered off, washed with THF. The filtrate was concentrated and the residue was purified by flash chromatography with DCM/MeOH (97/3 to 96/4) as eluent to give intermediate (**2.7**) (0.420 g, 93 *%*) as a colourless oil.
**Rf** (DCM/MeOH, 96/4) : 0.28.
**¹H NMR (400 MHz, DMSO-*d₆*) δ :** 1.27 (d, *J =* 6.9 Hz, 6H, 2xCH_{3 iPr}), 2.97 (t, *J =* 7.1 Hz, 2H, CH_{2-CH2-NH}), 3.08 (hept, *J =* 7.0 Hz, 1H, H_{iPr}), 3.68 (q, *J =* 6.8 Hz, 2H, CH_{2-NH}), 6.24 (s, 1H, H_{Ar}), 7.27 - 7.37 (m, 2H, 2xH_{Ar}), 7.99 (s, 1H, H_{Ar}), 8.29 (t, *J=* 6.0 Hz, 1H, NH), 8.41 - 8.50 (m, 2H, 2xH_{Ar}).
**¹³C NMR (101 MHz, DMSO-*d₆*) δ :** 23.1 (CH_{iPr}), 23.2 (2xCH_{3 iPr}), 33.5 (CH_{2-CH2-NH}), 41.5 (CH_{2-NH}), 83.6 (CH_{Ar}), 115.0 (C_{q}), 124.4 (2xCH_{Ar}), 141.4 (CH_{Ar}), 143.7 (C_{q}), 147.2 (C_{q}), 147.7 (C_{q}), 149.3 (C_{q}), 149.4 (2xCH_{Ar}).
**MS (ESI+) : m/z calcd for C₁₅H₁₆ClN₅ :** 316.1 [M+H]+, **found :** 316.2.

### Example 2.8: Synthesis of 5-chloro-3-isopropyl-N-(pyridazin-3-ylmethyl)pyrazolo[1,5-a]pyrimidin-7-amine (2.8)

A solution of intermediate (**1.3**) (0.330 g, 1.30 mmol, 1.0 eq.), pyridazin-3-ylmethanamine dihydrochloride (0.325 g, 1.69 mmol, 1.3 eq.), Et₃N (0.90 mL, 6.39 mmol, 4.9 eq.) in 2-MeTHF (5.0 mL) was refluxed 3 h. Water was added (0.5 mL) and the mixture was refluxed 1.5 h to complete the reaction. After cooling, 2-MeTHF (5.0 mL) and saturated NaHCO₃ were added and vigorously stirred few minutes. Layers were separated and organic layer was extracted twice with 2-MeTHF (2x5.0 mL). Organic layers were combined, dried over MgSO₄, filtered and concentrated. The residue was purified by flash chromatography with DCM/MeOH (99/1 to 96/4) as eluent to give intermediate (**2.8**) (0.350 g, 89 *%*) as a yellow solid.
**Rf** (DCM/MeOH, 96/4) : 0.50.
**¹H NMR (400 MHz, DMSO-*d₆*) δ :** 1.28 (d, *J =* 6.9 Hz, 6H, 2xCH_{3 iPr}), 3.10 (hept, *J* = 7.0 Hz, 1H, H_{iPr}), 4.93 (d, *J=* 6.5 Hz, 2H, CH_{2 Bn}), 6.19 (s, 1H, H_{Ar}), 7.60 - 7.82 (m, 2H, 2xH_{Ar}), 8.06 (s, 1H, H_{Ar}), 8.91 (t, *J=* 6.5 Hz, 1H, NH_{Bn}), 9.17 (dd, *J =* 3.8, 2.8 Hz, 1H, H_{Ar}).
**¹³C NMR (101 MHz, DMSO-*d₆*) δ :** 23.1 (CH_{iPr}), 23.2 (2xCH_{3 iPr}), 45.0 (CH_{2 Bn}), 84.2 (CH_{Ar}), 115.3 (C_{q}), 125.6 (CH_{Ar}), 127.5 (CH_{Ar}), 141.7 (CH_{Ar}), 143.7 (C_{q}), 147.5 (C_{q}), 149.1 (C_{q}), 151.1 (CH_{Ar}), 159.3 (C_{q}).
**MS (ESI+) : m/z calcd for C₁₄H₁₅ClN₆ :** 303.1 [M+H]+, **found :** 303.2.

### Example 2.9: Synthesis of 5-chloro-3-isopropyl-N-(pyridazin-4-ylmethyl)pyrazolo[1,5-a]pyrimidin-7-amine (2.9)

A solution of intermediate (**1.3**) (0.330 g, 1.30 mmol, 1.0 eq.), pyridazin-4-ylmethanamine hydrochloride (0.260 g, 1.69 mmol, 1.3 eq.), Et₃N (0.90 mL, 6.39 mmol, 4.9 eq.) in 2-MeTHF (5.0 mL) was refluxed 3 h. Water was added (0.5 mL) and the mixture was refluxed 1.5 h to complete the reaction. After cooling, 2-MeTHF (5.0 mL) and saturated NaHCOs were added and vigorously stirred few minutes. Layers were separated and organic layer was extracted twice with 2-MeTHF (2x5.0 mL). Organic layers were combined, dried over MgSO₄, filtered and concentrated. The residue was purified by flash chromatography with DCM/MeOH (99/1 to 96/4) as eluent to give intermediate (**2.9**) (0.250 g, 63 *%*) as a light red solid.
**Rf** (DCM/MeOH, 96/4) : 0.33.
**¹H NMR (400 MHz, DMSO-*d₆*) δ :** 1.28 (d, *J =* 6.9 Hz, 6H, 2xCH_{3 iPr}), 3.10 (hept, *J* = 6.9 Hz, 1H, H_{iPr}), 4.73 (d, *J =* 6.6 Hz, 2H, CH_{2 Bn}), 6.22 (s, 1H, H_{Ar}), 7.60 (dd, *J =* 5.4, 2.4 Hz, 1H, H_{Ar}), 8.07 (s, 1H, H_{Ar}), 8.95 (t, *J =* 6.7 Hz, 1H, NH_{Bn}), 9.14 (dd, *J =* 5.3, 1.3 Hz, 1H, H_{Ar}), 9.23 - 9.33 (m, 1H, H_{Ar}).
**¹³C NMR (101 MHz, DMSO-*d₆*) δ :** 23.1 (CH_{iPr}), 23.2 (CH_{3 iPr}), 41.5 (CH_{2 Bn}), 84.0 (CH_{Ar}), 115.3 (C_{q}), 124.5 (CH_{Ar}), 137.7 (C_{q}), 141.8 (CH_{Ar}), 143.8 (C_{q}), 147.3 (C_{q}), 149.2 (C_{q}), 151.0 (CH_{Ar}), 151.4 (CH_{Ar}).
**MS (ESI+) : m/z calcd for C₁₄H₁₅ClN₆ :** 303.1 [M+H]+, **found :** 303.2.

### Example 2.10: Synthesis of 5-chloro-3-isopropyl-N-(pyridin-2-yl)pyrazolo[1,5-a]pyrimidin-7-amine (2.10)

Under inert gas, to a solution of intermediate (**1.3**) (0.150 g, 0.65 mmol, 1.0 eq.) and pyridin-2-amine (0.092 g, 0.98 mmol, 1.5 eq.) in dry THF (4.0 mL), tBuOK 1M in THF (0.98 mL, 0.98 mmol, 1.5 eq.) was added dropwise. The mixture was stirred 3.5 h at room temperature then saturated NH₄Cl (1.0 mL), water (0.5 mL) and EtOAc (3.0 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/EtOAc (98/2) as eluent to give intermediate (**2.10**) (0.140 g, 75 *%*) as a light yellow solid.
**¹H NMR (400 MHz, DMSO-*d₆*) δ :** 1.33 (d, *J =* 6.9 Hz, 6H, 2xCH_{3 iPr}), 3.17 (hept, *J* = 6.9 Hz, 1H, H_{iPr}), 7.16 (ddd, *J* = 7.4, 5.0, 1.0 Hz, 1H, H_{Ar}), 7.68 (d, *J =* 8.3 Hz, 1H, H_{Ar}), 7.84 (ddd, *J* = 9.1, 7.4, 2.0 Hz, 1H, H_{Ar}), 7.98 (s, 1H, H_{Ar}), 8.17 (s, 1H, H_{Ar}), 8.46 (dd, *J =* 5.1, 1.9 Hz, 1H, H_{Ar}), 10.66 (s, 1H, NH).
**¹³C NMR (101 MHz, DMSO-*d₆*) δ :** 23.2 (2xCH_{3 iPr} & CH_{iPr}), 90.6 (CH_{Ar}), 114.9 (CH_{Ar}), 116.1 (C_{q}), 119.0 (CH_{Ar}), 138.4 (CH_{Ar}), 141.4 (CH_{Ar}), 142.7 (C_{q}), 143.8 (C_{q}), 147.4 (CH_{Ar}), 149.1 (C_{q}), 152.5 (C_{q}).
**MS (ESI+) : m/z calcd for C₁₄H₁₄ClN₅ :** 288.1 [M+H]+, **found :** 288.1.

### Example 2.11: Synthesis of 5-chloro-3-isopropyl-N-(pyridin-3-yl)pyrazolo[1,5-a]pyrimidin-7-amine (2.11)

Under inert gas, to a solution of intermediate (**1.3**) (0.150 g, 0.65 mmol, 1.0 eq.) and pyridin-3-amine (0.092 g, 0.98 mmol, 1.5 eq.) in dry THF (4.0 mL), tBuOK 1M in THF (0.98 mL, 0.98 mmol, 1.5 eq.) was added dropwise. The mixture was stirred 3.5 h at room temperature then saturated NH₄Cl (1.0 mL), water (0.5 mL) and EtOAc (3.0 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/EtOAc (98/2) as eluent to give intermediate (**2.11**) (0.140 g, 75 *%*) as a brown solid.
**¹H NMR (400 MHz, DMSO-*d₆*) δ :** 1.32 (d, *J =* 6.9 Hz, 6H, 2xCH_{3 iPr}), 3.15 (hept, *J* = 6.9 Hz, 1H, H_{iPr}), 6.10 (s, 1H, H_{Ar}), 7.52 (ddd, *J =* 8.2, 4.8, 0.8 Hz, 1H, H_{Ar}), 7.92 (ddd, *J =* 8.2, 2.6, 1.5 Hz, 1H, H_{Ar}), 8.17 (s, 1H, H_{Ar}), 8.50 (dd, *J =* 4.7, 1.5 Hz, 1H, H_{Ar}), 8.69 (d, *J =* 2.5 Hz, 1H, H_{Ar}), 10.40 (s, 1H, NH).
**¹³C NMR (101 MHz, DMSO-*d₆*) δ :** 23.2 (CH_{iPr}), 23.2 (2xCH_{3 iPr}), 85.3 (CH_{Ar}), 115.8 (C_{q}), 124.2 (CH_{Ar}), 132.1 (CH_{Ar}), 133.7 (C_{q}), 142.0 (CH_{Ar}), 144.1 (C_{q}), 146.0 (C_{q}), 146.2 (CH_{Ar}), 147.0 (CH_{Ar}), 149.1 (C_{q}).
**MS (ESI+) : m/z calcd for C₁₄H₁₄ClN₅ :** 288.1 [M+H]+, **found :** 288.1.

### Example 2.12: Synthesis of 5-chloro-3-isopropyl-N-(pyridazin-3-yl)pyrazolo[1,5-a]pyrimidin-7-amine (2.12)

Under inert gas, to a solution of intermediate (**1.3**) (0.080 g, 0.35 mmol, 1.0 eq.) and pyridazin-3-amine (0.040 g, 0.42 mmol, 1.2 eq.) in dry THF (10.0 mL), NaH 60*%* suspended in mineral oil (0.017 g, 0.42 mmol, 1.2 eq.) was added. The mixture was stirred 3 h at room temperature then saturated NH₄Cl (0.5 mL) was added then the mixture was vigorously stirred 5 min. The mixture was concentrated then the residu was taken up in DCM, the solid was filtered and the filtrate was purified by flash chromatography with DCM/MeOH (99/1 to 98/2) as eluent to give intermediate (**2.12**) (0.065 g, 65 *%*) as a white solid.
**Rf** (DCM/MeOH, 98/2) : 0.26.
**¹H NMR (400 MHz, DMSO-*d₆*) δ :** 1.34 (d, *J =* 6.9 Hz, 6H, 2xCH_{3 iPr}), 3.19 (hept, *J* = 6.9 Hz, 1H, H_{iPr}), 7.75 (dd, *J* = 9.0, 4.6 Hz, 1H, H_{Ar}), 7.99 (dd, *J* = 9.0, 1.4 Hz, 1H, H_{Ar}), 8.06 (s, 1H, H_{Ar}), 8.22 (s, 1H, H_{Ar}), 9.03 (dd, *J =* 4.6, 1.4 Hz, 1H, H_{Ar}), 10.94 (s, 1H, NH).
**¹³C NMR (101 MHz, DMSO-*d₆*) δ :** 23.2 (2xCH_{3 iPr} & CH_{iPr}), 91.9 (CH_{Ar}), 116.4 (C_{q}), 119.9 (CH_{Ar}), 128.8 (CH_{Ar}), 141.7 (CH_{Ar}), 142.2 (C_{q}), 143.8 (C_{q}), 148.2 (C_{q}), 149.2 (C_{q}), 156.0 (C_{q}).
**MS (ESI+) : m/z calcd for C₁₅H₁₆ClN₅ :** 289.1 [M+H]+, **found :** 289.1.

### Example 2.13: Synthesis of 5-chloro-3-isopropyl-N-(pyrimidin-4-yl)pyrazolo[1,5-a]pyrimidin-7-amine (2.13)

To a solution of 4-aminopyrimidine (0.054 g, 0.56 mmol, 1.3 eq.) in dry THF (6.0 mL), NaH 60*%* suspended in mineral oil (0.023 g, 0.56 mmol, 1.3 eq.) was added. The mixture was stirred 10 min at room temperature then a solution of intermediate (**1.3**) (0.100 g, 0.43 mmol, 1.0 eq.) in dry THF (4.0 mL) was added and the mixture was stirred 2 h at room temperature. After completion, saturated NH₄Cl (1.0 mL) and EtOAc (5.0 mL) were added and the mixture was vigorously stirred 5 min. Layers were separated then organic layer was dried over MgSO₄, filtrered, concentrated and the residue was purified by flash chromatography with DCM/MeOH (99/1) as eluent to give intermediate (**2.13**) (0.072 g, 57 *%*) as a light yellow solid.
**Rf** (DCM/MeOH, 96/4) : 0.66.
**¹H NMR (400 MHz, DMSO-*d₆*) δ :** 1.34 (d, *J =* 6.9 Hz, 6H, 2xCH_{3 iPr}), 3.19 (hept, *J* = 6.9 Hz, 1H, H_{iPr}), 7.73 (dd, *J* = 5.8, 1.3 Hz, 1H, H_{Ar}), 8.09 (s, 1H, H_{Ar}), 8.22 (s, 1H, H_{Ar}), 8.66 (d, *J =* 5.8 Hz, 1H, H_{Ar}), 9.03 (d, *J=* 1.2 Hz, 1H, H_{Ar}), 11.11 (s, 1H, NH).
**¹³C NMR (101 MHz, DMSO-*d₆*) δ :** 23.1 (2xCH_{3 iPr}), 23.2 (CH_{iPr}), 93.1 (CH_{Ar}), 111.1 (CH_{Ar}), 116.5 (C_{q}), 141.8 (CH_{Ar}), 141.8 (C_{q}), 143.7 (C_{q}), 148.9 (C_{q}), 157.3 (CH_{Ar}), 157.7 (CH_{Ar}), 158.6 (C_{q}).
**MS (ESI+) : m/z calcd for C₁₅H₁₆ClN₅ :** 289.1 [M+H]+, **found :** 289.2.

### Example 2.14: Synthesis of 5-chloro-3-isopropyl-N-(pyrazin-2-yl)pyrazolo[1,5-a]pyrimidin-7-amine (2.14)

Under inert gas, to a solution of pyrazin-2-amine (0.094 g, 0.98 mmol, 1.5 eq.) in dry THF (6.0 mL), tBuOK 1M in THF (0.98 mL, 0.98 mmol, 1.5 eq.) was added. The mixture was stirred 5 min at room temperature then intermediate (**1.3**) (0.150 g, 0.65 mmol, 1.0 eq.) was added and stirred 1.5 h at room temperature. After completion, saturated NH₄Cl (1.0 mL), water (0.5 mL) and EtOAc (3.0 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/MeOH (100/0 to 98/2) as eluent to give intermediate (**2.14**) (0.125 g, 66 *%*) as a white solid.
**¹H NMR (400 MHz, DMSO-*d₆*) δ :** 1.33 (d, *J =* 6.9 Hz, 6H, 2xCH_{3 iPr}), 3.17 (hept, *J* = 7.0 Hz, 1H, H_{iPr}), 7.86 (s, 1H, H_{Ar}), 8.21 (s, 1H, H_{Ar}), 8.35 (d, *J=* 2.7 Hz, 1H, H_{Ar}), 8.46 - 8.52 (m, 1H, H_{Ar}), 8.99 (d, *J=* 1.4 Hz, 1H, H_{Ar}), 11.09 (s, 1H, NH).
**¹³C NMR (101 MHz, DMSO-*d₆*) δ :** 23.2 (2xCH_{3 iPr} & CH_{iPr}), 91.1 (CH_{Ar}), 116.3 (CH_{Ar}), 138.0 (CH_{Ar}), 138.5 (CH_{Ar}), 141.2 (CH_{Ar}), 141.7 (CH_{Ar}), 142.4 (C_{q}), 143.8 (C_{q}), 149.1 (C_{q}), 149.3 (C_{q}).
**MS (ESI+) : m/z calcd for C₁₃H₁₃ClN₆ :** 289.1 [M+H]+, **found :** 289.1.

### Example 2.15: Synthesis of 5-chloro-3-isopropyl-N-(pyrimidin-5-yl)pyrazolo[1,5-a]pyrimidin-7-amine (2.15)

Under inert gas, to a solution of pyrimidin-5-amine (0.093 g, 0.98 mmol, 1.5 eq.) in dry THF (6.0 mL), tBuOK 1M in THF (0.98 mL, 0.98 mmol, 1.5 eq.) was added. The mixture was stirred 5 min at room temperature then intermediate (**1.3**) (0.150 g, 0.65 mmol, 1.0 eq.) was added and stirred 1.5 h at room temperature. After completion, saturated NH₄Cl (1.0 mL), water (0.5 mL) and EtOAc (3.0 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/MeOH (100/0 to 97/3) as eluent to give intermediate (**2.14**) (0.143 g, 76 *%*) as a white solid.
**¹H NMR (400 MHz, DMSO-*d₆*) δ :** 1.32 (d, *J =* 6.9 Hz, 6H, 2xCH_{3 iPr}), 3.16 (hept, *J* = 6.9 Hz, 1H, H_{iPr}), 6.33 (s, 1H, H_{Ar}), 8.19 (s, 1H, H_{Ar}), 8.96 (s, 2H, 2xH_{Ar}), 9.10 (s, 1H, H_{Ar}), 10.46 (s, 1H, NH).
**¹³C NMR (101 MHz, DMSO-*d₆*) δ :** 23.2 (2xCH_{3 iPr} & CH_{iPr}), 86.1 (CH_{Ar}), 115.9 (C_{q}), 132.9 (C_{q}), 142.1 (CH_{Ar}), 144.0 (C_{q}), 145.6 (C_{q}), 149.2 (C_{q}), 152.9 (2xCH_{Ar}), 155.3 (CH_{Ar}). **MS (ESI+) : m/z calcd for C₁₃H₁₃ClN₆ :** 289.1 [M+H]+, **found :** 289.1.

### Example 2.16: Synthesis of 5-chloro-3-isopropyl-N-(6-methoxypyridin-2-yl)pyrazolo[1,5-a]pyrimidin-7-amine (2.16)

To a solution of intermediate (**1.3**) (0.125 g, 0.54 mmol, 1.0 eq.) and 6-methoxypyridin-2-amine (0.103 g, 0.82 mmol, 1.5 eq.) in dry THF (6.0 mL) under argon was added t-BuOK 1M THF (0.81 mL, 0.82 mmol, 1.5 eq.) dropwise at 0 °C then the mixture was stirred at room temperature during 3 h. After completion, saturated NH₄Cl (1.0 mL), H₂O (0.5 mL) and EtOAc (3.0 mL) was added and the biphasic mixture was vigorously stirred 5min. Layers were separated and aqueous layer was extracted with EtOAc (2 x 3.0 mL). The combined organic phases were dried over MgSO₄ and concentrated. The residue was purified by flash chromatography with DCM/EtOAc (100/0 to 99/1) as eluent to give intermediate **(2.16)** (0.152 g, 88 *%*) as a light yellow solid.
**Rf** (DCM, 100*%*) : 0.58.
**¹H NMR (400 MHz, DMSO-*d*₆) δ :** 1.32 (d, *J =* 7.0 Hz, 6H, 2xCH_{3 iPr}), 3.17 (hept, *J* = 6.9 Hz, 1H, H_{iPr}), 3.95 (s, 3H, CH_{3-O}), 6.57 (d, *J* = 8.0 Hz, 1H, H_{Ar}), 7.25 (d, *J* = 7.8 Hz, 1H, H_{Ar}), 7.74 (t, *J=* 7.9 Hz, 1H, H_{Ar}), 7.94 (s, 1H, H_{Ar}), 8.16 (s, 1H, H_{Ar}), 10.65 (s, 1H, NH).
**¹³C NMR (101 MHz, DMSO-*d₆*) δ :** 23.2 (2xCH_{3 iPr} & CH_{iPr}), 53.6 (CH_{3-O}), 90.9 (CH_{Ar}), 104.1 (CH_{Ar}), 106.5 (CH_{Ar}), 116.1 (C_{q}), 141.1 (CH_{Ar}), 141.5 (CH_{Ar}), 142.5 (C_{q}), 143.8 (C_{q}), 149.0 (C_{q}), 150.5 (C_{q}), 162.3 (C_{q}).
**MS (ESI+) : m/z calcd for C₁₅H₁₆ClN₅O :** 318.1 [M+H]+, **found :** 318.3.

### Example 2.17: Synthesis of 5-chloro-3-isopropyl-N-(5-methoxypyridin-2-yl)pyrazolo[1,5-a]pyrimidin-7-amine (2.17)

To a solution of intermediate (**1.3**) (0.125 g, 0.54 mmol, 1.0 eq.) and 5-methoxypyridin-2-amine (0.106 g, 0.82 mmol, 1.5 eq.) in dry THF (6.0 mL) under argon was added t-BuOK 1M THF (0.81 mL, 0.82 mmol, 1.5 eq.) dropwise at 0 °C then the mixture was stirred at room temperature during 2.5 h. After completion, saturated NH₄Cl (1.0 mL), H₂O (0.5 mL) and EtOAc (3.0 mL) was added and the biphasic mixture was vigorously stirred 5min. Layers were separated and aqueous layer was extracted with EtOAc (2 x 3.0 mL). The combined organic phases were dried over MgSO₄ and concentrated. The residue was purified by flash chromatography with DCM/EtOAc (100/0 to 96/4) as eluent to give intermediate **(2.17)** (0.136 g, 79 *%*) as a white solid.
**Rf** (DCM, 100*%*) : 0.58.
**¹H NMR (400 MHz, DMSO-*d*₆) δ :** 1.32 (d, *J =* 6.8 Hz, 6H, 2xCH_{3 iPr}), 3.15 (hept, *J* = 7.1 Hz, 1H, H_{iPr}), 3.84 (s, 3H, CH_{3-O}), 7.51 (dt, *J =* 9.1, 2.4 Hz, 1H, H_{Ar}), 7.61 (dd, *J* = 9.0, 1.8 Hz, 1H, H_{Ar}), 7.76 (s, 1H, H_{Ar}), 8.14 (s, 1H, H_{Ar}), 8.18 (t, *J=* 2.5 Hz, 1H, H_{Ar}), 10.57 (s, 1H, NH).
**¹³C NMR (101 MHz, DMSO-*d₆*) δ :** 23.2 (CH₃ & CH_{iPr}), 55.9 (CH_{3-O}), 89.3 (CH_{Ar}), 115.9 (C_{q}), 116.0 (CH_{Ar}), 124.8 (CH_{Ar}), 133.0 (CH_{Ar}), 141.3 (CH_{Ar}), 142.9 (C_{q}), 143.8 (C_{q}), 145.7 (C_{q}), 149.2 (C_{q}), 152.1 (C_{q}).
**MS (ESI+): m/z calcd for C₁₅H₁₆ClN₅O :** 318.1 [M+H]+, **found :** 318.3.

### Example 2.18: Synthesis of 5-chloro-3-isopropyl-N-(6-methoxypyridin-3-yl)pyrazolo[1,5-a]pyrimidin-7-amine (2.18)

To a solution of intermediate (**1.3**) (0.150 g, 0.65 mmol, 1.0 eq.) and 6-methoxypyridin-3-amine (0.08 mL, 0.98 mmol, 1.5 eq.) in dry THF (3.0 mL) under argon was added t-BuOK 1M THF (0.98 mL, 0.98 mmol, 1.5 eq.) dropwise at 0 °C. The mixture was stirred at room temperature during 17 h. The reaction was monitored by CCM. It was neutralized with saturated NH₄Cl (1.0 mL) and H₂O (0.5 mL). The aqeous layer was extracted with EtOAc (3 x 2.5 mL) and the combined organic phases were dried over MgSO₄ and concentrated. The residue was purified by flash chromatography with DCM/MeOH (98/2) as eluent to give intermediate (**2.18**) (0.143 g, 69 *%*) as a yellow/orange solid.
**Rf** (DCM/MeOH, 98/2) : 0.84
**¹H NMR (400 MHz, DMSO-*d₆*) δ :** 1.31 (d, *J* = 6.9 Hz, 6H, 2 X CH_{3 iPr}), 3.14 (p, *J* = 6.9 Hz, 1H, CH _{iPr}), 3.89 (s, 3H, O-CH₃), 5.85 (s, 1H, CH_{Ar}), 6.94 (d, *J* = 8.8 Hz, 1H, CH_{Ar}), 7.80 (dd, *J* = 8.8, 2.8 Hz, 1H, CH_{Ar}), 8.14 (s, 1H, CH_{Ar}), 8.24 (d, *J* = 2.7 Hz, 1H, CH_{Ar}), 10.15 (s, 1H, NH).
¹³C NMR (101 MHz, DMSO-*d₆*) δ : 23.2 (CH_{3 iPr}), 23.2 (CH_{3 iPr}), 30.7 (CH _{iPr}), 53.5 O-CH₃), 84.8 (CH_{Ar}), 111.1 (CH_{Ar}), 115.6 (C_{q}), 127.3 (C_{q}), 137.2 (CH_{Ar}), 142.0 (CH_{Ar}), 144.1 (C_{q}), 144.1 (CH_{Ar}), 147.0 (C_{q}), 149.1 (C_{q}), 161.9 (C_{q}).
MS (ESI+) : m/z calcd for C₁₅H₁₆ClN₅O : 318.1 [M+H]+, found : 318.3.

### Example 2.19: Synthesis of 5-chloro-3-isopropyl-N-(2-methoxypyrimidin-4-yl)pyrazolo[1,5-a]pyrimidin-7-amine (2.19)

Under inert gas, to a solution of intermediate (1.3) (0.250 g, 1.09 mmol, 1.0 eq.) and 2-methoxypyrimidin-4-amine (0.222 g, 1.63 mmol, 1.5 eq.) in dry THF (6.0 mL), tBuOK 1M in THF (1.63 mL, 1.63 mmol, 1.5 eq.) was added dropwise. The mixture was stirred 1.5 h at room temperature then saturated NH₄Cl (1.0 mL), water (0.5 mL) and EtOAc (3.0 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/MeOH (99/1 to 98/2) as eluent to give intermediate **(2.19)** (0.240 g, 69 %) as a white solid.
**Rf** (DCM/MeOH, 96/4) : 0.56.
**¹H NMR (400 MHz, DMSO-*d₆*)** δ : 1.32 (d, *J* = 6.9 Hz, 6H, 2xCH_{3 iPr}), 3.18 (hept, *J* = 6.9 Hz, 1H, H_{iPr}), 3.96 (s, 3H, CH_{3-O}), 7.35 (d, *J* = 5.7 Hz, 1H, H_{Ar}), 8.04 (s, 1H, H_{Ar}), 8.21 (s, 1H, H_{Ar}), 8.43 (d, *J =* 5.6 Hz, 1H, H_{Ar}), 11.09 (s, 1H, NH).
**¹³C NMR (101 MHz, DMSO-*d₆*) δ** : 23.1 (2xCH_{3 iPr}), 23.2 (CH_{iPr}), 54.5 (CH_{3-O}), 93.5 (CH_{Ar}), 104.6 (CH_{Ar}), 116.5 (C_{q}), 141.7 (C_{q}), 141.8 (CH_{Ar}), 143.7 (C_{q}), 148.8 (C_{q}), 159.1 (CH_{Ar}), 160.6 (C_{q}), 164.3 (C_{q}).
**MS (ESI+) : m/z calcd for C₁₄H₁₅ClN₆O** : 319.1 [M+H]+, **found :** 319.2.

### Example 2.20: Synthesis of 5-chloro-3-isopropyl-N-(6-methoxypyridazin-3-yl)pyrazolo[1,5-a]pyrimidin-7-amine (2.20)

Under inert gas, to a solution of intermediate **(1.3)** (0.200 g, 0.87 mmol, 1.0 eq.) and 6-methoxypyrimidin-3-amine (0.172 g, 1.30 mmol, 1.5 eq.) in dry THF (8.0 mL), tBuOK 1M in THF (1.3 mL, 1.30 mmol, 1.5 eq.) was added dropwise. The mixture was stirred 3 h at room temperature then saturated NH₄Cl (1.0 mL), water (0.5 mL) and EtOAc (3.0 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/EtOAc (70/30 to 50/50) as eluent to give intermediate **(2.20)** (0.240 g, 69 %) as a white solid.
**¹H NMR (400 MHz, DMSO-*d*₆) δ :** 1.33 (d, *J* = 6.9 Hz, 6H, 2xCH_{3 iPr}), 3.18 (hept, *J* = 7.0 Hz, 1H, H_{iPr}), 4.03 (s, 3H, CH_{3-O}), 7.33 (d, *J* = 9.4 Hz, 1H, H_{Ar}), 7.93 (s, 1H, H_{Ar}), 7.94 (d, *J =* 9.4 Hz, 1H, H_{Ar}), 8.19 (s, 1H, H_{Ar}), 10.83 (s, 1H, NH).
**¹³C NMR (101 MHz, DMSO-*d₆*) δ :** 23.2 (2xCH_{3 iPr}), 23.2 (CH_{iPR}), 54.5 (CH_{3-O}), 91.1 (CH_{Ar}), 116.2 (C_{q}), 120.0 (CH_{Ar}), 124.4 (CH_{Ar}), 141.6 (CH_{Ar}), 142.3 (C_{q}), 143.8 (C_{q}), 149.3 (C_{q}), 152.4 (C_{q}), 162.0 (C_{q}).
**MS (ESI+) : m/z calcd for C₁₄H₁₅ClN₆O :** 319.1 [M+H]+, **found :** 319.2.

### Example 2.21: Synthesis of 5-chloro-3-isopropyl-N-(6-methoxypyrimidin-4-yl)pyrazolo[1,5-a]pyrimidin-7-amine (2.21)

Under inert gas at 0 °C, to a solution of 6-methoxypyrimidin-4-amine (0.104 g, 0.82 mmol, 1.5 eq.) and intermediate **(1.3)** (0.125 g, 0.55 mmol, 1.0 eq.) in dry THF (6.0 mL), tBuOK 1M THF (0.81 mL, 0.82 mmol, 1.5 eq.) was added dropwise and the mixture was stirred 3 h at room temperature. After completion, saturated NH₄Cl (1.0 mL), H₂O (0.5 mL) and EtOAc (3.0 mL) were added and biphasic mixture was stirred vigorously 5 min. Aqueous layer was extracted with EtOAc (2x3.0 mL) and the combined organic phases were dried over MgSO₄, filtered and concentrated. The residue was purified by flash chromatography with DCM/EtOAc (100/0 to 96/4) as eluent to give intermediate **(2.21)** (0.127 g, 73 %) as a white solid.
**Rf** (DCM, 100 %) : 0.45.
**¹H NMR (400 MHz, DMSO-*d*₆) δ :** 1.32 (d, *J* = 6.9 Hz, 6H, CH_{3 iPr}), 3.17 (hept, *J* = 6.9 Hz, 1H, H_{iPr}), 3.93 (s, 3H, CH_{3-O}), 7.12 (d, *J* = 0.9 Hz, 1H, H_{Ar}), 8.02 (s, 1H, H_{Ar}), 8.20 (s, 1H, H_{Ar}), 8.72 (d, *J* = 0.9 Hz, 1H, H_{Ar}), 10.92 (s, 1H, NH).
**¹³C NMR (101 MHz, DMSO-*d₆*) δ :** 23.2 (2×CH_{3 iPr}), 23.2 (CH_{iPr}), 54.0 (CH_{3-O}), 92.7 (CH_{Ar}), 94.3 (CH_{Ar}), 116.4 (C_{q}), 141.7 (CH_{Ar}), 142.1 (C_{q}), 143.7 (C_{q}), 148.9 (C_{q}), 157.7 (CH_{Ar}), 159.8 (C_{q}), 170.0 (C_{q}).
**MS (ESI+) : m/z calcd for C₁₄H₁₅ClN₆O :** 319.1 [M+H]+, **found :** 319.3.

### Example 2.22: Synthesis of 5-chloro-3-cyclopropyl-N-(2-(2-pyridyl)ethyl)pyrazolo[1,5-a]pyrimidin-7-amine (2.22)

A solution of intermediate **(1.4)** (0.125 g, 0.55 mmol, 1.0 eq.), 2-(pyridin-2-yl)ethan-1-amine (0.080 g, 66 mmol, 1.2 eq.), Et₃N (0.09 mL, 0.66 mmol, 1.2 eq.) in 2-MeTHF (3.0 mL) was refluxed 5 min. After cooling, THF (10.0 mL) was added and the solid was triturated, filtered off, washed with THF. The filtrate was concentrated and the residue was purified by flash chromatography with DCM/MeOH (99/1 to 95/5) as eluent to give intermediate (2.22) (0.160 g, 93 %) as a white solid.
**¹H NMR (400 MHz, DMSO-*d₆*) δ :** 0.70 - 0.75 (m, 2H, 2xH_{(CH2) cPr}), 0.83 - 0.89 (m, 2H, 2xH_{(CH2) cPr}), 1.87 (tt, *J* = 8.4, 5.1 Hz, 1H, H_{cPr}), 3.09 (t, *J* = 7.0 Hz, 2H, CH_{2-CH2-NH}), 3.75 (q, *J* = 6.8 Hz, 2H, CH_{2-NH}), 6.10 (s, 1H, H_{Ar}), 7.23 (ddd, *J =* 7.6, 4.9, 1.2 Hz, 1H, H_{Ar}), 7.32 (dt, *J =* 7.9, 1.1 Hz, 1H, H_{Ar}), 7.69 (td, *J* = 7.6, 1.8 Hz, 1H, H_{Ar}), 7.87 (s, 1H, H_{Ar}), 8.31 (t, *J* = 6.1 Hz, 1H, NH_{-CH2}), 8.51 (ddd, *J* = 4.9, 1.9, 0.9 Hz, 1H, H_{Ar}).
**¹³C NMR (101 MHz, DMSO-*d₆*) δ** : 4.7 (CH_{cPr}), 6.9 (2xCH_{2cPr}), 36.4 (CH_{2-CH2-NH}), 41.1 (CH_{2-NH}), 83.5 (CH_{Ar}), 110.5 (C_{q}), 121.7 (CH_{Ar}), 123.5 (CH_{Ar}), 136.6 (CH_{Ar}), 141.6 (CH_{Ar}), 144.6 (C_{q}), 147.2 (C_{q}), 149.1 (CH_{Ar}), 149.4 (C_{q}), 158.5 (C_{q}).
**MS (ESI+) : m/z calcd for C₁₆H₁₆ClN₅ :** 314.1 [M+H]+, **found :** 314.2.

### Example 2.23: Synthesis of 5-chloro-3-cyclopropyl-N-(pyridin-3-yl)pyrazolo[1,5-a]pyrimidin-7-amine (2.23)

Under inert gas at 0 °C, to a solution of 3-aminopyridine (0.072 g, 0.77 mmol, 1.4 eq.) and intermediate **(1.4)** (0.125 g, 0.55 mmol, 1.0 eq.) in dry THF (5.0 mL), tBuOK 1M THF (0.71 mL, 0.71 mmol, 1.3 eq.) was added dropwise and the mixture was stirred 21 h at room temperature. After completion, saturated NH₄Cl (1.0 mL), H₂O (0.5 mL) and EtOAc (3.0 mL) were added and biphasic mixture was stirred vigorously 5 min. Aqueous layer was extracted with EtOAc (2x3.0 mL) and the combined organic phases were dried over MgSO₄, filtered and concentrated. The residue was purified by flash chromatography with cyclohexane/DCM/EtOAc (50/48/2 to 50/45/5) as eluent to give intermediate **(2.23)** (0.060 g, 38 %) as a white solid.
**¹H NMR (400 MHz, DMSO-*d₆*) δ :** 0.76 - 0.82 (m, 2H, 2xH_{(CH2) cPr}), 0.88 - 0.95 (m, 2H, 2xH_{(CH2) cPr}), 1.94 (tt, *J* = 8.4, 5.2 Hz, 1H, H_{cPr}), 6.09 (s, 1H, H_{Ar}), 7.51 (ddd, *J* = 8.1, 4.7, 0.8 Hz, 1H, H_{Ar}), 7.92 (ddd, *J* = 8.2, 2.7, 1.5 Hz, 1H, H_{Ar}), 8.05 (s, 1H, H_{Ar}), 8.50 (dd, *J* = 4.7, 1.5 Hz, 1H, H_{Ar}), 8.69 (dd, *J* = 2.6, 0.8 Hz, 1H, H_{Ar}), 10.39 (s, 1H, NH).
**¹³C NMR (101 MHz, DMSO-*d₆*) δ :** 4.7 (CH_{cPr}), 7.0 (2xCH_{2 cPr}), 85.3 (CH_{Ar}), 111.3 (C_{q}), 124.2 (CH_{Ar}), 132.1 (CH_{Ar}), 133.6 (C_{q}), 142.2 (CH_{Ar}), 144.9 (C_{q}), 146.0 (C_{q}), 146.1 (CH_{Ar}), 147.0 (CH_{Ar}), 149.2 (C_{q}).
**MS (ESI+) : m/z calcd for C₁₄H₁₂ClN₅ :** 286.1 [M+H]+, **found :** 286.1.

### Example 2.24: Synthesis of 5-chloro-3-cyclopropyl-N-(pyridin-2-yl)pyrazolo[1,5-a]pyrimidin-7-amine (2.24)

Under inert gas at 0 °C, to a solution of 2-aminopyridine (0.072 g, 0.77 mmol, 1.4 eq.) and intermediate **(1.4)** (0.125 g, 0.55 mmol, 1.0 eq.) in dry THF (5.0 mL), tBuOK 1M THF (0.71 mL, 0.71 mmol, 1.3 eq.) was added dropwise and the mixture was stirred 21 h at room temperature. After completion, saturated NH₄Cl (1.0 mL), H₂O (0.5 mL) and EtOAc (3.0 mL) were added and biphasic mixture was stirred vigorously 5 min. Aqueous layer was extracted with EtOAc (2x3.0 mL) and the combined organic phases were dried over MgSO₄, filtered and concentrated. The residue was purified by flash chromatography with DCM/EtOAc (95/5) as eluent to give intermediate **(2.24)** (0.100 g, 64 %) as a white solid.
**¹H NMR (400 MHz, DMSO-*d₆*) δ :** 0.77 - 0.84 (m, 2H, 2xH_{(CH2) cPr}), 0.88 - 0.97 (m, 2H, 2xH_{(CH2) cPr}), 1.96 (tt, *J =* 8.4, 5.1 Hz, 1H, H_{cPr}), 7.15 (ddd, *J* = 7.3, 5.0, 1.0 Hz, 1H, H_{Ar}), 7.66 (dt, *J* = 8.4, 1.0 Hz, 1H, H_{Ar}), 7.83 (ddd, *J* = 8.3, 7.3, 2.0 Hz, 1H, H_{Ar}), 7.96 (s, 1H, H_{Ar}), 8.05 (s, 1H, H_{Ar}), 8.45 (ddd, *J* = 5.0, 2.0, 0.8 Hz, 1H, H_{Ar}), 10.63 (s, 1H, NH).
**¹³C NMR (101 MHz, DMSO-*d₆*) δ :** 4.7 (CH_{cPr}), 7.1 (2xCH_{2 cPr}), 90.7 (CH_{Ar}), 111.6 (C_{q}), 114.9 (CH_{Ar}), 119.0 (CH_{Ar}), 138.4 (CH_{Ar}), 141.5 (CH_{Ar}), 142.7 (C_{q}), 144.6 (C_{q}), 147.3 (CH_{Ar}), 149.3 (C_{q}), 152.4 (C_{q}).
**MS (ESI+) : m/z calcd for C₁₄H₁₂ClN₅ :** 286.1 [M+H]+, **found :** 286.1.

### Example 2.25: Synthesis of 5-chloro-3-cyclopropyl-N-(pyrimidin-4-yl)pyrazolo[1,5-a]pyrimidin-7-amine (2.25)

To a solution of pyrimidin-4-amine (0.046 g, 0.48 mmol, 1.1 eq.) in dry THF (1 mL) under argon was added t-BuOK 1M THF (0.57 mL, 0.57 mmol, 1.3 eq.) at 0 °C. The mixture was stirred during 5 minutes before addition dropwise at 0 °C of intermediate **(1.4)** (0.1 g, 0.44 mmol, 1.0 eq.) previously diluted in dry THF. The mixture was then stirred at room temperature during 4 h, neutralized with saturated NH₄Cl (1.0 mL) and H₂O (0.5 mL). The aqueous layer was extracted with EtOAc (3 × 2.5 mL) and the combined organic phases were dried over MgSO₄ and concentrated. The residue was purified by flash chromatography with DCM/MeOH (100/0 to 98/2) as eluent to give intermediate **(2.25)** (0.035 g, 28 %) as a yellow solid.
**Rf** (DCM/MeOH, 96/4) : 0.54.
**¹H NMR (400 MHz, DMSO-*d₆*) δ :** 0.78 - 0.85 (m, 2H, CH_{2 cPr}), 0.89 - 0.97 (m, 2H, CH_{2 cPr}), 1.92 - 2.02 (m, 1H, CH _{cPr}), 7.71 (dd, *J* = 5.8, 1.5 Hz, 1H, CH_{Ar}), 8.06 (d, *J* = 2.1 Hz, 1H, CH_{Ar}), 8.10 (d, *J =* 1.9 Hz, 1H, CH_{Ar}), 8.65 (dd, *J* = 5.8, 1.3 Hz, 1H, CH_{Ar}), 9.01 (d, *J* = 1.8 Hz, 1H, CH_{Ar}), 11.07 (s, 1H, NH).
**¹³C NMR (101 MHz, DMSO-*d₆*) δ :** 4.6 (CH _{cPr}), 7.1 (2 × CH_{2 cPr}), 93.2 (CH_{Ar}), 111.1 (CH_{Ar}), 112.1 (C_{q}), 141.8 (CH_{Ar}), 141.9 (C_{q}), 144.6 (C_{q}), 149.1 (C_{q}), 157.3 (CH_{Ar}), 157.7 (CH_{Ar}), 158.5 (C_{q}).
**MS (ESI+) : m/z calcd for C₁₃H₁₁ClN₆ :** 287.1 [M+H]+, **found :** 287.1.

### Example 2.26: Synthesis of 5-chloro-3-cyclopropyl-N-(pyridazin-3-yl)pyrazolo[1,5-a]pyrimidin-7-amine (2.26)

Under inert gas, to a solution of 3-aminopyridazine (0.177 g, 1.84 mmol, 1.4 eq.) in dry THF (15.0 mL), NaH 60% suspended in mineral oil (0.079 g, 1.97 mmol, 1.5 eq.) was added and the mixture was stirred 10 minutes at room temperature. Next, intermediate **(1.4)** (0.300 g, 1.32 mmol, 1.0 eq.) was added then the mixture was stirred 5.5 h at r.t. and after completion, saturated NH₄Cl (2.0 mL), H₂O (1.0 mL) and EtOAc (10.0 mL) were added and vigorously stirred 5 min. The aqueous layer was extracted with EtOAc (2x2.0 mL) and the combined organic phases were dried over MgSO₄ and concentrated. The residue was purified by flash chromatography with DCM/EtOAc (97/3 to 77/23) as eluent to give intermediate **(2.26)** (0.289 g, 77 %) as a yellow solid.
**Rf** (DCM/MeOH, 96/4) : 0.54.
**¹H NMR (400 MHz, DMSO-*d₆*) δ :** 0.82 (h, *J* = 4.1 Hz, 2H, CH_{2 cPr}), 0.90 - 0.97 (m, 2H, CH_{2 cPr}), 1.98 (td, *J* = 8.7, 4.4 Hz, 1H, CH _{cPr}), 7.74 (dd, *J* = 9.0, 4.6 Hz, 1H, CH_{Ar}), 7.97 (d, *J* = 8.7 Hz, 1H, CH_{Ar}), 8.04 (s, 1H, CH_{Ar}), 8.10 (s, 1H, CH_{Ar}), 9.02 (d, *J* = 4.5 Hz, 1H, CH_{Ar}), 10.91 (s, 1H, NH).
**¹³C NMR (101 MHz, DMSO-*d₆*) δ :** 4.7 (CH _{cPr}), 7.1 (2 × CH_{2 cPr}), 92.0 (CH_{Ar}), 112.0 (C_{q}), 119.8 (CH_{Ar}), 128.8 (CH_{Ar}), 141.7 (CH_{Ar}), 142.2 (C_{q}), 144.6 (C_{q}), 148.3 (CH_{Ar}), 149.4 (C_{q}), 156.0(C_{q}).
**MS (ESI+) : m/z calcd for C₁₃H₁₁ClN₆ :** 287.1 [M+H]+, **found :** 287.1.

### Example 2.27: Synthesis of 5-chloro-3-cyclopropyl-N-(pyrazin-2-yl)pyrazolo[1,5-a]pyrimidin-7-amine (2.27)

Under inert gas, to a solution of pyrazin-2-amine (0.094 g, 0.98 mmol, 1.5 eq.) in dry THF (6.0 mL), tBuOK 1M in THF (0.98 mL, 0.98 mmol, 1.5 eq.) was added. The mixture was stirred 5 min at room temperature then intermediate **(1.4)** (0.150 g, 0.65 mmol, 1.0 eq.) was added and stirred 2 h at room temperature. After completion, saturated NH₄Cl (1.0 mL), water (0.5 mL) and EtOAc (3.0 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/MeOH (100/0 to 98/2) as eluent to give intermediate **(2.27)** (0.125 g, 66 %) as a yellow solid.
**¹H NMR (400 MHz, DMSO-*d₆*) δ :** 0.77 - 0.86 (m, 2H, 2xH_{(CH2) cPr}), 0.92 (dt, *J* = 8.5, 3.0 Hz, 2H, 2xH_{(CH2) cPr}), 1.97 (tt, *J* = 8.4, 5.1 Hz, 1H, H_{cPr}), 7.85 (s, 1H, H_{Ar}), 8.10 (s, 1H, H_{Ar}), 8.36 (d, *J* = 2.7 Hz, 1H, H_{Ar}), 8.49 (dd, *J* = 2.7, 1.4 Hz, 1H, H_{Ar}), 8.99 (d, *J* = 1.4 Hz, 1H, H_{Ar}), 11.08 (s, 1H, NH).
**¹³C NMR (101 MHz, DMSO-*d₆*) δ :** 4.7 (CH_{cPr}), 7.1 (2xCH_{2 cPr}), 91.1 (CH_{Ar}), 111.9 (C_{q}), 137.9 (CH_{Ar}), 138.5 (CH_{Ar}), 141.2 (CH_{Ar}), 141.8 (CH_{Ar}), 142.4 (C_{q}), 144.6 (C_{q}), 149.2 (C_{q}), 149.3 (C_{q}).
**MS (ESI+) : m/z calcd for C₁₃H₁₁ClN₆ :** 287.1 [M+H]+, **found** : 287.1.

### Example 2.28: Synthesis of 5-chloro-3-cyclopropyl-N-(pyrimidin-5-yl)pyrazolo[1,5-a]pyrimidin-7-amine (2.28)

Under inert gas, to a solution of pyrimidin-5-amine (0.073 g, 0.77 mmol, 1.4 eq.) and intermediate **(1.4)** (0.125 g, 0.55 mmol, 1.0 eq.) in dry THF (6.0 mL), tBuOK 1M THF (0.71 mL, 0.71 mmol, 1.3 eq.) was added dropwise and the mixture was stirred 21 h at room temperature. After completion, saturated NH₄Cl (1.0 mL), H₂O (0.5 mL) and EtOAc (3.0 mL) were added and biphasic mixture was stirred vigorously 5 min. Aqueous layer was extracted with EtOAc (2x3.0 mL) and the combined organic phases were dried over MgSO₄, filtered and concentrated. The residue was purified by flash chromatography with DCM/EtOAc (95/5 to 80/20) as eluent to give intermediate **(2.28)** (0.086 g, 55 %) as a white solid.
**¹H NMR (400 MHz, DMSO-*d₆*) δ :** 0.76 - 0.83 (m, 2H, 2xH_{(CH2) cPr}), 0.88 - 0.95 (m, 2H, 2xH_{(CH2) cPr}), 1.95 (tt, *J* = 8.4, 5.8 Hz, 1H, H_{cPr}), 6.32 (s, 1H, H_{Ar}), 8.07 (s, 1H, H_{Ar}), 8.96 (s, 2H, 2xH_{Ar}), 9.10 (s, 1H, H_{Ar}), 10.44 (s, 1H, NH).
**¹³C NMR (101 MHz, DMSO-*d₆*) δ :** 4.7 (CH_{cPr}), 7.1 (2xCH_{2 cPr}), 86.1 (CH_{Ar}), 111.4 (C_{q}), 132.9 (C_{q}), 142.3 (CH_{Ar}), 144.9 (C_{q}), 145.6 (C_{q}), 149.3 (C_{q}), 152.9 (CH_{Ar}), 155.3 (CH_{Ar}).
**MS (ESI+) : m/z calcd for C₁₃H₁₁ClN₆ :** 287.1 [M+H]+, **found :** 287.1.

### Example 2.29: Synthesis of 5-chloro-3-cyclopropyl-N-(6-methoxypyridin-2-yl)pyrazolo[1,5-a]pyrimidin-7-amine (2.29)

To a solution of 6-methoxypyridin-2-amine (0.110 g, 0.89 mmol, 1.5 eq.) in dry THF (1 mL) under argon was added t-BuOK 1M THF (0.89 mL, 0.89 mmol, 1.5 eq.) at 0 °C. The mixture was stirred during 5 minutes before addition dropwise at 0 °C of intermediate **(1.4)** (0.135 g, 0.59 mmol, 1.0 eq.) previously diluted in dry THF. The mixture was then stirred at room temperature during 4 h, neutralized with saturated NH₄Cl (1.0 mL) and H₂O (0.5 mL). The aqueous layer was extracted with EtOAc (3 × 2.5 mL) and the combined organic phases were dried over MgSO₄ and concentrated. The residue was purified by flash chromatography with DCM/MeOH (100/0 to 98/2) as eluent to give intermediate (2.29) (0.051 g, 27 %) as a white solid.
**Rf** (DCM/MeOH, 98/2) : 0.51
**¹H NMR (400 MHz, DMSO-*d₆*) δ :** 0.75 - 0.85 (m, 2H, CH_{2 cPr}), 0.88 - 0.98 (m, 2H, CH_{2 cPr}), 1.96 (tt, *J* = 8.4, 5.1 Hz, 1H, CH _{cPr}), 6.57 (d, *J* = 8.0 Hz, 1H, CH_{Ar}), 7.24 (d, *J* = 7.8 Hz, 1H, CH_{Ar}), 7.74 (t, *J* = 8.0 Hz, 1H, CH_{Ar}), 7.93 (s, 1H, CH_{Ar}), 8.05 (s, 1H, CH_{Ar}), 10.63 (s, 1H, NH).
**¹³C NMR (101 MHz, DMSO-*d₆*) δ :** 4.7 (CH _{cPr}), 7.1 (2 × CH_{2 cPr}), 53.6 (O-CH₃), 90.9 (CH_{Ar}), 104.1 (CH_{Ar}), 106.5 (CH_{Ar}), 111.6 (C_{q}), 141.1 (CH_{Ar}), 141.5 (CH_{Ar}), 142.6 (C_{q}), 144.7 (C_{q}), 149.2 (C_{q}), 150.5 (C_{q}), 162.4 (C_{q}).
**MS (ESI+) : m/z calcd for C₁₅H₁₄ClN₅O :** 316.1 [M+H]+, **found :** 316.2.

### Example 2.30: Synthesis of 5-chloro-3-cyclopropyl-N-(6-methoxypyridazin-3-yl)pyrazolo[1,5-a]pyrimidin-7-amine (2.30)

To a solution of 6-methoxypyridazin-3-amine (0.111 g, 0.89 mmol, 1.5 eq.) in dry THF (1 mL) under argon was added t-BuOK 1M THF (0.89 mL, 0.89 mmol, 1.5 eq.) at 0 °C. The mixture was stirred during 5 minutes before addition dropwise at 0 °C of intermediate **(1.4)** (0.135 g, 0.59 mmol, 1.0 eq.) previously diluted in dry THF. The mixture was then stirred at room temperature during 4 h, neutralized with saturated NH₄Cl (1.0 mL) and H₂O (0.5 mL). The aqueous layer was extracted with EtOAc (3 × 2.5 mL) and the combined organic phases were dried over MgSO₄ and concentrated. The residue was purified by flash chromatography with cyclohexane/EtOAc (70/30 to 50/50) as eluent to give intermediate (2.30) (0.023 g, 12 %) as a yellow solid.
**Rf** (EtOAc/Cyclohexane, 50/50) : 0.58
**¹H NMR (400 MHz, DMSO-*d₆*) δ :** 0.82 (dt, *J* = 5.3, 2.9 Hz, 2H, CH_{2 cPr}), 0.92 (dt, *J* = 8.5, 2.9 Hz, 2H, CH_{2 cPr}), 1.93 - 2.01 (m, 1H, CH _{cPr}), 4.03 (s, 3H, CH₃), 7.34 (d, *J* = 9.4 Hz, 1H, CH_{Ar}), 7.91 (s, 1H, CH_{Ar}), 7.93 (d, *J* = 9.4 Hz, 1H, CH_{Ar}), 8.08 (s, 1H, CH_{Ar}), 10.81 (s, 1H, NH).
**¹³C NMR (101 MHz, DMSO-*d₆*) δ :** 4.7 (CH_{cPr}), 7.1 (2 × CH_{2 cPr}), 54.5 (CH₃), 91.1 (CH_{Ar}), 111.8 (C_{q}), 120.1 (CH_{Ar}), 124.4 (CH_{Ar}), 141.7 (CH_{Ar}), 142.3 (C_{q}), 144.6 (C_{q}), 149.4 (C_{q}), 152.4 (C_{q}), 162.0 (C_{q}).
**MS (ESI+) : m/z calcd for C₁₄H₁₃ClN₆O :** 317.1 [M+H]+, **found :** 317.1.

### Example 2.31: Synthesis of 5-chloro-3-cyclopropyl-N-(2-methoxypyrimidin-4-yl)pyrazolo[1,5-a]pyrimidin-7-amine (2.31)

To a solution of 2-methoxypyrimidin-4-amine (0.077 g, 0.61 mmol, 1.5 eq.) in dry THF was added t-BuOK 1M THF (0.61 mL, 0.61 mmol, 1.5 eq.) dropwise at 0 °C. The mixture was stirred for 5 minutes before addition of intermediate **(1.4)** (0.093 g, 0.41 mmol, 1.0 eq.) dropwise at 0 °C. The mixture was then stirred at room temperature during 20 h. The reaction was monitored by CCM. It was neutralized with saturated NH₄Cl (1.0 mL) and H₂O (0.5 mL). The aqueous layer was extracted with EtOAc (3 × 2.5 mL) and the combined organic phases were dried over MgSO₄ and concentrated. The residue was purified by flash chromatography with DCM/MeOH (100/0 to 98/2 then 98/2) as eluent to give intermediate **(2.31)** (0.074 g, 57 %) as a white solid.
**Rf** (DCM/MeOH, 98/2) : 0.51
**¹H NMR (400 MHz, DMSO-*d₆*) δ :** 0.77 - 0.85 (m, 2H, CH_{2 cPr}), 0.90 - 0.97 (m, 2H, CH_{2 cPr}), 1.92 - 2.03 (m, 1H, CH _{cPr}), 3.97 (s, 3H, O-CH₃), 7.35 (d, *J* = 5.6 Hz, 1H, CH_{Ar}), 8.03 (s, 1H, CH_{Ar}), 8.10 (s, 1H, CH_{Ar}), 8.44 (d, *J* = 5.6 Hz, 1H, CH_{Ar}), 11.08 (s, 1H, NH).
**¹³C NMR (101 MHz, DMSO-*d₆*) δ :** 4.6 (CH_{cPr}), 7.1 (2 × CH_{2 cPr}), 54.6 (O-CH₃), 93.6 (CH_{Ar}), 104.6 (CH_{Ar}), 112.1 (C_{q}), 141.8 (C_{q}), 141.8 (CH_{Ar}), 144.6 (C_{q}), 149.0 (C_{q}), 159.2 (CH_{Ar}), 160.5 (C_{q}), 164.3 (C_{q}).
**MS (ESI+) : m/z calcd for C₁₄H₁₃ClN₆O :** 317.1 [M+H]+, **found :** 317.2.

### Example 2.32: Synthesis of 5-chloro-3-isopropyl-N-(4-methoxybenzyl)pyrazolo[1,5-a]pyrimidin-7-amine (2.32)

A microwave vial 10 - 20 mL with a stir bar was charged with **(1.3)** (1.000 g, 4.75 mmol, 1.0 eq.), THF (10.0 mL), (4-methoxyphenyl)methanamine (0.76 mL, 5.70 mmol, 1.2 eq.) and Et₃N (0.87 mL, 6.71 mmol, 1.3 eq.). The vial was closed and put in a microwave reactor: 100 °C, 30 min. After cooling, the mixture was diluted in THF then the solid was filtered off, washed with THF and the filtrate was concentrated to give **(2.32)** (1.560 g, 99 %) as a light yellow solid.
**¹H NMR (400 MHz, DMSO-*d₆*) δ :** 1.27 (d, *J* = 6.9 Hz, 6H, 2xCH_{3 iPr}), 3.08 (hept, *J* = 6.9 Hz, 1H, H_{iPr}), 3.71 (s, 3H, CH_{3-O}), 4.54 (s, 2H, CH_{2 Bn}), 6.07 (s, 1H, H_{Ar}), 6.87 - 6.92 (m, 2H, 2xH_{Ar}), 7.30 - 7.38 (m, 2H, 2xH_{Ar}), 8.03 (s, 1H, H_{Ar}), 8.84 (s, 1H, NH_{Bn}).
**¹³C NMR (101 MHz, DMSO-*d₆*) δ :** 23.1 (CH_{iPr}), 23.2 (2xCH_{3 iPr}), 43.8 (CH_{2 Bn}), 55.0 (CH_{3-O}), 84.0 (CH_{Ar}), 113.9 (2xCH_{Ar}), 115.1 (C_{q}), 128.5 (2xCH_{Ar}), 129.6 (C_{q}), 141.5 (CH_{Ar}), 143.8 (C_{q}), 147.2 (C_{q}), 149.0 (C_{q}), 158.5 (C_{q}).
**MS (ESI+) : m/z calcd for C₁₄H₁₅ClN₆O :** 331.1 [M+H]+, **found :** 331.2.

### Example 3: Synthesis of pyrazolo[1,5-a]pyrimidin-5,7-diamine derivatives, as illustrated in step 4 of scheme 1

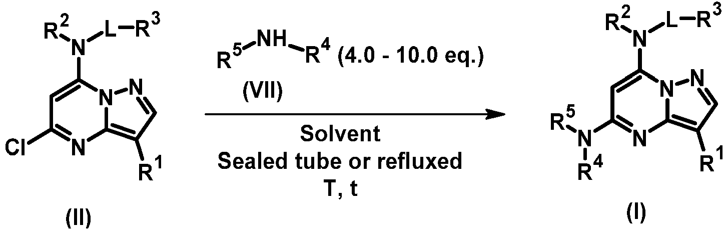

### Example 3.1: Synthesis of (2R)-2-((7-(benzylamino)-3-isopropylpyrazolo[1,5-a]pyrimidin-5-yl)amino)butan-1-ol (1)

In a sealed tube 2-5 mL with a stir bar was charged intermediate **(2.1)** (0.130 g, 0.43 mmol, 1.0 eq.) and (S)-2-aminobutan-1-ol (0.63 mL, 6.48 mmol, 15.0 eq.). The vial was sealed and then put on heating block: 100 °C, 3.5 h. The mixture was concentrated and directly purified by flash chromatography with DCM/MeOH (99/1 to 96/4) as eluent to give the title compound **(1)** (0.050 g, 33 %) as a white solid.

### Example 3.2: Synthesis of N7-Benzyl-3-isopropyl-N5-[(3R)-tetrahydrofuran-3-yl]pyrazolo[1,5-a]pyrimidine-5,7-diamine (2)

In a sealed tube 2-5 mL with a stir bar was charged intermediate (2.1) (0.200 g, 0.66 mmol, 1.0 eq) was added (*R*)-3-aminotetrahydrofuran (0.20 mL, 2.33 mmol, 3.5 eq). The vial was sealed and then put on heating block: 160 °C, 16 h. After cooling, the mixture was diluted into DCM (15.0 mL) and washed with saturated NH₄Cl (15.0 mL). The aqueous layer was extracted twice with DCM (2 × 15 mL). The combined organic layers were washed with brine, dried over MgSO₄, filtered and concentrated. The crude residue was purified by flash chromatography with cyclohexane/EtOAc (100/0 to 70/30) to afford compound (2) (0.066 g, 28 %) as a slightly yellow solid.

### Example 3.3: Synthesis of N7-benzyl-3-isopropyl-N5-(pentan-3-yl)pyrazolo[1,5-a]pyrimidine-5,7-diamine (3)

In a sealed tube 2 - 5 mL with a stir bar was charged intermediate **(2.1)** (0.150 g, 0.50 mmol, 1.0 eq.), NMP (1.0 mL) and pentan-3-amine (0.29 mL, 2.49 mmol, 5.0 eq.). The vial was sealed and then put on heating block: 170 °C, 21 h. After cooling, the mixture was poured in H₂O (20.0 mL) and extracted with EtOAc (3×15.0 mL). Organic layers were combined, washed with brine, dried over MgSO₄, filtered and concentrated. The residue was purified by flash chromatography with DCM/MeOH (100/0 to 96/4) as eluent to give compound **(3)** (0.090 g, 51 %) as white solid.

### Example 3.4: Synthesis of N7-benzyl-3-isopropyl-N5-(2-methylpyrazol-3-yl)pyrazolo[1,5-a]pyrimidine-5,7-diamine (58)

In a sealed tube 2 - 5 mL with a stir bar was charged intermediate **(2.1)** (0.150 g, 0.50 mmol, 1.0 eq.), cineol (2.0 mL), 1-methyl-1H-pyrazol-5-amine (0.149 g, 1.50 mmol, 3.0 eq.) and tBuOK (0.223 g, 1.99 mmol, 4.0 eq.) then the vial was sealed and put on heating block: 120 °C, 20 h. After cooling, the mixture was concentrated with SiO₂ to make solid deposit and directly purified by flash chromatography with DCM/MeOH (99/1 to 97/3) as eluent to give compound **(58)** (0.140 g, 78 %) as a white solid.

### Example 3.5: Synthesis of 3-isopropyl-N5-(pentan-3-yl)-N7-(pyridin-4-ylmethyl)pyrazolo[1,5-a]pyrimidine-5,7-diamine (4)

In a sealed tube 2 - 5 mL with a stir bar was charged intermediate **(2.2)** (0.150 g, 0.50 mmol, 1.0 eq.), NMP (1.0 mL) and corresponding amine (0.29 mL, 2.49 mmol, 5.0 eq.). The vial was sealed and then put on heating block: 170 °C, 21 h. After cooling, the mixture was poured in H₂O (20.0 mL) and extracted with EtOAc (3×15.0 mL). Organic layers were combined, washed with brine, dried over MgSO₄, filtered and concentrated. The residue was purified by flash chromatography with DCM/MeOH (99/1 to 95/5) as eluent to give compound **(4)** (0.070 g, 40 %) as white solid.

### Example 3.6: Synthesis of 3-isopropyl-N5-(pentan-3-yl)-N7-(pyridin-3-ylmethyl)pyrazolo[1,5-a]pyrimidine-5,7-diamine (5)

In a sealed tube 2 - 5 mL with a stir bar was charged intermediate **(2.3)** (0.150 g, 0.50 mmol, 1.0 eq.), NMP (1.0 mL) and corresponding amine (0.29 mL, 2.49 mmol, 5.0 eq.). The vial was sealed and then put on heating block: 170 °C, 21 h. After cooling, the mixture was poured in H₂O (20.0 mL) and extracted with EtOAc (3×15.0 mL). Organic layers were combined, washed with brine, dried over MgSO₄, filtered and concentrated. The residue was purified by flash chromatography with DCM/MeOH (99/1 to 94/6) as eluent to give compound **(5)** (0.105 g, 60 %) as light brown solid.

### Example 3.7: Synthesis of 3-isopropyl-N5-(pentan-3-yl)-N7-(pyridin-2-ylmethyl)pyrazolo[1,5-a]pyrimidine-5,7-diamine (6)

In a sealed tube 2 - 5 mL with a stir bar was charged intermediate **(2.4)** (0.150 g, 0.50 mmol, 1.0 eq.), NMP (1.0 mL) and corresponding amine (0.29 mL, 2.49 mmol, 5.0 eq.). The vial was sealed and then put on heating block: 170 °C, 21 h. After cooling, the mixture was poured in H₂O (20.0 mL) and extracted with EtOAc (3×15.0 mL). Organic layers were combined, washed with brine, dried over MgSO₄, filtered and concentrated. The residue was purified by flash chromatography with DCM/MeOH (99/1 to 95/5) as eluent to give compound **(6)** (0.130 g, 74 %) as light brown solid.

### Example 3.8: Synthesis of 3-isopropyl-N5-(pentan-3-yl)-N7-(2-(pyridin-2-yl)ethyl)pyrazolo[1,5-a]pyrimidine-5,7-diamine (7)

In a sealed tube 2 - 5 mL with a stir bar was charged intermediate **(2.5)** (0.150 g, 0.48 mmol, 1.0 eq.), NMP (0.5 mL) and corresponding amine (0.39 mL, 3.33 mmol, 7.0 eq.). The vial was sealed and then put on heating block: 170 °C, 20 h. After cooling, the mixture was poured in EtOAc (30.0 mL) then organic layer was washed three times with saturated NaCl + H₂O (3/1, 3x10.0 mL), dried over MgSO₄, filtered and concentrated. The residue was purified by flash chromatography with DCM/MeOH (98/2) as eluent to give compound (7) (0.085 g, 49 %) as a white solid.

### Example 3.9: Synthesis of 3-isopropyl-N5-(pentan-3-yl)-N7-(2-(pyridin-3-yl)ethyl)pyrazolo[1,5-a]pyrimidine-5,7-diamine (8)

In a sealed tube 2 - 5 mL with a stir bar was charged intermediate **(2.6)** (0.150 g, 0.48 mmol, 1.0 eq.), NMP (0.5 mL) and corresponding amine (0.39 mL, 3.33 mmol, 7.0 eq.). The vial was sealed and then put on heating block: 170 °C, 20 h. After cooling, the mixture was poured in EtOAc (30.0 mL) then organic layer was washed three times with saturated NaCl + H₂O (3/1, 3x10.0 mL), dried over MgSO₄, filtered and concentrated. The residue was purified by flash chromatography with DCM/MeOH (98/2 to 95/5) as eluent to give compound **(8)** (0.073 g, 42 %) as a white oil.

### Example 3.10: Synthesis of 3-isopropyl-N5-(pentan-3-yl)-N7-(2-(pyridin-4-yl)ethyl)pyrazolo[1,5-a]pyrimidine-5,7-diamine (9)

In a sealed tube 2 - 5 mL with a stir bar was charged intermediate **(2.7)** (0.150 g, 0.48 mmol, 1.0 eq.), NMP (0.5 mL) and corresponding amine (0.39 mL, 3.33 mmol, 7.0 eq.). The vial was sealed and then put on heating block: 170 °C, 20 h. After cooling, the mixture was poured in EtOAc (30.0 mL) then organic layer was washed three times with saturated NaCl + H₂O (3/1, 3x10.0 mL), dried over MgSO₄, filtered and concentrated. The residue was purified by flash chromatography with DCM/MeOH (98/2 to 95/5) as eluent to give compound **(9)** (0.090 g, 52 %) as a white solid.

### Example 3.11: Synthesis of N5-(2-ethylbutyl)-3-isopropyl-N7-(2-(pyridin-2-yl)ethyl)pyrazolo[1,5-a]pyrimidine-5,7-diamine (10)

In a sealed tube 2 - 5 mL with a stir bar was charged intermediate **(2.5)** (0.100 g, 0.32 mmol, 1.0 eq.), NMP (0.75 mL), corresponding amine hydrochloride (0.256 g, 2.22 mmol, 7.0 eq.) and DiPEA (0.39 mL, 22.2 mmol, 7.0 eq.) then the vial was sealed and put on heating block: 170 °C, 16 h. After cooling, the mixture was diluted in EtOAc (20.0 mL) then the organic layer was washed with a mixture of brine/saturated NaHCO₃ (3/1, 3×15mL), dried over MgSO₄, filtered, concentrated and purified by flash chromatography with DCM/MeOH (99/1) as eluent to give compound **(10)** (0.035 g, 29 %) as a cloudy oil.

### Example 3.12: Synthesis of N5-(2-ethylbutyl)-3-isopropyl-N7-(2-(pyridin-3-yl)ethyl)pyrazolo[1,5-a]pyrimidine-5,7-diamine (11)

In a sealed tube 2 - 5 mL with a stir bar was charged intermediate **(2.6)** (0.100 g, 0.32 mmol, 1.0 eq.), NMP (0.75 mL), corresponding amine hydrochloride (0.256 g, 2.22 mmol, 7.0 eq.) and DiPEA (0.39 mL, 22.2 mmol, 7.0 eq.) then the vial was sealed and put on heating block: 170 °C, 16 h. After cooling, the mixture was diluted in EtOAc (20.0 mL) then the organic layer was washed with a mixture of brine/saturated NaHCO₃ (3/1, 3×15mL), dried over MgSO₄, filtered, concentrated and purified by flash chromatography with DCM/MeOH (99/1) as eluent to give compound **(11)** (0.040 g, 33 %) as a brown solid.

### Example 3.13: Synthesis of N5,N7-bis(2-ethylbutyl)-3-isopropylpyrazolo[1,5-a]pyrimidine-5,7-diamine (12)

In a sealed tube 2 - 5 mL with a stir bar was charged intermediate **(2.6)** (0.100 g, 0.32 mmol, 1.0 eq.), NMP (0.75 mL), corresponding amine hydrochloride (0.256 g, 2.22 mmol, 7.0 eq.) and DiPEA (0.39 mL, 22.2 mmol, 7.0 eq.) then the vial was sealed and put on heating : 170 °C, 16 h. After cooling, the mixture was diluted in EtOAc (20.0 mL) then the organic layer was washed with a mixture of brine/saturated NaHCO₃ (3/1, 3×15mL), dried over MgSO₄, filtered, concentrated and purified by flash chromatography with DCM/MeOH (98/2 to 95/5) as eluent to give the secondary compound **(12)** (0.054 g, 47 %) as a light brown oil.

### Example 3.14: Synthesis of N5-cyclopentyl-3-isopropyl-N7-(2-(2-pyridyl)ethyl)pyrazolo[1,5-a]pyrimidine-5,7-diamine (13)

In a sealed tube 2 - 5 mL with a stir bar was charged intermediate **(2.5)** (0.100 g, 0.32 mmol, 1.0 eq.), NMP (0.75 mL) and cyclopentylamine (0.22 mL, 2.22 mmol, 7.0 eq.) then the vial was sealed and put on heating block: 170 °C, 16 h. After cooling, the mixture was diluted in EtOAc (20.0 mL) then the organic layer was washed with a mixture of brine/saturated NaHCO₃ (3/1, 3x15mL), dried over MgSO₄, filtered, concentrated and purified by flash chromatography with DCM/MeOH (99/1 to 97/3) as eluent to give compound **(13)** (0.038 g, 33 %) as a light brown solid.

### Example 3.15: Synthesis of N5-cyclopentyl-3-isopropyl-N7-(2-(3-pyridyl)ethyl)pyrazolo[1,5-a]pyrimidine-5,7-diamine (14)

In a sealed tube 2 - 5 mL with a stir bar was charged intermediate **(2.6)** (0.100 g, 0.32 mmol, 1.0 eq.), NMP (0.75 mL) and cyclopentylamine (0.22 mL, 2.22 mmol, 7.0 eq.) then the vial was sealed and put on heating block: 170 °C, 17 h. After cooling, the mixture was diluted in EtOAc (20.0 mL) then the organic layer was washed with a mixture of brine/saturated NaHCO₃ (3/1, 3×15.0 mL), dried over MgSO₄, filtered, concentrated and purified by flash chromatography with DCM/MeOH (99/1 to 97/3) as eluent to give compound **(14)** (0.067 g, 58 %) as a brown solid.

### Example 3.16: Synthesis of N5,N7-dicyclopentyl-3-isopropylpyrazolo[1,5-a]pyrimidine-5,7-diamine (15)

In a sealed tube 2 - 5 mL with a stir bar was charged intermediate **(2.6)** (0.100 g, 0.32 mmol, 1.0 eq.), NMP (0.75 mL) and cyclopentylamine (0.22 mL, 2.22 mmol, 7.0 eq.) then the vial was sealed and put on heating block: 170 °C, 17 h. After cooling, the mixture was diluted in EtOAc (20.0 mL) then the organic layer was washed with a mixture of brine/saturated NaHCO₃ (3/1, 3×15.0 mL), dried over MgSO₄, filtered, concentrated and purified by flash chromatography with DCM/MeOH (99/1 to 97/3) as eluent to give the secondary compound **(15)** (0.067 g, 37 %) as a light brown oil.

### Example 3.17: Synthesis of 3-isopropyl-N5-(pentan-3-yl)-N7-(pyridazin-3-ylmethyl)pyrazolo[1,5-a]pyrimidine-5,7-diamine (16)

In a sealed tube 2 - 5 mL with a stir bar was charged intermediate **(2.8)** (0.100 g, 0.33 mmol, 1.0 eq.), NMP (1.0 mL) and pentan-3-amine (0.39 mL, 3.30 mmol, 10.0 eq.) then the vial was sealed and put on heating block: 160 °C, 19 h. After cooling, the mixture was diluted in EtOAc (15.0 mL) then the organic layer was washed with a mixture of brine/saturated NaHCO₃ (3/1, 3x8.0 mL), dried over MgSO₄, filtered, concentrated and purified by flash chromatography with DCM/MeOH (98/2 to 94/6) as eluent to give compound **(16)** (0.056 g, 48 %) as a white solid.

### Example 3.18: Synthesis of 3-isopropyl-N5-(pentan-3-yl)-N7-(pyridazin-4-ylmethyl)pyrazolo[1,5-a]pyrimidine-5,7-diamine (17)

In a sealed tube 2 - 5 mL with a stir bar was charged intermediate **(2.9)** (0.100 g, 0.33 mmol, 1.0 eq.), NMP (1.0 mL) and pentan-3-amine (0.39 mL, 3.30 mmol, 10.0 eq.) then the vial was sealed and put on heating block: 160 °C, 19 h. After cooling, the mixture was diluted in EtOAc (15.0 mL) then the organic layer was washed with a mixture of brine/saturated NaHCO₃ (3/1, 3x8.0 mL), dried over MgSO₄, filtered, concentrated and purified by flash chromatography with DCM/MeOH (98/2 to 95/5) as eluent to give compound **(17)** (0.051 g, 44 %) as a light brown solid.

### Example 3.19: Synthesis of 3-isopropyl-N5-(pentan-3-yl)-N7-(pyridin-2-yl)pyrazolo[1,5-a]pyrimidine-5,7-diamine (18)

In a 2-5 mL vial with a stir bar was charged intermediate **(2.10)** (0.130 g, 0.45 mmol, 1.0 eq.), anisol (1.5 mL) and pentan-3-amine (0.53 mL, 4.52 mmol, 10.0 eq.). The vial was sealed and put on heating block: 180 °C, 16 h. After cooling, the mixture was diluted in THF, the precipitate was filtered off, washed with THF and the filtrate was concentrated. The residue was purified by flash chromatography with DCM/MeOH (99/1 to 97/3) as eluent to give compound **(18)** (0.056 g, 37 %) as a white solid.

### Example 3.20: Synthesis of 3-isopropyl-N5-(pentan-3-yl)-N7-(pyridin-3-yl)pyrazolo[1,5-a]pyrimidine-5,7-diamine (19)

In a 2-5 mL vial with a stir bar was charged intermediate **(2.11)** (0.080 g, 0.28 mmol, 1.0 eq.), anisol (1.0 mL) and pentan-3-amine (0.32 mL, 2.78 mmol, 10.0 eq.). The vial was sealed and put on heating block: 180 °C, 22 h. After cooling, the mixture was concentrated and the residue was directly purified by flash chromatography with DCM/MeOH (99/1 to 96/4) as eluent to give compound **(19)** (0.050 g, 53 %) as a white solid.

### Example 3.21: Synthesis of 3-isopropyl-N5-(pentan-3-yl)-N7-(pyridazin-3-yl)pyrazolo[1,5-a]pyrimidine-5,7-diamine (20)

In a sealed tube 2 - 5 mL with a stir bar was charged intermediate **(2.12)** (0.050 g, 0.17 mmol, 1.0 eq.), NMP (0.5 mL) and pentan-3-amine (0.17 mL, 1.40 mmol, 8.0 eq.). The vial was sealed and put on heating block: 170 °C, 15 h. After cooling, the mixture was diluted in EtOAc (15.0 mL) and organic layer was washed with a mixture of saturated NaCl / H₂O (75/25, 3x8.0 mL). Organic layer was dried over MgSO₄ and concentrated then the residue was purified by flash chromatography with DCM/MeOH (95/5 to 93/7) as eluent to give compound **(20)** (0.010 g, 17 %) as a white solid.

### Example 3.22: Synthesis of N5-(2-ethylbutyl)-3-isopropyl-N7-(pyridazin-3-yl)pyrazolo[1,5-a]pyrimidine-5,7-diamine (21)

In a sealed tube 2 - 5 mL with a stir bar was charged intermediate **(2.12)** (0.080 g, 0.25 mmol, 1 eq.), NMP (2.0 mL) and 2-ethylbutylamine (0.33 mL, 2.51 mmol, 10.0 eq.). The vial was sealed and put on heating block: 170 °C, 5 h. After cooling, the mixture was diluted in EtOAc (10.0 mL) and organic layer was washed with a mixture of saturated NaCl / H₂O (75/25, 3x5.0 mL). Organic layer was dried over MgSO₄ and concentrated then the residue was purified by flash chromatography with DCM/MeOH (100/0 to 98/2) as eluent. Impure product was purified a second time with cyclohexane/EtOAc (60/40 to 40/60) as eluent to give compound **(21)** (0.037 g, 42 %) as a white solid.

### Example 3.23: Synthesis of N5-(3-ethylpentyl)-3-isopropyl-N7-(pyridazin-3-yl)pyrazolo[1,5-a]pyrimidine-5,7-diamine (22)

In a sealed tube 2 - 5 mL with a stir bar was charged intermediate **(2.12)** (0.050 g, 0.17 mmol, 1.0 eq.), NMP (1.5 mL) and 3-ethylpentan-1-amine (0.120 g, 1.04 mmol, 6.0 eq.). The vial was sealed and put on heating block: 170 °C, 5.6 h. After cooling, the mixture was diluted in EtOAc (10.0 mL) and organic layer was washed with a mixture of saturated NaCl / H₂O (75/25, 3x5.0 mL). Organic layer was dried over MgSO₄ and concentrated then the residue was purified by flash chromatography with DCM/MeOH (98/2) as eluent. Impure product was triturated in Et₂O, filtered and dried under vacuum to give compound (22) (0.053 g, 83 %) as a white solid.

### Example 3.24: Synthesis of (S)-2-((3-isopropyl-7-(pyridazin-3-ylamino)pyrazolo[1,5-a]pyrimidin-5-yl)amino)butan-1-ol (23)

In a sealed tube 2-5 mL with a stir bar was charged intermediate **(2.12)** (0.050 g, 0.17 mmol, 1.0 eq.), NMP (0.5 mL) and (*S*)-2-aminobutan-1-ol (0.08 mL, 0.87 mmol, 5.0 eq.). The vial was sealed and put on heating block 7 h at 170 °C. After cooling, the mixture was diluted in EtOAc (15.0 mL) and organic layer was washed with a mixture of saturated NaCl / H₂O (75/25, 3x5.0 mL). Organic layer was dried over MgSO₄ and concentrated then the residue was purified by flash chromatography with DCM/MeOH (100/0 to 94/6) as eluent to give compound **(23)** (0.012 g, 20 %) as a beige solid.

### Example 3.25: Synthesis of 3-isopropyl-N5-(pentan-3-yl)-N7-(pyrimidin-4-yl)pyrazolo[1,5-a]pyrimidine-5,7-diamine (24)

In a sealed tube 2-5 mL with a stir bar was charged intermediate **(2.13)** (0.050 g, 0.17 mmol, 1.0 eq.), NMP (0.5 mL) and pentan-3-amine (0.17 mL, 1.38 mmol, 8.0 eq.). The vial was sealed and then put on heating block, 15 h at 170 °C. After cooling, the mixture was poured in EtOAc (15.0 mL) then organic layer was washed three times with saturated NaCl + H₂O (3/1, 3x8.0 mL), dried over MgSO₄, filtered and concentrated. The residue was purified by flash chromatography with DCM/MeOH (95/5 to 93/7) as eluent to give compound **(24)** (0.015 g, 26 %) as a white solid.

### Example 3.26: Synthesis of 3-isopropyl-N5-(pentan-3-yl)-N7-(pyrazin-2-yl)pyrazolo[1,5-a]pyrimidine-5,7-diamine (25)

In a sealed tube 2-5 mL with a stir bar was charged intermediate **(2.14)** (0.070 g, 0.24 mmol, 1.0 eq.), anisol (1.0 mL) and pentan-3-amine (0.28 mL, 2.42 mmol, 10.0 eq.). The vial was sealed and put on heating block : 170 °C, 17 h. After cooling, the mixture was diluted in THF, the precipitate was filtered off, washed with THF and the filtrate was concentrated. The residue was purified by flash chromatography with DCM/EtOAc (95/5 to 85/15) as eluent to give compound **(25)** (0.143 g, 76 %) as a white solid.

### Example 3.27: Synthesis of 3-isopropyl-N5-(pentan-3-yl)-N7-(pyrimidin-5-yl)pyrazolo[1,5-a]pyrimidine-5,7-diamine (26)

In a sealed tube 2-5 mL with a stir bar was charged intermediate **(2.15)** (0.070 g, 0.24 mmol, 1.0 eq.), anisol (1.0 mL) and pentan-3-amine (0.28 mL, 2.42 mmol, 10.0 eq.). The vial was sealed and put on heating block : 170 °C, 17 h. After cooling, the mixture was diluted in THF, the precipitate was filtered off, washed with THF and the filtrate was concentrated. The residue was purified by flash chromatography with DCM/EtOAc (95/5 to 85/15) as eluent to give compound **(26)** (0.021g, 26 %) as a beige solid.

### Example 3.28: Synthesis of 3-isopropyl-N7-(6-methoxypyridin-2-yl)-N5-(pentan-3-yl)pyrazolo[1,5-a]pyrimidine-5,7-diamine (27)

In a sealed tube 2-5 mL with a stir bar was charged intermediate **(2.16)** (0.100 g, 0.32 mmol, 1.0 eq.), NMP (1.0 mL) and pentan-3-amine (0.29 mL, 2.52 mmol, 8.0 eq.). The vial was sealed and then put on heating block, 16 h at 160 °C. After cooling, the mixture was poured in EtOAc (10.0 mL) then organic layer was washed three times with saturated NaCl + H₂O (3/1, 3x5.0 mL), dried over MgSO₄, filtered and concentrated. The residue was purified by flash chromatography with DCM/MeOH (99/1 to 97/3) as eluent to give compound (27) (0.035 g, 30 %) as a white solid.

### Example 3.29: Synthesis of 3-isopropyl-N7-(5-methoxypyridin-2-yl)-N5-(pentan-3-yl)pyrazolo[1,5-a]pyrimidine-5,7-diamine (28)

In a sealed tube 2-5 mL with a stir bar was charged intermediate **(2.17)** (0.075 g, 0.24 mmol, 1.0 eq.), NMP (1.0 mL) and pentan-3-amine (0.22 mL, 1.89 mmol, 10.0 eq.). The vial was sealed and put on heating block 16 h at 160 °C. After cooling, the mixture was diluted in EtOAc (10.0 mL) and organic layer was washed with a mixture of saturated NaCl / H₂O (75/25, 3x5.0 mL), dried over MgSO₄, filtered and concentrated then the residue was purified by flash chromatography with DCM/MeOH (99/1 to 95/5) as eluent to give compound **(28)** (0.050 g, 57 %) as a white solid.

### Example 3.30: Synthesis of 3-isopropyl-N7-(6-methoxypyridin-3-yl)-N5-(pentan-3-yl)pyrazolo[1,5-a]pyrimidine-5,7-diamine (29)

In a sealed tube 2-5 mL with a stir bar was charged intermediate **(2.18)** (0.134 g, 0.42 mmol, 1.0 eq.), NMP (3.0 mL) and pentan-3-amine (0.49 mL, 4.22 mmol, 10.0 eq.). The vial was sealed and put on heating block 20 h at 170 °C. After cooling, the mixture was diluted in EtOAc (10.0 mL), and organic layer was washed with a mixture of saturated NaCl / H₂O (75/25, 3x5.0 mL), dried over MgSO₄, filtered and concentrated. The residue was purified by flash chromatography with DCM/MeOH (99/1) as eluent to give compound **(29)** (0.032 g, 21 %) as a white solid.

### Example 3.31: Synthesis of 3-isopropyl-N7-(2-methoxypyrimidin-4-yl)-N5-(pentan-3-yl)pyrazolo[1,5-a]pyrimidine-5,7-diamine (30)

In a sealed tube 2-5 mL with a stir bar was charged intermediate **(2.19)** (0.100 g, 0.31 mmol, 1.0 eq.), anisol (1.0 mL) and pentan-3-amine (0.29 mL, 2.51 mmol, 8.0 eq.). The vial was sealed and put on heating block: 140 °C, 21 h. After cooling, the mixture was concentrated and the residue was directly purified by flash chromatography with DCM/EtOAc (97/3 to 67/33) as eluent to give compound **(30)** (0.035 g, 30 %) as a white solid.

### Example 3.32: Synthesis of 3-isopropyl-N7-(6-methoxypyridazin-3-yl)-N5-(pentan-3-yl)pyrazolo[1,5-a]pyrimidine-5,7-diamine (31)

In a sealed tube 2-5 mL with a stir bar was charged intermediate **(2.20)** (0.075 g, 0.24 mmol, 1.0 eq.), NMP (1.0 mL) and pentan-3-amine (0.22 mL, 1.88 mmol, 10.0 eq.). The vial was sealed and put on heating block 16 h at 160 °C. After cooling, the mixture was diluted in EtOAc (10.0 mL) and organic layer was washed with a mixture of saturated NaCl / H₂O (75/25, 3x5.0 mL), dried over MgSO₄, filtered and concentrated then the residue was purified by flash chromatography with DCM/MeOH (99/1 to 90/10) as eluent to give compound **(31)** (0.032 g, 37 %) as a white solid.

### Example 3.33: 3-isopropyl-N7-(6-methoxypyrimidin-4-yl)-N5-(pentan-3-yl)pyrazolo[1,5-a]pyrimidine-5,7-diamine (32)

In a sealed tube 2-5 mL with a stir bar was charged intermediate **(2.21)** (0.050 g, 0.18 mmol, 1.0 eq.), anisol (1.0 mL) and pentan-3-amine (0.20 mL, 1.75 mmol, 10.0 eq.). The vial was sealed and put on heating block: 160 °C, 21 h. After cooling, the mixture was diluted in THF, the precipitate was filtered off, washed with THF and the filtrate was concentrated. The residue was purified by flash chromatography with cyclohexane/DCM/EtOAc (34/33/33) as eluent to give compound **(32)** (0.032 g, 46 %) as a white solid.

### Example 3.34: Synthesis of N5-(2-ethylbutyl)-3-isopropyl-N7-(pyridin-2-yl)pyrazolo[1,5-a]pyrimidine-5,7-diamine (35)

In a sealed tube 2-5 mL with a stir bar was charged intermediate **(2.10)** (0.080 g, 0.28 mmol, 1.0 eq.), anisol (1.0 mL) and 2-ethylbutan-1-amine (0.15 mL, 1.11 mmol, 4.0 eq.). The vial was sealed and put on heating block: 140 °C, 16 h. After cooling, the mixture was concentrated and the residue was directly purified by flash chromatography with cyclohexane/DCM/EtOAc (10/89/1 to 10/88/2) as eluent to give compound (35) (0.040 g, 41 %) as a white solid.

### Example 3.35: Synthesis of N5-(2-ethylbutyl)-3-isopropyl-N7-(pyridin-3-yl)pyrazolo[1,5-a]pyrimidine-5,7-diamine (36)

In a sealed tube 2-5 mL with a stir bar was charged intermediate **(2.11)** (0.080 g, 0.28 mmol, 1.0 eq.), anisol (1.0 mL) and 2-ethylbutan-1-amine (0.15 mL, 1.11 mmol, 4.0 eq.). The vial was sealed and put on heating block: 140 °C, 16 h. After cooling, the mixture was concentrated and the residue was directly purified by flash chromatography with cyclohexane/DCM/EtOAc (10/89/1 to 10/88/2) as eluent to give compound (36) (0.030 g, 31 %) as a white solid.

### Example 3.36: Synthesis of N5-(2-ethylbutyl)-3-isopropyl-N7-(2-methoxypyrimidin-4-yl)pyrazolo[1,5-a]pyrimidine-5,7-diamine (37)

In a sealed tube 2-5 mL with a stir bar was charged intermediate **(2.19)** (0.050 g, 0.16 mmol, 1.0 eq.), anisol (1.0 mL) and 2-ethylbutan-1-amine (0.09 mL, 0.63 mmol, 4.0 eq.). The vial was sealed and put on heating block: 140 °C, 21 h. After cooling, the mixture was concentrated and the residue was directly purified by flash chromatography with DCM/EtOAc (95/5 to 80/20) as eluent to give compound (37) (0.020 g, 33 %) as a white solid.

### Example 3.37: Synthesis of N5-(2-ethylbutyl)-3-isopropyl-N7-(6-methoxypyridazin-3-yl)pyrazolo[1,5-a]pyrimidine-5,7-diamine (38)

In a sealed tube 2-5 mL with a stir bar was charged intermediate **(2.20)** (0.100 g, 0.31 mmol, 1.0 eq.), anisol (1.0 mL) and 2-ethylbutan-1-amine (0.17 mL, 1.26 mmol, 4.0 eq.). The vial was sealed and put on heating block: 140 °C, 21 h. After cooling, the mixture was concentrated and the residue was directly purified by flash chromatography with DCM/EtOAc (95/5 to 85/15) as eluent to give compound **(38)** (0.048 g, 41 %) as a white solid.

### Example 3.38: Synthesis of 3-cyclopropyl-N5-(pentan-3-yl)-N7-(2-(pyridin-2-yl)ethyl)pyrazolo[1,5-a]pyrimidine-5,7-diamine (39)

In a sealed tube 2-5 mL with a stir bar was charged intermediate **(2.22)** (0.060 g, 0.19 mmol, 1.0 eq.), anisol (1.0 mL) and pentan-3-amine (0.22 mL, 1.91 mmol, 10.0 eq.). The vial was sealed and put on heating block: 170 °C, 17 h. After cooling, the mixture was diluted in THF, the precipitate was filtered off, washed with THF and the filtrate was concentrated. The residue was purified by flash chromatography with DCM/MeOH (99/1 to 95/5) as eluent to give compound **(39)** (0.006 g, 9 %) as a brown oil.

### Example 3.39: Synthesis of 3-cyclopropyl-N5-(pentan-3-yl)-N7-(pyridin-3-yl)pyrazolo[1,5-a]pyrimidine-5,7-diamine (40)

In a sealed tube 2-5 mL with a stir bar was charged intermediate **(2.23)** (0.050 g, 0.18 mmol, 1.0 eq.), anisol (1.0 mL) and pentan-3-amine (0.20 mL, 1.75 mmol, 10.0 eq.). The vial was sealed and put on heating block: 170 °C, 17 h. After cooling, the mixture was diluted in THF, the precipitate was filtered off, washed with THF and the filtrate was concentrated. The residue was purified by flash chromatography with DCM/MeOH (99/1 to 95/5) as eluent to give compound **(40)** (0.020 g, 34 %) as a white solid.

### Example 3.40: Synthesis of 3-cyclopropyl-N5-(pentan-3-yl)-N7-(pyridin-2-yl)pyrazolo[1,5-a]pyrimidine-5,7-diamine (41)

In a sealed tube 2-5 mL with a stir bar was charged intermediate **(2.24)** (0.060 g, 0.21 mmol, 1.0 eq.), anisol (1.0 mL) and pentan-3-amine (0.24 mL, 2.10 mmol, 10.0 eq.). The vial was sealed and put on heating block: 170 °C, 21 h. After cooling, the mixture was diluted in THF, the precipitate was filtered off, washed with THF and the filtrate was concentrated. The residue was purified by flash chromatography with DCM/EtOAc (95/5 to 85/15) as eluent to give compound **(41)** (0.015 g, 21 %) as a white solid.

### Example 3.41: Synthesis of 3-cyclopropyl-N5-(pentan-3-yl)-N7-(pyrimidin-4-yl)pyrazolo[1,5-a]pyrimidine-5,7-diamine (42)

In a sealed tube 2-5 mL with a stir bar was charged intermediate (2.25) (0.042 g, 0.15 mmol, 1.0 eq.), NMP (1.0 mL) and pentan-3-amine (0.17 mL, 1.46 mmol, 10.0 eq.). The vial was sealed and put on heating block 19.5 h at 170 °C. After cooling, the mixture was diluted in EtOAc (10.0 mL) and organic layer was washed with a mixture of saturated NaCl / H₂O (75/25, 3x5.0 mL). Organic layer was dried over MgSO₄ and concentrated then the residue was purified by flash chromatography with cyclohexane/EtOAc (70/30 to 50/50) as eluent to give compound **(42)** (0.041 g, 83 %) as a yellow solid.

### Example 3.42: Synthesis of 3-cyclopropyl-N5-(pentan-3-yl)-N7-(pyridazin-3-yl)pyrazolo[1,5-a]pyrimidine-5,7-diamine (43)

In a sealed tube 2-5 mL with a stir bar was charged intermediate **(2.26)** (0.050 g, 0.17 mmol, 1.0 eq.), NMP (0.5 mL) and pentan-3-amine (0.20 mL, 1.74 mmol, 10.0 eq.). The vial was sealed and put on heating block 22 h at 170 °C. After cooling, the mixture was diluted in EtOAc (10.0 mL) and organic layer was washed with a mixture of saturated NaCl / H₂O (75/25, 3x5.0 mL). Organic layer was dried over MgSO₄ and concentrated then the residue was purified by flash chromatography with DCM/MeOH (100/0 to 97/3) as eluent to give compound **(43)** (0.040 g, 68 %) as a brown yellow solid.

### Example 3.43: Synthesis of 3-cyclopropyl-N5-(pentan-3-yl)-N7-(pyrazin-2-yl)pyrazolo[1,5-a]pyrimidine-5,7-diamine (44)

In a sealed tube 2-5 mL with a stir bar was charged intermediate **(2.27)** (0.058 g, 0.20 mmol, 1.0 eq.), NMP (1.5 mL) and pentan-3-amine (0.24 mL, 2.02 mmol, 10.0 eq.). The vial was sealed and put on heating block: 170 °C, 24 h. After cooling, the mixture was diluted in EtOAc (10.0 mL) and organic layer was washed with a mixture of saturated NaCl / H₂O (3/1, 3x5.0 mL). Organic layer was dried over MgSO₄ and concentrated then the residue was purified by flash chromatography with DCM/MeOH (100/0 to 96/4) as eluent to give compound **(44)** (0.056 g, 82 %) as an orange solid.

### Example 3.44: Synthesis of 3-cyclopropyl-N5-(pentan-3-yl)-N7-(pyrimidin-5-yl)pyrazolo[1,5-a]pyrimidine-5,7-diamine (45)

In a sealed tube 2-5 mL with a stir bar was charged intermediate (2.28) (0.050 g, 0.18 mmol, 1.0 eq.), anisol (1.0 mL) and pentan-3-amine (0.20 mL, 1.75 mmol, 10.0 eq.). The vial was sealed and put on heating block: 170 °C, 17 h. After cooling, the mixture was diluted in THF, the precipitate was filtered off, washed with THF and the filtrate was concentrated. The residue was purified by flash chromatography with DCM/MeOH (99/1 to 95/5) as eluent to give compound **(45)** (0.030 g, 39 %) as a white solid.

### Example 3.45: Synthesis of (S)-2-((3-cyclopropyl-7-(pyridazin-3-ylamino)pyrazolo[1,5-a]pyrimidin-5-yl)amino)butan-1-ol (46)

In a sealed tube 2-5 mL with a stir bar was charged intermediate **(2.26)** (0.100 g, 0.35 mmol, 1 eq.), anisol (1.0 mL) and (*S*)-2-aminobutan-1-ol (0.17 mL, 1.74 mmol, 5.0 eq.). The vial was sealed and put on heating block: 170 °C, 16 h. After cooling, the solvent was removed and the residue was purified by flash chromatography with DCM/MeOH (99/1 to 94/6) as eluent to give compound **(46)** (0.005 g, 4 %) as a grey solid.

### Example 3.46: Synthesis of 3-cyclopropyl-N7-(6-methoxypyridin-2-yl)-N5-(pentan-3-yl)pyrazolo[1,5-a]pyrimidine-5,7-diamine (47)

In a sealed tube 2-5 mL with a stir bar was charged intermediate **(2.29)** (0.046 g, 0.15 mmol, 1.0 eq.), NMP (0.5 mL) and pentan-3-amine (0.17 mL, 1.46 mmol, 10.0 eq.). The vial was sealed and put on heating block: 170 °C, 24 h. After cooling, the mixture was diluted in EtOAc (10.0 mL) and organic layer was washed with a mixture of saturated NaCl / H₂O (3/1, 3x5.0 mL). Organic layer was dried over MgSO₄ and concentrated then the residue was purified by flash chromatography with DCM/MeOH (100/0 to 96/4) as eluent to give compound **(47)** (0.032 g, 60 %) as a white oil/solid.

### Example 3.47: Synthesis of 3-cyclopropyl-N7-(6-methoxypyridazin-3-yl)-N5-(pentan-3-yl)pyrazolo[1,5-a]pyrimidine-5,7-diamine (48)

In a sealed tube 2-5 mL with a stir bar was charged intermediate (2.30) (0.047 g, 0.15 mmol, 1.0 eq.), NMP (2.0 mL) and pentan-3-amine (0.17 mL, 1.48 mmol, 10.0 eq.). The vial was sealed and put on heating block: 170 °C, 20 h. After cooling, the mixture was diluted in EtOAc (10.0 mL) and organic layer was washed with a mixture of saturated NaCl / H₂O (3/1, 3x5.0 mL). Organic layer was dried over MgSO₄ and concentrated then the residue was purified by flash chromatography with cyclohexane/EtOAc (70/30 to 50/50) as eluent to give compound **(48)** (0.028 g, 51 %) as a white solid.

### Example 3.48: Synthesis of 3-cyclopropyl-N5-(2-ethylbutyl)-N7-(pyridazin-3-yl)pyrazolo[1,5-a]pyrimidine-5,7-diamine (50)

In a sealed tube 2-5 mL with a stir bar was charged intermediate **(2.26)** (0.111 g, 0.39 mmol, 1.0 eq.), NMP (2.0 mL), 2-ethylbutan-1-amine hydrochloride (0.533 g, 3.87 mmol, 10.0 eq.) and DiPEA (0.74 mL, 4.26 mmol, 11.0 eq.). The vial was sealed and put on heating block: 170 °C, 24 h. After cooling, the mixture was diluted in EtOAc (10.0 mL) and organic layer was washed with a mixture of saturated NaCl/ H₂O (3/1, 3x5.0 mL). Organic layer was dried over MgSO₄ and concentrated then the residue was purified by flash chromatography with DCM/MeOH (100/0 à 98/2) as eluent to give compound **(50)** (0.086 g, 63 %) as a white solid.

### Example 3.49: Synthesis of 3-cyclopropyl-N5-(2-ethylbutyl)-N7-(6-methoxypyridazin-3-yl)pyrazolo[1,5-a]pyrimidine-5,7-diamine (51)

In a sealed tube 2-5 mL with a stir bar was charged intermediate **(2.30)** (0.144 g, 0.46 mmol, 1.0 eq.), NMP (2.0 mL) and 2-ethylbutan-1-amine (0.59 mL, 4.55 mmol, 10.0 eq.). The vial was sealed and put on heating block: 170 °C, 24 h. After cooling, the mixture was diluted in EtOAc (10.0 mL) and organic layer was washed with a mixture of saturated NaCl / H₂O (3/1, 3x5.0 mL). Organic layer was dried over MgSO₄ and concentrated then the residue was purified by flash chromatography with DCM/MeOH (100/0 à 98/2) as eluent to give compound **(51)** (0.016 g, 9 %) as a red solid.

### Example 3.50: Synthesis of 3-cyclopropyl-N5-(2-ethylbutyl)-N7-(2-methoxypyrimidin-4-yl)pyrazolo[1,5-a]pyrimidine-5,7-diamine (52)

In a sealed tube 2-5 mL with a stir bar was charged intermediate **(2.31)** (0.104 g, 0.33 mmol, 1.0 eq.), dioxane (4.0 mL) and 2-ethylbutan-1-amine (0.44 mL, 3.28 mmol, 10.0 eq.). The vial was sealed and put on heating block: 110 °C, 22 h. After cooling, the mixture was diluted in EtOAc (10.0 mL) and organic layer was washed with a mixture of saturated NaCl / H₂O (3/1, 3x5.0 mL). Organic layer was dried over MgSO₄ and concentrated then the residue was purified by flash chromatography with cyclohexane/EtOAc (60/40 to 40/60). Impur product was purified a second time by flash chromatography with DCM/MeOH (100/0 to 98/2) as eluent to give compound **(52)** (0.037 g, 42 %) as a yellow solid.

### Example 3.51: Synthesis of 3-isopropyl-N7-(4-methoxybenzyl)-N5-(pentan-3-yl)pyrazolo[1,5-a]pyrimidine-5,7-diamine (53)

In a sealed tube 2-5 mL with a stir bar was charged intermediate **(2.32)** (0.150 g, 0.48 mmol, 1.0 eq.), NMP (0.5 mL) and corresponding amine (0.39 mL, 3.33 mmol, 7.0 eq.). The vial was sealed and then put on heating block: 170 °C, 20 h. After cooling, the mixture was poured in EtOAc (30.0 mL) then organic layer was washed three times with saturated NaCl + H₂O (3/1, 3x8.0 mL), dried over MgSO₄, filtered and concentrated. The residue was purified by flash chromatography with cyclohexane/DCM/EtOAc (50/48/2 to 42/48/10) as eluent to give compound **(53)** (1.600 g, 82 %) as a white solid.

### Example 4: Synthesis of pyrazolo[1,5-a]pyrimidin-5,7-diamine derivatives, as illustrated in step 5 of scheme 1

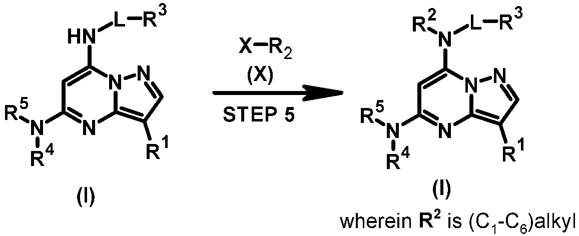

### Example 4.1: Synthesis of 3-cyclopropyl-N7-methyl-N5-(pentan-3-yl)-N7-(pyridazin-3-yl)pyrazolo[1,5-a]pyrimidine-5,7-diamine (49)

To a solution of **(43)** (0.080 g, 0.24 mmol, 1.0 eq.) in dry THF (3.0 mL) was added NaH 60% suspended in mineral oil (0.014 g, 0.36 mmol, 1.5 eq.). The mixture was stirred for 5 min before addition of MeI (0.019 mL, 0.31 mmol, 1.3 eq.) at room temperature. After completion, it was neutralized with saturated NH₄Cl (1.0 mL) and H₂O (0.5 mL). The aqueous layer was extracted with EtOAc (3 × 2.5 mL) and the combined organic phases were dried over MgSO₄, filtered and concentrated. The residue was purified by flash chromatography with DCM/MeOH (100/0 to 96/4) as eluent to give compound **(49)** (0.021 g, 25 %) as a yellow solid.

### Example 5: Synthesis of pyrazolo[1,5-a]pyrimidin-5,7-diamine derivatives, as illustrated in scheme 2

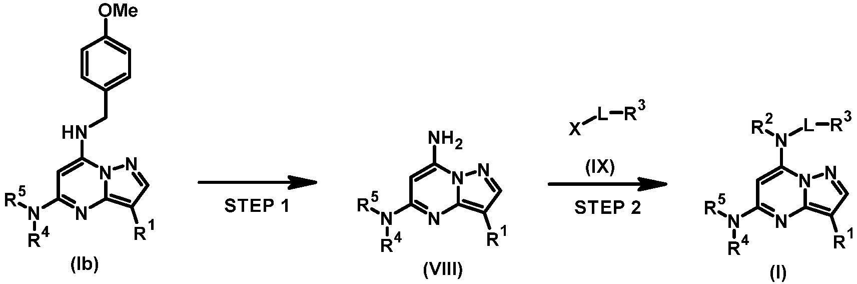

### Example 5.1: Synthesis of (3-isopropyl-N5-(pentan-3-yl)pyrazolo[1,5-a]pyrimidine-5,7-diamine (5.1) as illustrated in step 1 of scheme 2

In a microwave vial 10 - 20 mL with a stir bar was charged compound **(53)** (1.100 g, 2.88 mmol, 1.0 eq.), DCM (6.0 mL) and TFA (6.0 mL). The vial was closed and then put in microwave reactor: 100 °C, 45 min. After cooling, the solvent was removed and the residue was solubilized in DCM and neutralised with NH₃ 7N in MeOH. The mixture was concentrated and the residue was purified by flash chromatography with cyclohexane/DCM/MeOH (30/68/2 then 30/67/3) as eluent. The protonated compound was taken up in Et₂O, washed with saturated NaHCO₃ then aqueous layer was extracted twice with Et₂O (2x10.0 mL) and organic layers were combined, dried over MgSO4, filtered, concentrated to give intermediate **(5.1)** (0.675 g, 90 %) as a white solid.
**¹H NMR (400 MHz, DMSO-*d*₆) δ :** 0.85 (t, *J* = 7.4 Hz, 6H, 2xCH_{3-CH2}), 1.24 (d, *J* = 6.8 Hz, 6H, 2xCH_{3 iPr}), 1.36 - 1.61 (m, 4H, 2xCH_{2-CH3}), 2.92 (hept, *J* = 7.0 Hz, 1H, H_{iPr}), 3.78 (s, 1H, H_{iPent}), 5.29 (s, 1H, H_{Ar}), 6.30 (d, *J* = 8.2 Hz, 1H, NH_{iPent}), 6.71 (s, 2H, NH₂), 7.53 (s, 1H, H_{Ar}).
**¹³C NMR (101 MHz, DMSO-*d₆*) δ :** 10.5 (2xCH_{3-CH2}), 23.0 (2xCH_{3 iPr}), 23.5 (CH_{iPr}), 26.7 (2xCH_{2-CH3}), 52.2 (CH_{iPent}), 73.9 (CH_{Ar}), 109.8 (C_{q}), 139.6 (CH_{Ar}), 145.8 (C_{q}), 146.9 (C_{q}), 156.8 (C_{q}).
**MS (ESI+) : m/z calcd for C₁₄H₂₃N₅ :** 262.38 [M+H]+, **found** : 262.30.

### Example 5.2: Synthesis of N7-(6-chloropyridazin-3-yl)-3-isopropyl-N5-(pentan-3-yl)pyrazolo[1,5-a]pyrimidine-5,7-diamine(33) as illustrated in step 2 of scheme 2

Under inert gas, to a solution of intermediate **(5.1)** (0.050 g, 0.19 mmol, 1.0 eq.) in dry THF (2.0 mL), NaH 60% suspended in mineral oil (0.015 g, 0.38 mmol, 2.0 eq.) was added. The mixture was stirred 5 min at room temperature then 2,4-dichloropyrimidine (0.040 g, 0.27 mmol, 1.4 eq.) was added and stirred 5 h at room temperature. After completion, saturated NH₄Cl (0.5 mL), water (0.2 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/MeOH (100/0 to 97/3) as eluent. Impur product was purified a second time with Cyclohexane/EtOAc (75/25) as eluent to give compound **(33)** (0.006 g, 8 %) as a white solid.

### Example 5.3: Synthesis of N7-(2-chloropyrimidin-4-yl)-3-isopropyl-N5-(pentan-3-yl)pyrazolo[1,5-a]pyrimidine-5,7-diamine (34) as illustrated in step 2 of scheme 2

Under inert gas, to a solution of intermediate **(5.1)** (0.050 g, 0.19 mmol, 1.0 eq.) in dry THF (2.0 mL), NaH 60% suspended in mineral oil (0.015 g, 0.38 mmol, 2.0 eq.) was added. The mixture was stirred 5 min at room temperature then 2,4-dichloropyrimidine (0.040 g, 0.27 mmol, 1.4 eq.) was added and stirred 5 h at room temperature. After completion, saturated NH₄Cl (0.5 mL), water (0.2 mL) were added then the mixture was vigorously stirred 5 min. Layers were separated and aqueous layer was extracted twice with EtOAc (2x3.0 mL). Organic layers were combined, dried over MgSO₄, filtered, concentrated and the residue was purified by flash chromatography with DCM/MeOH (100/0 to 97/3) as eluent. Impur product was purified a second time with Cyclohexane/EtOAc (75/25) as eluent to give compound **(34)** (0.031 g, 43 %) as a white solid.

### Example 5.4: Synthesis of N-(3-isopropyl-5-(pentan-3-ylamino)pyrazolo[1,5-a]pyrimidin-7-yl)-2-phenylacetamide (54) as illustrated in step 2 of scheme 2

Under inert atmosphere, to a solution of intermediate **(5.1)** (0.050 g, 0.19 mmol, 1.0 eq.) in dry THF (2.0 mL), NaH 60% suspended in mineral oil (0.015 g, 38 mmol, 2.0 eq.) was added and the mixture was stirred 10 min at room temperature. 2-phenylacetyl chloride (0.04 mL, 0.27 mmol, 1.4 eq.) was added dropwise then the mixture was stirred 5 h at room temperature. After completion, saturated NH₄Cl (0.5 mL), water (0.2 mL) were added and the mixture was stirred vigorously 5 min. Layers were separated, aqueous layer was extracted twice with EtOAc (2x 3.0 mL) then organic layers were combined, dried over MgSO₄, filtered and concentrated. The residue was purified by flash chromatography with Cyclohexane/EtOAc (85/15) as eluent to give compound **(54)** (0.025 g, 34 %) as a brown oil.

### Example 5.5: Synthesis of N-(3-isopropyl-5-(pentan-3-ylamino)pyrazolo[1,5-a]pyrimidin-7-yl)-2-(pyridin-2-yl)acetamide (55) as illustrated in step 2 of scheme 2

To a solution of intermediate **(5.1)** (0.100 g, 0.38 mmol, 1.0 eq.) in DMF (1.5 mL), 2-(pyridin-2-yl)acetic acid hydrochloride (0.077 g, 0.42 mmol, 1.1 eq.), DiPEA (0.18 mL, 1.03 mmol, 2.7 eq.) and HATU (0.267 g, 0.69 mmol, 1.8 eq.) were added and the mixture was stirred 24 h at 60 °C. After cooling, the mixture was poured onto saturated NaHCO₃ (20.0 mL) then EtOAc (15.0 mL) was added and the biphasic mixture was vigorously stirred 5 min. Layers were separated, aqueous layer was extracted twice with EtOAc (2x 10.0 mL) then organic layers were combined, washed with brine, dried over MgSO₄, filtered and concentrated. The residue was purified by flash chromatography with DCM/EtOAc (95/5 then 80/20) as eluent to give compound **(55)** (0.021 g, 14 %) as a yellow solid.

### Example 5.6: Synthesis of N-(3-isopropyl-5-(pentan-3-ylamino)pyrazolo[1,5-a]pyrimidin-7-yl)-2-(pyridin-3-yl)acetamide (56) as illustrated in step 2 of scheme 2

Under inert atmosphere, to a solution of intermediate **(5.1)** (0.080 g, 0.31 mmol, 1.0 eq.) in dry THF (5.0 mL), NaH 60% suspended in mineral oil (0.037 g, 0.92 mmol, 3.0 eq.) was added and the mixture was stirred 5 min at room temperature. 2-(pyridin-3-yl)acetyl chloride hydrochloride (0.076 g, 0.40 mmol, 1.3 eq.) was added then the mixture was stirred 5 h at room temperature. After completion, saturated NH₄Cl (1.0 mL), water (0.5 mL) and EtOAc (3.0 mL) were added and the mixture was stirred vigorously 5 min. Layers were separated, aqueous layer was extracted twice with EtOAc (2x 2.0 mL) then organic layers were combined, dried over MgSO₄, filtered and concentrated. The residue was purified by flash chromatography with DCM/EtOAc (90/10 to 10/90) as eluent to give compound **(56)** (0.032 g, 27 %) as a white solid.

### Example 5.7: Synthesis of N-(3-isopropyl-5-(pentan-3-ylamino)pyrazolo[1,5-a]pyrimidin-7-yl)-2-(pyridin-4-yl)acetamide (57) as illustrated in step 2 of scheme 2

To a solution of intermediate **(5.1)** (0.100 g, 0.38 mmol, 1.0 eq.) in DMF (1.5 mL), 2-(pyridin-4-yl)acetic acid hydrochloride (0.077 g, 0.42 mmol, 1.1 eq.), DiPEA (0.18 mL, 1.03 mmol, 2.7 eq.) and HATU (0.267 g, 0.69 mmol, 1.8 eq.) were added and the mixture was stirred 24 h at 60 °C. After cooling, the mixture was poured onto saturated NaHCO₃ (20.0 mL) then EtOAc (15.0 mL) was added and the biphasic mixture was vigorously stirred 5 min. Layers were separated, aqueous layer was extracted twice with EtOAc (2x 10.0 mL) then organic layers were combined, washed with brine, dried over MgSO₄, filtered and concentrated. The residue was purified by flash chromatography with DCM/EtOAc (95/5 then 80/20) as eluent to give compound **(57)** (0.020 g, 14 %) as a light yellow solid.

### Example 6: Biological Activity:ADORA3 inhibition potency

### MATERIAL AND METHODS

### Adenosine receptors antagonism assay (Eurofins)

The compounds of formula (I) were tested as potential antagonists on human recombinant A₃AR (expressed in HEK-293 cells) in an assay based on the use of an agonist radioligand, [¹²⁵I]-AB-MECA (0.15 nM) and 1 µM IB-MECA. Assays were carried out at r.t. for 120 min. Compounds were initially tested at three concentrations (0.01, 0.1, 1 µM). Active compounds were tested in duplicates in 8-point dose-response curves. IC₅₀ values, which is the concentration causing a half-maximal inhibition of control specific binding, and Hill coefficients (nH) were determined by non-linear regression analysis of the competition curves generated with mean replicate values using Hill equation curve fitting. The inhibition constants (Ki) were calculated using the Cheng Prusoff equation. IB-MECA was used as a positive control (IC₅₀: 0.215 nM; Ki: 0.130 nM).

### RESULTS

The A₃AR inhibition potency classification was done according to the following ranges of IC₅₀ values:
Some compounds have an IC₅₀ being inferior or equal to 100 nM and superior or equal to 10 nM. These compounds correspond to the below-reported class **A.** Some compounds have an IC₅₀ inferior to 10 nM and superior or equal to 1 nM. These compounds correspond to the below-reported class **B.** Some compounds have an IC₅₀ inferior to 1 nM. These compounds correspond to the below-reported class **C.** Said letters **A** to **C** were used to quote the activity/efficacy of the compounds of the invention in the following **Table 3.**

**Table 3: A₃AR inhibitory activities of the compounds of formula (I). IC₅₀ values were calculated from dose-response curves. Compounds were further classified in terms of efficacy on A₃AR (increasing potency range: A to C).**

| **Cpd N°** | **Potency Class A₃AR** |
|---|---|
| **1** | **A** |
| **2** | **B** |
| **3** | **B** |
| **4** | **B** |
| **5** | **C** |
| **6** | **C** |
| **7** | **C** |
| **8** | **C** |
| **9** | **C** |
| **10** | **C** |
| **11** | **B** |
| **12** | **B** |
| **13** | **C** |
| **14** | **B** |
| **15** | **B** |
| **16** | **C** |
| **17** | **B** |
| **18** | **C** |
| **19** | **C** |
| **20** | **C** |
| **21** | **C** |
| **22** | **C** |
| **23** | **C** |
| **24** | **C** |
| **25** | **C** |
| **26** | **C** |
| **27** | **C** |
| **28** | **C** |
| **29** | **C** |
| **30** | **C** |
| **31** | **C** |
| **32** | **C** |
| **33** | **C** |
| **34** | **C** |
| **35** | **B** |
| **36** | **C** |
| **37** | **C** |
| **38** | **C** |
| **39** | **C** |
| **40** | **C** |
| **41** | **C** |
| **42** | **C** |
| **43** | **C** |
| **44** | **C** |
| **45** | **C** |
| **46** | **C** |
| **47** | **C** |
| **48** | **C** |
| **49** | **A** |
| **50** | **C** |
| **51** | **C** |
| **52** | **C** |
| **53** | **B** |
| **54** | **C** |
| **55** | **C** |
| **56** | **C** |
| **57** | **C** |
| **58** | **A** |

### CONCLUSION

Based on the previous results, it can be concluded that the compounds of formula (I) exert an inhibitory activity on A₃AR and thus are useful in the treatment of pathologies mediated by A₃AR. The most potent inhibitory activity on A₃AR is displayed by compounds (1) to (58) in particular compounds (2) to (48) and (50) to (57) and more particularly compounds (5) to (10), (13), (16), (18) to (34), (36) to (48), (50) to (52) and (54) to (57).

The present invention further relates to a pharmaceutical composition comprising at least one compound of formula (I) as defined above or any of its pharmaceutically acceptable salts, or at least any of compounds (1) to (58) as defined above or any of their pharmaceutically acceptable salts, and also at least one pharmaceutically acceptable excipient.

Pharmaceutical compositions of the invention can contain one or more compound(s) of the invention in any form described herein.

Still a further object of the present invention consists of the use of at least one compound of formula (I) as defined above, and compounds (1) to (58) as defined above, or one of their pharmaceutically acceptable salts according to the present invention for preparing a drug for preventing and/or treating diseases associated with A₃AR. According to a particular embodiment, the treatment is continuous or non-continuous.

A "continuous treatment" means a long-term treatment which can be implemented with various administration frequencies, such as once every day, every three days, once a week, or once every two weeks or once every month or by transdermal patch delivery.

According to one embodiment, the compound of formula (I), or any of its pharmaceutically acceptable salts, is administered at a dose varying from 0.1 to 1000 mg. in particular varying from 1 to 500 mg, or for example varying from 5 to 100 mg.

Another object of the invention relates to a therapeutic method for the treatment and/or for the prevention of diseases associated with A₃AR, and in particular for the treatment and/or for the prevention of ulcerative colitis, atopic dermatitis, glaucoma, asthma, liver fibrosis, kidney diseases in particular kidney fibrosis, nephropathy, acute kidney injury (AKI), chronic kidney disease (CKD) more particularly induced by ischemia and/or reperfusion injury, toxic nephropathy or myoglobinuria, diabetic kidney disease, kidney diseases induced by nephrotoxicity caused by therapeutic drugs, atherosclerosis, hypercholestemia, partial or a complete hearing loss induced by noise, acoustic trauma or ototoxic agents or conditions, , dementia, in particular vascular dementia, and cancers developing under hypoxic conditions, in particular glioblastoma, consisting in administering to a patient in need thereof of a compound of formula (I) or any of its pharmaceutically acceptable salt or of at least any of compounds (1) to (58) as defined above, of any of their pharamecutically acceptable salt.

According to one embodiment, the invention relates to a therapeutic method for the treatment and/or for the prevention of hearing loss, in particular induced by ototoxic agents, as well as in the treatment and/or in the prevention of kidney diseases, in particular induced by nephrotoxicity, consisting in administering to a patient in need thereof of a compound of formula (I) or any of its pharmaceutically acceptable salt or of at least any of compounds (1) to (58) as defined above, of any of their pharamecutically acceptable salt.

According to one embodiment, the invention relates to a therapeutic method for the treatment and/or for the prevention of partial or a complete hearing loss, in particular induced by noise, acoustic trauma or ototoxic agents or conditions, more particularly induced by ototoxic agents or conditions, consisting in administering to a patient in need thereof of a compound of formula (I) or any of its pharmaceutically acceptable salt or of at least any of compounds (1) to (58) as defined above, of any of their pharamecutically acceptable salt.

According to one embodiment, the invention relates to a therapeutic method for the treatment and/or for the prevention of partial or a complete hearing loss induced by ototoxic agents or conditions as described hereinafter, consisting in administering to a patient in need thereof of a compound of formula (I) or any of its pharmaceutically acceptable salt or of at least any of compounds (1) to (58) as defined above, of any of their pharamecutically acceptable salt.

As ototoxic agents, i.e. causing hear loss, the following may be cited:
- solvents, such as polyethylene glycol, propylene glycol or benzalkonium chloride;
- antibiotics, in particular topical and/or systemic antibiotics, more particularly aminoglycosides, macrolides or phenicol antibiotics, such as gentamycin, neomycin, tobramycin, kanamycin, nystatin, polymyxin B, amphotericin B, bacitracin or chloramphenicol;
- anticancer drugs, in particular platinum-based anticancer drugs, such as cisplatin or carboplatin,
- antiseptics, in particular acetic acid, alcohols such as ethanol, chlorhexidine, cresylate, gentian violet or povidone iodine;
- nonsteroidal anti-inflammatory drugs (NSAIDs), in particular salicylates, cyclodextrins, indomethacin, ibuprofen, phenylbutazone or paracetamol;
- topical combinations, in particular polymyxin/neomycin/hydrocortisone or ticarcilline/clavulanate;
- drugs for COVID-19, in particular lopinavir or ritonavir;
- antimalarial drugs, in particular quinine or chloroquine;
- cardiovascular drugs, in particular loop diuretics; or
- erectile dysfunction drugs, in particular phosphodiesterase type 5 (PDE-5) inhibitors.

According to one embodiment, the invention relates to a therapeutic method for the treatment and/or for the prevention of hearing loss being a partial or a complete hearing loss, in particular being induced by noise, acoustic trauma or ototoxic agents or conditions, more particularly platin-based anticancer drugs such as cisplatin or carboplatin, antibiotics, even more particularly macrolides or aminoglycosides such as gentamycin or neomycin, drugs for COVID-19 such as lopinavir or ritonavir, antimalarial drugs such as quinine or chloroquine, cardiovascular drugs such as loop diuretics, non-steroidal anti-inflammatory drugs such as salicylate or cyclodextrins, erectile dysfunction drugs such as phosphodiesterase type 5 inhibitors, consisting in administering to a patient in need thereof of a compound of formula (I) or any of its pharmaceutically acceptable salt or of at least any of compounds (1) to (58) as defined above, of any of their pharamecutically acceptable salt.

According to one embodiment, the invention relates to a therapeutic method for the treatment and/or for the prevention of kidney diseases, in particular selected from nephropathy, acute kidney injury (AKI), chronic kidney disease (CKD) more particularly induced by ischemia and/or reperfusion injury, toxic nephropathy or myoglobinuria, and diabetic kidney disease, or kidney diseases induced by nephrotoxicity caused by therapeutic drugs, consisting in administering to a patient in need thereof of a compound of formula (I) or any of its pharmaceutically acceptable salt or of at least any of compounds (1) to (58) as defined above, of any of their pharamecutically acceptable salt.

According to one embodiment, the invention relates to a therapeutic method for the treatment and/or for the prevention of kidney diseases induced by nephrotoxicity caused by therapeutic drugs as described hereinafter, consisting in administering to a patient in need thereof of a compound of formula (I) or any of its pharmaceutically acceptable salt or of at least any of compounds (1) to (58) as defined above, of any of their pharamecutically acceptable salt.

As therapeutic drugs causing nephrotoxicity, the following may be cited: acyclovir, ambisome, amikacin, aminoglycosides, antibiotics, amphotericin B, captopril, carboplatin, cefotaxime, ceftazidime, cefuroxime, cephalosporins, cidofovir, ciprofloxacin, cisplatin, colistimethate, cyclosporine, dapsone, enalaprilat, enalapril, Foscarnet, gadopentetate dimeglumine, gadoxetate, ganciclovir gentamicin, ibuprofen, ifosfamide, iodixanol, iohexol, iopamidol, ioversol, ketorolac, lisinopril, lithium, mesalamine, methotrexate, nafcillin disodium, penicillins, piperacillin/tazobactam, piperacillin, rifampin, sirolimus, sulfasalazine, tacrolimus, ticarcillin/clavulanic acid, tobramycin, topiramate, valacyclovir, valganciclovir, vancomycin or zonisamide.

All combinations of doses, frequencies and treatment period are encompassed within the scope of the present invention.

Various sequences or cycles of administration respectively of the compound of formula (I) as defined herein above, a pharmaceutically acceptable salt thereof, prodrug thereof may be implemented within the framework of the present invention.

The compounds of formula (I) as defined above can be administered through any mode of administration such as, for example, intramuscular, intravenous. intranasal or oral route, transtympanic, intra-cochlear, transdermal patch, etc.

The inventive composition can further include one or more additives such as diluents, excipients, stabilizers and preservatives. Such additives are well known to those skilled in the art and are described notably in "Ullmann's Encyclopedia of Industrial Chemistry, 6th Ed." (various editors, 1989-1998, Marcel Dekker) and in "Pharmaceutical Dosage Forms and Drug Delivery Systems" (ANSEL et al, 1994, WILLIAMS & WILKINS).

The aforementioned excipients are selected according to the dosage form and the desired mode of administration.

Compositions of this invention may be administered in any manner, including, but not limited to, orally, parenterally, sublingually, transdermally, vaginally, rectally, transmucosally, topically, intranasally via inhalation, via buccal or intranasal administration, or combinations thereof. Parenteral administration includes, but is not limited to, intravenous, intra-arterial, intra-peritoneal, subcutaneous, intramuscular, intra-thecal, and intra-articular. The compositions of this invention may also be administered in the form of an implant, which allows slow release of the compositions as well as a slow controlled i.v. infusion.

For example, a compound of formula (I) according to the present invention can be present in any pharmaceutical form which is suitable for enteral or parenteral administration, in association with appropriate excipients, for example in the form of plain or coated tablets, hard gelatine, soft shell capsules and other capsules, suppositories, or drinkable, such as suspensions, syrups, or injectable solutions or suspensions.

In a particular embodiment, a compound of formula (I) according to the invention is administered orally.

Oral route of administration is in particular preferred in the prophylaxis or treatment aspect of the invention.

## Claims

1. A compound of formula (I) or any of its pharmaceutically acceptable salt 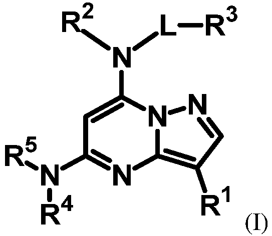 wherein
R¹ represents a hydrogen atom, a linear or branched (C₁-C₆)alkyl group optionnaly deuterated, or a (C₃-C₆)cycloalkyl group;
R² represents a hydrogen atom or a (C₁-C₆)alkyl;
R³ represents
- a linear or branched (C₁-C₆)alkyl group, optionnaly substituted by
• one phenyl group, optionally substituted by one to three substituents selected from a -OR⁶ group, a -NH₂ group, a halogen atom and a (C₁-C₆)alkyl group,
• one (C₅-C₁₀)heteroaryl group, optionally substituted by one to three substituents selected from -OR⁶ group, a -NH₂ group, a halogen atom and a (C₁-C₆)alkyl group, or
• one -OR⁶ group;
- a phenyl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom;
- a (C₅-C₆)heteroaryl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom; or
- a (C₃-C₈)cycloalkyl group, optionally substituted by one to three substituents selected from a (C₁-C₄)alkyl group, a deuterated (C₁-C₄)alkyl group, a (C₁-C₄)alkoxy group, a deuterated (C₁-C₄)alkoxy group and a halogen atom;
**L** represents a bond or a -CO- group;
**R⁴** represents
- a linear or branched (C₁-C₈)alkyl group, optionally substituted by
• one phenyl group, optionally substituted by one to three substituents selected from a (C₁-C₄)alkyl group and a (C₁-C₄)alkoxy group,
• one (C₃-C₈)cycloalkyl group, optionally substituted by one to three substituents selected from a halogen atom, a (C₁-C₄)alkyl group and a -OR⁶ group,
• one (C₅-C₆)heterocycloalkyl,
• one (C₅-C₆)heteroaryl group, optionally substituted by one to three (C₁-C₆)alkyl group, or
• one or two -OR⁶ groups;
- a (C₃-C₇)cycloalkyl group, optionally substituted by one to three substituents selected from a halogen atom, a (C₁-C₄)alkyl group and a -OR⁶ group;
- a (C₅-C₆)heteroaryl group, optionally substituted by one to three substituents selected from a halogen atom, a (C₁-C₄)alkyl group and a -OR⁶ group; or
- a (C₃-C₆)heterocycloalkyl group;
**R⁵** represents a hydrogen atom or a (C₁-C₄)alkyl group;
or **R⁴** and **R⁵** form together with the nitrogen atom bearing them a (C₅-C₆)heterocycloalkyl group; and
**R⁶** represents a hydrogen atom, a (C₁-C₄)alkyl group or a deuterated (C₁-C₄)alkyl group.

2. The compound of formula (I) or any of its pharmaceutically acceptable salt as defined in claim 1, wherein **R¹** represents a linear or branched (C₁-C₆)alkyl group or a (C₃-C₆)cycloalkyl group, in particular a linear or branched (C₁-C₄)alkyl group or a (C₃-C₅)cycloalkyl group, and more particularly an isopropyl group or a cyclopropyl group.

3. The compound of formula (I) or any of its pharmaceutically acceptable salt, as defined in claim 1 or 2, wherein **R²** represents a hydrogen atom or a (C₁-C₄)alkyl, in particular a hydrogen atom or a methyl group.

4. The compound of formula (I) or any of its pharmaceutically acceptable salt, as defined in any claim 1 to 3, wherein **R³** represents
- a linear or branched (C₁-C₆)alkyl group, optionally substituted by
• one phenyl group, optionally substituted by one to three -OR⁶ group(s), in particular one methoxy group, or
• one (C₅-C₁₀)heteroaryl group, in particular one (C₅-C₆)heteroaryl group, more particularly selected from one pyridyl, one pyrazinyl, one pyrimidinyl and one pyridazinyl group, and even more particularly selected from one pyridyl and one pyridazinyl group, said (C₅-C₁₀)heteroaryl group being optionally substituted by one to three -OR⁶ group(s);
- a (C₅-C₆)heteroaryl group, in particular selected from a pyridyl, a pyrazinyl, a pyrimidinyl or a pyridazinyl group, said (C₅-C₆)heteroaryl group being optionally substituted by one to three substituents selected from a (C₁-C₄)alkyl group, a deuterated (C₁-C₄)alkyl group, a (C₁-C₄)alkoxy group, for example one methoxy group, a deuterated (C₁-C₄)alkoxy group, for example one deuterated methoxy group, and a halogen atom, for example one chlorine atom; or
- a (C₃-C₈)cycloalkyl group, in particular a (C₄-C₆)cycloalkyl group, and more particularly a cyclopentyl group; and
**R⁶** represents a hydrogen atom, a (C₁-C₄)alkyl group or a deuterated (C₁-C₄)alkyl group.

5. The compound of formula (I) or any of its pharmaceutically acceptable salt, as defined in any claim 1 to 4, wherein
**R⁴** represents
- a linear or branched (C₁-C₈)alkyl group, in particular a linear or branched (C₃-C₈)alkyl group, said (C₁-C₈)alkyl group being optionally substituted by one to three -OR⁶ group(s), in particular one hydroxyl group;
- a (C₃-C₇)cycloalkyl group, in particular a (C₄-C₆)cycloalkyl group, more particularly a cyclopentyl group;
- a (C₅-C₆)heteroaryl group, in particular a pyrazolyl group, said (C₅-C₆)heteroaryl group being optionally substituted by one to three substituents selected from a halogen atom, a (C₁-C₄)alkyl group and a -OR⁶ group, in particular one methyl group; or
- a (C₃-C₆)heterocycloalkyl group, in particular a tetrahydrofuranyl group; and
**R⁶** represents a hydrogen atom, a (C₁-C₄)alkyl group or a deuterated (C₁-C₄)alkyl group.

6. The compound of formula (I) or any of its pharmaceutically acceptable salt as defined in any claim 1 to 5, wherein **R⁵** represents a hydrogen atom.

7. A compound of formula (I) as defined in anyone of claims 1 to 5, or any of its pharmaceutically acceptable salt, selected from
**(1)** (2*R*)-2-((7-(benzylamino)-3-isopropylpyrazolo[1,5-*a*]pyrimidin-5-yl)amino)butan-1-ol;
**(2)** *N7*-benzyl-3-isopropyl-*N5*-[(3*R*)-tetrahydrofuran-3-yl]pyrazolo[1,5-*a*]pyrimidine-5,7-diamine;
**(3)** *N7*-benzyl-3-isopropyl-*N5*-(pentan-3-yl)pyrazolo[1,5-*a*]pyrimidine-5,7-diamine
**(4)** 3-isopropyl-*N5*-(pentan-3-yl)-*N7*-(pyridin-4-ylmethyl)pyrazolo[1,5-*a*]pyrimidine-5,7-diamine;
**(5)** 3-isopropyl-*N5*-(pentan-3-yl)-*N7*-(pyridin-3-ylmethyl)pyrazolo[1,5-*a*]pyrimidine-5,7-diamine;
**(6)** 3-isopropyl-*N5*-(pentan-3-yl)-*N7*-(pyridin-2-ylmethyl)pyrazolo[1,5-*a*]pyrimidine-5,7-diamine;
**(7)** 3-isopropyl-*N5*-(pentan-3-yl)-*N7*-(2-(pyridin-2-yl)ethyl)pyrazolo[1,5-*a*]pyrimidine-5,7-diamine;
**(8)** 3-isopropyl-*N5*-(pentan-3-yl)-*N7*-(2-(pyridin-3-yl)ethyl)pyrazolo[1,5-*a*]pyrimidine-5,7-diamine;
**(9)** 3-isopropyl-*N5*-(pentan-3-yl)-*N7*-(2-(pyridin-4-yl)ethyl)pyrazolo[1,5-*a*]pyrimidine-5,7-diamine;
**(10)** *N5*-(2-ethylbutyl)-3-isopropyl-*N7*-(2-(pyridin-2-yl)ethyl)pyrazolo[1,5-*a*]pyrimidine-5,7-diamine;
**(11)** *N5*-(2-ethylbutyl)-3-isopropyl-*N7*-(2-(pyridin-3-yl)ethyl)pyrazolo[1,5-*a*]pyrimidine-5,7-diamine;
**(12)** *N5,N7*-bis(2-ethylbutyl)-3-isopropylpyrazolo[1,5-*a*]pyrimidine-5,7-diamine
**(13)** *N5*-cyclopentyl-3-isopropyl-*N7*-(2-(2-pyridyl)ethyl)pyrazolo[1,5-*a*]pyrimidine-5,7-diamine;
**(14)** *N5*-cyclopentyl-3-isopropyl-*N7*-(2-(3-pyridyl)ethyl)pyrazolo[1,5-*a*]pyrimidine-5,7-diamine;
**(15)** *N5,N7*-dicyclopentyl-3-isopropylpyrazolo[1,5-*a*]pyrimidine-5,7-diamine
**(16)** 3-isopropyl-*N5*-(pentan-3-yl)-*N7*-(pyridazin-3-ylmethyl)pyrazolo[1,5-*a*]pyrimidine-5,7-diamine;
**(17)** 3-isopropyl-N5-(pentan-3-yl)-N7-(pyridazin-4-ylmethyl)pyrazolo[1,5-a]pyrimidine-5,7-diamine;
**(18)** 3-isopropyl-*N5*-(pentan-3-yl)-*N7*-(pyridin-2-yl)pyrazolo[1,5-*a*]pyrimidine-5,7-diamine;
**(19)** 3-isopropyl-*N5*-(pentan-3-yl)-*N7*-(pyridin-3-yl)pyrazolo[1,5-*a*]pyrimidine-5,7-diamine;
**(20)** 3-isopropyl-*N5*-(pentan-3-yl)-*N7*-(pyridazin-3-yl)pyrazolo[1,5-*a*]pyrimidine-5,7-diamine;
(21) *N5*-(2-ethylbutyl)-3-isopropyl-*N7*-(pyridazin-3-yl)pyrazolo[1,5-*a*]pyrimidine-5,7-diamine;
(22) *N5*-(3-ethylpentyl)-3-isopropyl-*N7*-(pyridazin-3-yl)pyrazolo[1,5-*a*]pyrimidine-5,7-diamine;
(23) (*S*)-2-((3-isopropyl-7-(pyridazin-3-ylamino)pyrazolo[1,5-*a*]pyrimidin-5-yl)amino)butan-1-ol;
(24) 3-isopropyl-*N5*-(pentan-3-yl)-*N7*-(pyrimidin-4-yl)pyrazolo[1,5-*a*]pyrimidine-5,7-diamine;
(25) 3-isopropyl-*N5*-(pentan-3-yl)-*N7*-(pyrazin-2-yl)pyrazolo[1,5-*a*]pyrimidine-5,7-diamine;
**(26)** 3-isopropyl-*N5*-(pentan-3-yl)-*N7*-(pyrimidin-5-yl)pyrazolo[1,5-*a*]pyrimidine-5,7-diamine;
**(27)** 3-isopropyl-*N7*-(6-methoxypyridin-2-yl)-*N5*-(pentan-3-yl)pyrazolo[1,5-*a*]pyrimidine-5,7-diamine;
**(28)** 3-isopropyl-*N7*-(5-methoxypyridin-2-yl)-*N5*-(pentan-3-yl)pyrazolo[1,5-*a*]pyrimidine-5,7-diamine;
**(29)** 3-isopropyl-*N7*-(6-methoxypyridin-3-yl)-*N5*-(pentan-3-yl)pyrazolo[1,5-*a*]pyrimidine-5,7-diamine;
**(30)** 3-isopropyl-*N7*-(2-methoxypyrimidin-4-yl)-*N5*-(pentan-3-yl)pyrazolo[1,5-*a*]pyrimidine-5,7-diamine;
**(31)** 3-isopropyl-*N7*-(6-methoxypyridazin-3-yl)-*N5*-(pentan-3-yl)pyrazolo[1,5-*a*]pyrimidine-5,7-diamine;
**(32)** 3-isopropyl-*N7*-(6-methoxypyrimidin-4-yl)-*N5*-(pentan-3-yl)pyrazolo[1,5-*a*]pyrimidine-5,7-diamine;
**(33)** *N7*-(6-chloropyridazin-3-yl)-3-isopropyl-*N5*-(pentan-3-yl)pyrazolo[1,5-*a*]pyrimidine-5,7-diamine;
**(34)** *N7*-(2-chloropyrimidin-4-yl)-3-isopropyl-*N5*-(pentan-3-yl)pyrazolo[1,5-*a*]pyrimidine-5,7-diamine;
**(35)** *N5*-(2-ethylbutyl)-3-isopropyl-*N7*-(pyridin-2-yl)pyrazolo[1,5-*a*]pyrimidine-5,7-diamine;
**(36)** *N5*-(2-ethylbutyl)-3-isopropyl-*N7*-(pyridin-3-yl)pyrazolo[1,5-*a*]pyrimidine-5,7-diamine;
**(37)** *N5*-(2-ethylbutyl)-3-isopropyl-*N7*-(2-methoxypyrimidin-4-yl)pyrazolo[1,5-*a*]pyrimidine-5,7-diamine;
**(38)** *N5*-(2-ethylbutyl)-3-isopropyl-N7-(6-methoxypyridazin-3-yl)pyrazolo[1,5-*a*]pyrimidine-5,7-diamine;
**(39)** 3-cyclopropyl-*N5*-(pentan-3-yl)-*N7*-(2-(pyridin-2-yl)ethyl)pyrazolo[1,5-*a*]pyrimidine-5,7-diamine;
**(40)** 3-cyclopropyl-*N5*-(pentan-3-yl)-*N7*-(pyridin-3-yl)pyrazolo[1,5-*a*]pyrimidine-5,7-diamine;
**(41)** 3-cyclopropyl-*N5*-(pentan-3-yl)-*N7*-(pyridin-2-yl)pyrazolo[1,5-*a*]pyrimidine-5,7-diamine;
**(42)** 3-cyclopropyl-*N5*-(pentan-3-yl)-*N7*-(pyrimidin-4-yl)pyrazolo[1,5-*a*]pyrimidine-5,7-diamine;
**(43)** 3-cyclopropyl-*N5*-(pentan-3-yl)-*N7*-(pyridazin-3-yl)pyrazolo[1,5*-a*]pyrimidine-5,7-diamine;
**(44)** 3-cyclopropyl-*N5*-(pentan-3-yl)-*N7*-(pyrazin-2-yl)pyrazolo[1,5-*a*]pyrimidine-5,7-diamine;
**(45)** 3-cyclopropyl-*N5*-(pentan-3-yl)-*N7*-(pyrimidin-5-yl)pyrazolo[1,5-*a*]pyrimidine-5,7-diamine;
**(46)** (*S*)-2-((3-cyclopropyl-7-(pyridazin-3-ylamino)pyrazolo[1,5-*a*]pyrimidin-5-yl)amino)butan-1-ol;
**(47)** 3-cyclopropyl-*N7*-(6-methoxypyridin-2-yl)-*N5*-(pentan-3-yl)pyrazolo[1,5-*a*]pyrimidine-5,7-diamine;
**(48)** 3-cyclopropyl-*N7*-(6-methoxypyridazin-3-yl)-*N5*-(pentan-3-yl)pyrazolo[1,5-*a*]pyrimidine-5,7-diamine;
**(49)** 3-cyclopropyl-*N7*-methyl-*N5*-(pentan-3-yl)-*N7-*(pyridazin-3-yl)pyrazolo[1,5-*a*]pyrimidine-5,7-diamine;
**(50)** 3-cyclopropyl-*N5*-(2-ethylbutyl)-*N7*-(pyridazin-3-yl)pyrazolo[1,5-*a*]pyrimidine-5,7-diamine;
**(51)** 3-cyclopropyl-*N5*-(2-ethylbutyl)-*N7*-(6-methoxypyridazin-3-yl)pyrazolo[1,5-*a*]pyrimidine-5,7-diamine;
**(52)** 3-cyclopropyl-*N5*-(2-ethylbutyl)-*N7*-(2-methoxypyrimidin-4-yl)pyrazolo[1,5-*a*]pyrimidine-5,7-diamine;
**(53)** 3-isopropyl-*N7*-(4-methoxybenzyl)-*N5*-(pentan-3-yl)pyrazolo[1,5-*a*]pyrimidine-5,7-diamine;
**(54)** *N*-(3-isopropyl-5-(pentan-3-ylamino)pyrazolo[1,5-*a*]pyrimidin-7-yl)-2-phenylacetamide
**(55)** *N*-(3-isopropyl-5-(pentan-3-ylamino)pyrazolo[1,5-*a*]pyrimidin-7-yl)-2-(pyridin-2-yl)acetamide;
**(56)** *N*-(3-isopropyl-5-(pentan-3-ylamino)pyrazolo[1,5-*a*]pyrimidin-7-yl)-2-(pyridin-3-yl)acetamide;
**(57)** *N*-(3-isopropyl-5-(pentan-3-ylamino)pyrazolo[1,5-*a*]pyrimidin-7-yl)-2-(pyridin-4-yl)acetamide;
**(58)** *N7*-benzyl-3-isopropyl-*N5*-(1-methyl-1*H*-pyrazol-5-yl)pyrazolo[1,5-*a*]pyrimidine-5,7-diamine;
in particular selected from compounds (2) to (48) and (50) to (57) and their pharmaceutically acceptable salts, and more particularly from compounds (5) to (10), (13), (16), (18) to (34), (36) to (48), (50) to (52) and (54) to (57) and their pharmaceutically acceptable salts.

8. Synthesis process for manufacturing a compound of formula (I) as defined in anyone of claim 1 to 6 or any of its pharmaceutically acceptable salts, or at least any of compounds (1) to (58) as defined in claim 7 or any of their pharmaceutically acceptable salts, comprising at least a step of reacting a compound of formula (II) 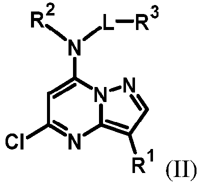
wherein L, R¹, R² and R³ are as defined in anyone of claim 1 to 4 with an amine of formula (VII) 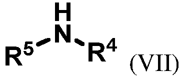
wherein R⁴ and R⁵ are as defined in claim 1, 5 or 6, in particular in an aprotic solvent, protic solvent or without solvent, in particular in a molar ratio ranging from 1 to 20 eq with respect to the compound of formula (II).

9. Synthesis process according to the preceding claim, wherein compound of formula (II) is obtained by a step of reacting a compound of formula (III) 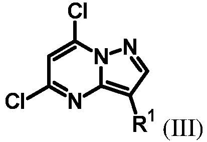
wherein R¹ is as defined in anyone of claim 1 or 2, with an amine of formula (VI) 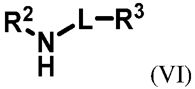
wherein L, R² and R³ are as defined in anyone of claim 1, 3 or 4, in particular in an aprotic solvent, in particular in a molar ratio ranging from 1 to 3 eq and more particularly equal to 1.5 eq with respect to the compound of formula (III).

10. Synthesis process for manufacturing a compound of formula (I) as defined in anyone of claim 1 to 6 or any of its pharmaceutically acceptable salts, or at least any of compounds (1) to (58) as defined in claim 7 or any of their pharmaceutically acceptable salts, comprising at least a step of reacting a compound of formula (VIII) 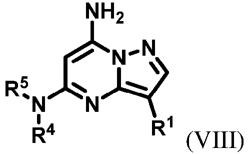
wherein R¹, R⁴ and R⁵ are as defined in claim 1, 2, 5 or 6, with a compound of formula (IX) 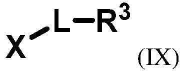
wherein L and R³ are as defined in claim 1 or 4, and X represents a hydroxy group, a chlorine atom, a bromine atom, an (C₁-C₃)alkyl sulfonate group or a phenyl sulfonate group, in particular in an aprotic solvent for example in a molar ratio ranging from 1 to 10 eq, with respect to the compound of formula (VIII).

11. Synthesis process according to the preceding claim, wherein compound of formula (VIII) is obtained by a step of deprotection of para-methoxybenzyl group of a compound of formula (Ib) 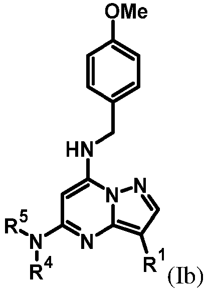 wherein R¹, R⁴ and R⁵ are as defined in claim 1, 2, 5 or 6, in particular in an aprotic solvent with trifluoroacetic acid and more particularly the trifluoroacetic acid being in a volume ratio of 1:10 to 1: 1, with respect to the aprotic solvent.

12. A compound of formula (II) or (VIII), in particular as intermediate compounds 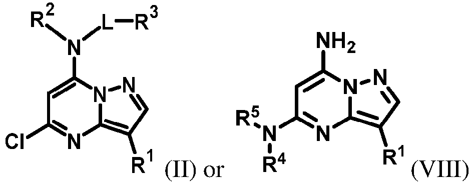 wherein L, R¹, R², R³, R⁴ and R⁵ are as defined in claims 1 to 6.

13. Synthesis process for manufacturing a compound of formula (II) 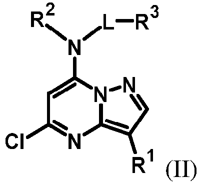
wherein L, R¹, R² and R³ are as defined in anyone of claim 1 to 4,
comprising at least a step of reacting a compound of formula (III) 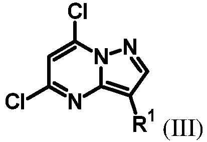
wherein R¹ is as defined in anyone of claim 1 or 2, with an amine of formula (VI) 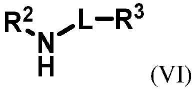
wherein L, R² and R³ are as defined in anyone of claim 1, 3 or 4, in particular in an aprotic solvent, in particular in a molar ratio ranging from 1 to 3 eq and more particularly equal to 1.5 eq with respect to the compound of formula (III).

14. Synthesis process for manufacturing a compound of formula (VIII) 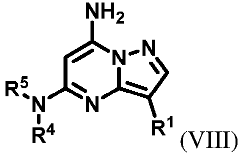
wherein R¹, R⁴ and R⁵ are as defined in claim 1, 2, 5 or 6,
comprising at least a step of deprotection of para-methoxybenzyl group of a compound of formula (Ib) 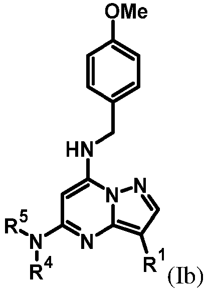
wherein R¹, R⁴ and R⁵ are as defined in claim 1, 2, 5 or 6, in particular in an aprotic solvent with trifluoroacetic acid and more particularly the trifluoroacetic acid being in a volume ratio of 1:10 to 1: 1, with respect to the aprotic solvent.

15. A compound of formula (I) as defined in anyone of claim 1 to 6 or any of its pharmaceutically acceptable salts, or at least any of compounds (1) to (58) as defined in claim 7 or any of their pharmaceutically acceptable salts, for use as a medicament.

16. A compound of formula (I) as defined in anyone of claim 1 to 6 or any of its pharmaceutically acceptable salts, or at least any of compounds (1) to (58) as defined in claim 7 or any of their pharmaceutically acceptable salts, for use in the prevention and/or the treatment of ulcerative colitis, atopic dermatitis, glaucoma, asthma, liver fibrosis, kidney diseases, atherosclerosis, hypercholestemia, hearing loss, dementia, in particular vascular dementia, and cancers developing under hypoxic conditions, in particular glioblastoma.

17. A compound for use as defined in claim 16, wherein the hearing loss is a partial or a complete hearing loss, in particular being induced by noise, acoustic trauma or ototoxic agents or conditions, more particularly platin-based anticancer drugs such as cisplatin or carboplatin, antibiotics, even more particularly macrolides or aminoglycosides such as gentamycin or neomycin, drugs for COVID-19 such as lopinavir or ritonavir, antimalarial drugs such as quinine or chloroquine, cardiovascular drugs such as loop diuretics, non-steroidal anti-inflammatory drugs such as salicylate or cyclodextrins, erectile dysfunction drugs such as phosphodiesterase type 5 inhibitors.

18. A compound for use as defined in claim 16, wherein the kidney disease is selected from kidney fibrosis, nephropathy, acute kidney injury, chronic kidney disease in particular induced by ischemia and/or reperfusion injury, toxic nephropathy or myoglobinuria, and diabetic kidney disease, or the kidney disease is induced by nephrotoxicity caused by therapeutic drugs, more particularly cytotoxic agents, such as platin-based anticancer drugs for example cisplatin or carboplatin.

19. A pharmaceutical composition comprising at least one compound of formula (I) as defined in anyone of claim 1 to 6 or any of its pharmaceutically acceptable salt, or at least any of compounds (1) to (58) as defined in claim 7 or any of their pharmaceutically acceptable salts.
